(19) Europäisches Patentamt
European Patent Office
Office européen des brevets

(11) EP 1 601 646 B1

(12) **EUROPEAN PATENT SPECIFICATION**

(45) Date of publication and mention of the grant of the patent:
**27.07.2011 Bulletin 2011/30**

(21) Application number: **04718886.7**

(22) Date of filing: **10.03.2004**

(51) Int Cl.:
***C07C 401/00*** *(2006.01)* ***A61K 31/59*** *(2006.01)*
***A61P 35/00*** *(2006.01)* ***A61P 19/08*** *(2006.01)*
***A61P 19/10*** *(2006.01)*

(86) International application number:
**PCT/BE2004/000037**

(87) International publication number:
**WO 2004/080922 (23.09.2004 Gazette 2004/39)**

(54) **2-SUBSTITUTED VITAMIN D ANALOGUES AND THEIR THERAPEUTIC USES**

2-SUBSTITUIERTE VITAMIN D ANALOGEN UND IHRE THERAPEUTISCHE ANWENDUNGEN

ANALOGUES DE LA VITAMINE D ET LEURS UTILISATIONS THERAPEUTIQUES

(84) Designated Contracting States:
**AT BE BG CH CY CZ DE DK EE ES FI FR GB GR HU IE IT LI LU MC NL PL PT RO SE SI SK TR**

(30) Priority: **10.03.2003 GB 0305332**

(43) Date of publication of application:
**07.12.2005 Bulletin 2005/49**

(73) Proprietors:
- **K.U. Leuven Research and Development 3000 Leuven (BE)**
- **Universiteit Gent 9000 Gent (BE)**

(72) Inventors:
- **BOUILLON, Roger B-3020 Winksele (BE)**
- **VERSTUYF, Annemieke B-3050 Oud-Heverlee (BE)**
- **VANDEWALLE, Maurits B-9000 Gent (BE)**
- **DE CLERCQ, Pierre B-9000 Gent (BE)**

(74) Representative: **Bird, Ariane et al**
**Bird Goën & Co**
**Klein Dalenstraat 42A**
**3020 Winksele (BE)**

(56) References cited:
**US-A- 5 936 105**

- **SICINSKI RAFAL R ET AL: "2-Ethyl and 2-ethylidene analogues of 1alpha,25-dihydroxy-19-norvita min D3: Synthesis, conformational analysis, biological activities, and docking to the modeled rVDR ligand binding domain" JOURNAL OF MEDICINAL CHEMISTRY, vol. 45, no. 16, 1 August 2002 (2002-08-01), pages 3366-3380, XP002291416 ISSN: 0022-2623**
- **POSNER GARY H ET AL: "2,2-disubstituted analogues of the natural hormone 1alpha,25-dihydroxyvitamin D3: Chemistry and biology" BIOORGANIC AND MEDICINAL CHEMISTRY, vol. 10, no. 7, July 2002 (2002-07), pages 2353-2365, XP002291417 ISSN: 0968-0896**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 601 646 B1

## Description

FIELD OF THE INVENTION

The present invention relates to novel vitamin D analogues, more specifically to 2-substituted-19-nor-14-epi-1,25$(OH)_2D_3$ and 2-substituted-19-nor-1,25$(OH)2_D3$ analogues, to their use as a medicine and to pharmaceutical preparations containing the vitamin D analogues of the invention. The present invention also relates to methods of preparation of these 2-substituted-19-nor-14-epi-1,25$(OH)_2D_3$ and 2-substituted-19-nor-1,25$(OH)2D_3$ analogues.

BACKGROUND OF THE INVENTION

Vitamin D of either nutritional (vitamin $D_2$ or $D_3$) origin or produced in the skin under the influence of ultraviolet light is metabolized in several tissues to produce firstly 25-hydroxyvitamin $D_3$ [25OH$D_3$] and later 1$\alpha$,25-dihydroxyvitamin $D_3$ [1$\alpha$,25$(OH)_2D_3$] and numerous other vitamin D metabolites. Several hydroxylases present in different tissues (e.g. liver, kidney, placenta, keratinocytes, fibroblasts, monocytes, bone cells, ...) are responsible for both the activating and inactivating pathways of the parent vitamin D molecules. 1$\alpha$,25$(OH)_2D_3$ behaves as a classical steroid hormone as its synthesis is feedback controlled by several hormones, ions and humoral factors to maintain a normal body homeostasis of plasma and bone minerals. Moreover the vitamin D hormone(s) act(s) via binding and activation of specific vitamin D receptors, present in most tissues and cells. High affinity binding of 1$\alpha$,25$(OH)_2D_3$ to the nuclear vitamin D receptor (VDR), followed by dimerization of the liganded VDR with the retinoid X receptor (RXR) and binding of the VDR-RXR heterodimer to specific vitamin D responsive elements (VDREs) in the promoter region of vitamin D target genes regulates the transcription of a large and diverse set of genes. Moreover there is some evidence for vitamin D, its metabolites and analogues to act via nongenomic mechanisms, either by activating ion channels or other membrane related or second messenger signals. Vitamin D, its metabolites and analogues have potent effects on calcium and phosphate metabolism, and therefore they can be used for prevention and therapy of vitamin D deficiency and other disorders of plasma and bone mineral homeostasis (e.g. osteomalacia, osteoporosis, renal osteodystrophy, disorders of the parathyroid function). Moreover vitamin D receptors are found in numerous tissues and cells that do not belong to the target tissues responsible for the above mentioned calcium homeostasis. Vitamin D receptors and vitamin D activity have also been documented in calcium transporting tissues other than the intestine and bone (e.g. placenta and mammary glands). In addition vitamin D receptors and vitamin D action have been observed in most other cells (e.g. cells of the immune system, skin cells, colon, brain, endocrine glands, muscle cells). These cells or tissues can be of a benign, adenomatous or of a malignant type. The hormone was found to be capable of regulating proliferation and differentiation of a variety of immunological and malignant cells. 1$\alpha$,25$(OH)_2D_3$ appears to determine the transition from a state of proliferation to a state of differentiation. It is involved in the stimulation of non-specific immunicty by monocytes and the inhibition of lymphocytic specific immunity as well as in the regulation of the growth and differentiation of normal cells (embryogenesis) and cancer cells (induction of differentiation of melanoma, breast cancer, myeloid leukaemia, lymphoma and osteosarcoma cells). These so-called non-calcemic effects of vitamin D, its metabolites and analogues create the possibility to use such compounds for various therapeutic applications such as modululation of the immune system, modification of hormone secretion, altering calcium transport in several tissues, influencing intercellular calcium concentration, induction of cell differentiation or inhibition of cell proliferation. In particular such compounds have been considered to be potentially useful in the therapy of hyperproliferative disorders [e.g. psoriasis, cancer, (auto)immune diseases].

At the present time, the pathological conditions associated with vitamin D are classified as vitamin D deficiencies or excesses. Vitamin D deficiencies are due either to insufficient exposure to sunlight combined with an inadequate exogenous provision in the food, or to abnormalities of vitamin D metabolism. Genetic abnormalities are described in respect of the renal hydroxylase (1$\alpha$-hydroxylase) or in respect of the vitamin D receptor (vitamin D resistance). Interference with the metabolism can also occur in the course of various pathological conditions, and especially renal insufficiency and hypoparathyroidism, or as a result of pharmacological interactions, for example with antiepileptic drugs and corticoids.

The clinical manifestations of these vitamin D deficiencies are most clearly apparent at bone level: rickets, osteomalacia and possibly participation in the phenomena of osteoporosis. There are other, less obvious associated disorders, for example an immune deficiency and a higher incidence of certain cancers and of vascular and endocrine disorders. Most deficiencies are readily corrected by the exogenous provision of vitamin D. An active form, for example 1$\alpha$,25$(OH)_2D_3$, must be administered if the metabolism is abnormal, in particular in case of renal insufficiency. 1$\alpha$,25 $(OH)_2D_3$ however has a short half-life, which often justifies the taking of two doses daily.

Vitamin D excesses are encountered essentially during vitamin D poisoning, or during an ectopic production of active metabolites, for example during granulomatous diseases (sarcoidosis). Hyperparathyroidism stimulates the excessive production of 1,25-(OH)2-vitamin D, which also appears to accompany familial idiopathic hypercalciuria. The active derivatives of vitamin D employed therapeutically, and especially 1,25-(OH)2)-vitamin D used, in particular, in

renal insufficiency, have a very narrow therapeutic index, so that Vitamin poisoning is common during their administration. Thus, the treatment of a vitamin D poisoning is often the result of the treatment of a vitamin D deficiency;

The major drawback regarding the use of 1α,25$(OH)_2D_3$ is its toxicity associated with its calcemic effect, which prevents the application of pharmaceutical doses. Current research is therefore aimed at the synthesis of analogues with superagonistic potentcy but, in particular at the decoupling of the advantageous effects from the calcemic effects.

A large number of analogues incorporating modifications in the A-ring, in the CD-ring fragment and, especially, in the side chain have been synthesized and tested biologically. It is now well established that removal of the 19-exomethylene function is beneficial: 19-nor-1α,25-dihydroxyvitamin $D_3$ displays a smaller calcemic effect (10 % of that of 1α,25$(OH)_2D_3$) while retaining good cell-differentiating properties. It was further observed that, among other modifications, 14-epimers (first disclosed in U.S. Patent No. 6,017,907) are among the best analogues known to date in that they show still smaller hypercalcemic effects (circa 0.1 % of that of 1α,25$(OH)_2D_3$).

Recently a new class of vitamin D analogues have been discovered namely 19-nor-14-epi-1,25$(OH)_2D_3$ analogues (Novel structural analogues of vitamin D" (EP 0 972 762 A2, US 6,017,907 Bouillon R., De Clercq P., Vandewalle M). Biological testing of such analogues revealed a selective activity profile with strong antiproliferative activity and very low calcemic effects. These compounds could be used as therapeutic agents for the treatment of cancer or various skin disorders.

2p-hydroxy and alkoxy analogues of 1,25$(OH)_2D_3$ have been described by Chugai Company in the US patent No 4,666,634. 2-alkyl and 2-hydroxyalkyl analogues of 1,25$(OH)_2D_3$ have been described by Nikagawa et al. Biochem Pharmacol 60, 1937-1947, 2000; Nakagawa et al., Biochem Pharmacol 59, 691-702, 2000; Takayama et al., Steroids 66,277-285 (2001); Fujishima et al. Bioorg Med Chem Lett 8, 2145-2148, 1998; Suhara et al. J Org Chem 66, 8760-8771, 2001; Konno et al. J Med Chem 43, 4247-4265, 2000. Synthesis and biological activity of 2-hydroxy and 2-alkoxy analogues of 19-nor-1,25$(OH)_2D_3$ have been described in J Med Chem 37, 3730-3738, 1994. 2-substituted A-ring 19-nor-analogues have been described and examined such as 2-alkyl (US No 6,127,559) and 2-alkylidene (US No 5,936,133) vitamin D compounds. See also Sicinski et al. J Med Chem 45, 3366-338, 2002; Sicinski et al. J Med Chem 41, 4662-4674, 1998. Only one 14-epi-19-nor-1,25$(OH)_2D_3$ analogue with a substitution on carbon 2 has already been described, namely 2-methylene-14-epi-19-nor-1,25$(OH)_2D_3$ in US 5,936,105.

## SUMMARY OF THE INVENTION

The present invention relates to novel vitamin D analogues and the use of vitamin D analogues with improved properties in the treatment and prevention of particular conditions and diseases.

Thus, a first aspect of the invention relates to vitamin D derivatives having a different pharmaco-kinetic profile and a more favourable therapeutic index.

According to a particular aspect, the analogues of the present invention enable the different biological activities with respect to the target cells to be dissociated; more particularly a dissociation of the beneficial effects of vitamin D from the calcemic effects is envisaged.

It was surprisingly found that the compounds described herein have a selective activity on cell function, such as inhibition of cell proliferation (non-malignant cells such as keratinocytes as well as malignant cell such as breast carcinoma, osteo-sarcoma and leukemia cells) and/or have a high potency for induction of cell differentiation (e.g. cell types as just mentioned) but on the other hand have strikingly lower effect on calcium and bone homeostasis as evaluated in rachitic chicks (by measuring serum and bone calcium, and by measurement of two vitamin D-dependent proteins, serum osteocalcin and duodenal calbindin D) as well as in vitamin D repleted normal mice (using similar end points). Thus, unlike the classical vitamin D compounds, the new drugs do not have the same toxic effect on calcium and bone homeostasis. Specifically the new drugs can generally be used for the therapy or prevention of:

immune disorders, such as auto-immune diseases (such as, but not limited to diabetes mellitus type 1, multiple sclerosis, lupus and lupus like disorders, asthma, glomerulonephritis, auto-immune thyroidis, etc.), selective dysfunctions of the immune system (e.g. AIDS) and prevention of immune rejection [such as rejections of grafts (e.g. kidney, heart, bone marrow, liver, islets or whole pancreas, skin etc.) or prevention of graft versus host disease] and other inflammatory diseases (e.g. rheumatoid arthritis). The newly invented drugs can either be used alone or in combination with other immuno-modulating drugs known to interfere with the immune system (e.g. cyclosporin, FK 506, glucocorticoids, monoclonal antibodies, cytokines or growth factors).

skin disorders either characterized by hyperproliferation and/or inflammation and/or (auto)immune reaction (e.g. psoriasis, dyskeratosis, acne). Moreover since these drugs can stimulate the differentiation of skin cells they can be used for the treatment or prevention of alopecia of whatever origin (including alopecia due to chemotherapy or irradiation). hyperproliferative disorders and cancer such as hyperproliferative skin diseases (e.g. psoriasis) and several types of cancers and their metastases (all types of cancer which have or can be induced to have vitamin D receptors such as but not limited to breast cancer, leukemia, myelo-dysplastic syndromes and lymphomas, squamous

cell carcinomas and gastrointestinal cancers, melanomas, osteosarcoma, etc). The newly invented drugs can be used in combination with other chemotherapeutic drugs known to be of therapeutic value in such disorders. These new drugs may be particularly advantageous for such diseases as they can, in contrast to classical chemo-therapeutic agents, also stimulate cell differentiation;
endocrine disorders since vitamin D analogues can modulate hormone secretion, such as increased insulin secretion or selective modulation of parathyroid hormone secretion (e.g. in chronic renal failure, secondary hyperparathyroidism and hypoparathyroidism); and diseases characterized by abnormal intracellular calcium handling since the new drugs have favourable effects in cells whose functions depend largely on intracellular calcium movements (e.g. endocrine cells, muscle, etc.).

The use of the new compounds can find application as well in human disorders as in veterinary medicine.

The amount of the new compounds necessary for their therapeutic effect can vary according to its indication, route of administration and species (animal/man) treated. The compounds can be administered by enteral, parenteral or local topical route. In the treatment of dermatological disorders a topical application as ointment, cream or lotion is to be preferred over systemic treatment, preferably in a dose of 0.1 to 500 $\mu$g / g. The systemic administration as tablets, capsules, liquid or as sterile preparation in an appropriate carrier, diluent and/or solvent for parenteral injection will use microgram quantities of the compounds per day depending on the indication and the clinical/veterinary situation.

According to a particular embodiment of the invention analogues are described with increased activity on bone forming cells without a simultaneous potency on bone resorbing cells or vice versa. Such analogues are usefull in the treatment of bone disorders such as osteoporosis.

According to another particular embodiment of the invention analogues are described which have an increased potency to inhibit proliferation and/or increase differentiation of cancer cells (e.g. leukemia or breast cancer cells) and at the same time have a reduced potency to influence serum, urinary or bone calcium or phosphate homeostasis. Such analogues are of interest in cancer treatment.

According to yet a further aspect of the invention, enhanced effects on proliferation and differentiation are obtained by combining analogues of 1 $\alpha$,25$(OH)_2D_3$ with other drugs (e.g. growth factors or cytokines, other steroid or antisteroid hormones or retinoic acids or related compounds, chemotherapeutics). Similarly, analogues are envisaged with increased activity on specific hormone secretion (e.g. parathyroid hormone, insulin) without the same relative potency for the other activities of the natural vitamin D hormohe(s). Analogues with increased activity on of the immune system (activated T-cells, antigen presenting cells) are envisaged for the treatment of immune disorders. Indeed, analgues of vitamin D have proven to be effective in experimental models of type I diabetes, graft rejection without major effects on calcium and phosphate metabolism.

According to a further aspect of the invention, the use of inactive precursors is envisaged, which has the advantage of limiting direct activity on the intestine when an oral dose is taken. Some precursors can then be activated by pathways independent of the normal metabolism of vitamin D (the so-called " prodrugs "). Such derivatives display a special biodistribution capable of imparting a selective biological effect *in vivo*. Some of them are especially well suited for other administration routes such as transcutaneous administration which constitutes, for example, an effective treatment for psoriasis.

The present invention relates to vitamin D compounds and more particularly to certain stereoisomers of 14-epl-19-nor-1,25$(OH)_2D_3$, analogues with one lower alkyl substituent at carbon 2, the said lower alkyl substituent being optionally substituted with functional atoms or groups, with or without modification of the side chain at carbons 20 and higher. In particular it was found surprisingly found that certain combinations of stereoisomeric configurations at carbon 1, carbon 2 and carbon 3 provide unexpected biological activity profile, toxicity profile and pharmaco-kinetic profile to the corresponding vitamin D analogues. Thus, according to a particular embodiment, the present invention relates to certain stereoisomers of 14-epi-19-nor-1,25$(OH)_2D_3$ with only one lower alkyl substituent present at carbon 2 and preferably when said lower alkyl substituent has no more than 1 carbon atom, whereby the selection of a configuration $\alpha$ at said carbon 2 is able to provide a significant and useful decrease of side effects with respect to the corresponding stereoisomer having a configuration $\beta$ at said carbon 2. It is demonstrated that such compounds are capable of increasing the level of calcium in bone, without resulting in an elevation in urine and/or serum calcium. Consequently the selected stereoisomer (having a configuration $\alpha$ at said carbon 2) is of preferred interest in the treatment of bone diseases such as osteoporosis.

According to another particular embodiment of the invention, 14-epi-19-nor-1,25$(OH)_2D_3$ having only one lower alkyl substituent present at carbon 2, whereby the lower alkyl substituent has 2 to 5 carbon atoms are provided which are able to provide a significantly higher specificity ratio than the corresponding analogues with an alkyl substituent having at least five carbon atoms at position 2. Consequently the selected analogues (having an alkyl substituent with 2 to 5 carbon atoms, particularly having an alkyl substituents with 2 carbon atoms, with either a configuration $\alpha$ or a configuration $\beta$ at said carbon 2) are of preferred interest in the treatment of cancer.

Thus, the present invention relates to novel vitamin D analogues and to their use as a medicine. The present

invention relates more in particular to the use of the specific compounds of the invention for the preparation of a medicament for the treatment of cancer or other diseases characterised by a cellular hyperproliferation, for induction of cell differentiation, for the treatment or prevention of immunological or inflammatory disorders and for the improvement of the function of cells in which calcium is an essential regulating agent, such as for the treatment and prevention of osteoporosis. The present invention furthermore relates to a suitable method of preparation of the specific compounds of the invention in high yield and purity.

BRIEF DESCRIPTION OF THE DRAWINGS

Figure 1 schematically represents a method of synthesis of the vitamin D analogues of this invention.
Figure 2 schematically represents an alternative method of synthesis of the vitamin D analogues of this invention.
Figure 3 schematically represents an alternative method of synthesis of the vitamin D analogues of this invention.
Figure 4 schematically represents a method of synthesis of precursors useful for making the vitamin D analogues of this invention.
Figure 5 schematically represents a method of synthesis of precursors useful for making the vitamin D analogues of this invention.
Figure 6 schematically represents a method of synthesis of precursors useful for making the vitamin D analogues of this invention.
Figure 7 shows the detailed formulae of individual vitamin D analogues of this invention.
Figure 8 schematically represents an alternative method of synthesis of the vitamin D analogues of this invention.
Figure 9 shows differentiating effects of vitamin D analogues of this invention on HL-60 cells.
Figure 10 shows antiproliferative effects of vitamin D analogues of this invention on MCF-7 cells.

DEFINITIONS

As used herein with respect to a substituting group and unless otherwise stated, the terms "$C_{1-5}$ alkyl" and "alkyl groups having from 1 to 5 carbon atoms " mean straight and branched chain saturated acyclic hydrocarbon monovalent radicals or groups having from 1 to 5 carbon atoms such as, for example, methyl, ethyl, propyl, n-butyl, 1-methylethyl (isopropyl), 2-methylpropyl (isobutyl), 1,1-dimethylethyl (ter-butyl), 2-methylbutyl, n-pentyl, dimethylpropyl, and the like;

As used herein with respect to a substituting group and unless otherwise stated, the term aryl " designate any mono- or polyaromatic monovalent hydrocarbon radical having from 6 up to 30 carbon atoms such as but not limited to phenyl, naphthyl, anthracenyl, phenantracyl, fluoranthenyl, chrysenyl, pyrenyl, biphenylyl, terphenyl, picenyl, indenyl, indacenyl, benzocyclobutenyl, benzocyclooctenyl and the like, including fused benzo-$C_{5-8}$ cycloalkyl radicals (the latter being as defined above) such as, for instance, indanyl, tetrahydronaphtyl, fluorenyl and the like;

As used herein, the term " unsaturated " refers to aliphatic unsaturated hydrocarbon radical, i.e. straight or branched acyclic hydrocarbon monovalent radicals having one or more ethylenic or acetylenic unsaturations.

As used herein and unless otherwise stated, the term "cycloalkyl" means a monocyclic saturated hydrocarbon monovalent radical having from 3 to 10 carbon atoms, such as for instance cyclopropyl, cyclobutyl, cyclopentyl, cyclohexyl, cycloheptyl, cyclooctyl and the like, or a $C_{7-10}$ polycyclic saturated hydrocarbon monovalent radical having from 7 to 10 carbon atoms such as, for instance, norbornyl, fenchyl, trimethyltricycloheptyl or adamantyl.

As used herein with respect to a substituting group and unless otherwise stated, the term " heterocyclic " means a mono- or polycyclic, saturated or mono-unsaturated or polyunsaturated monovalent hydrocarbon radical having from 2 up to 15 carbon atoms and including one or more heteroatoms in one or more heterocyclic rings, each of said rings having from 3 to 10 atoms (and optionally further including one or more heteroatoms attached to one or more carbon atoms of said ring, for instance in the form of a carbonyl or thiocarbonyl or selenocarbonyl group and/or to one or more heteroatoms of said ring, for instance in the form of a sulfone, sulfoxide, N-oxide, phosphate, phosphonate or selenium oxide group), each of said heteroatoms being independently selected from the group consisting of nitrogen, oxygen, sulfur, selenium and phosphorus, also including radicals wherein a heterocyclic ring is fused to one or more aromatic hydrocarbon rings for instance in the form of benzo-fused, dibenzo-fused and naphto-fused heterocyclic radicals;

As used herein and unless otherwise stated, the term " enantiomer " means each individual optically active form of a compound or an intermediate of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

DETAILED DESCRIPTION OF THE INVENTION

A first aspect of the invention thus relates to novel 2-alkylated-19-nor-14-epi-1,25$(OH)_2D_3$ compounds.

The invention relates to the synthesis and biological evaluation of said novel compounds which still maintain some of the essential characteristics of vitamin D action but with a more selective pattern (i.e. not all the actions of the physiological vitamin D hormone are maintained with the same relative potency) and with a structure being modified in the A-ring and in the side-chain and with cis-fused CD-ring systems (i.e. 14-epi analogues) as represented in the following general formula (I). The invention thus relates to 2-alkylated-19-nor-14-epi-1,25$(OH)_2D_3$ compounds. In particular the invention relates to a 2-substltuted-14-epi-19-nor-vitamin D having, general formula (I), and optionally pharmaceutically acceptable salts and/or solvates thereof,

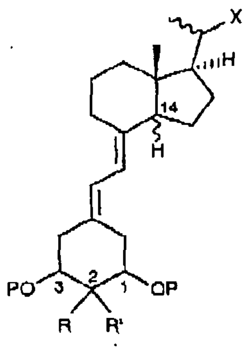

wherein:

- P is selected from the group consisting of hydrogen, alkyl (preferably $C_{1-7}$ alkyl),cycloalkyl (preferably $C_{3-10}$ cycloalkyl), acyl (preferably $C_{1-7}$ acyl), and other protecting groups;
- carbon atom 20 may have either the R or S configuration;
- the hydrogen atom at carbon 14 is in the β configuration:
- R' is hydrogen;
- R is selected from the group consisting of normal or branched alkyl groups having from 1 to 5 carbon atoms, preferably 1 to 4 carbon atoms, more preferably 1 to 3 carbon atoms, and most preferably 1 or 2 carbon atoms, and being optionally substituted with one or more functional atoms or groups selected from the group consisting of fluoro, chloro, hydroxy, sulfhydryl and amino, provided that when R is an alkyl group with only one carbon atom, said group is in a configuration α at carbon 2;
- X represents an alkyl side-chain consisting of 2 to 15 carbon atom which can be substituted or functionalised as follows:

hydroxyl substituent at one or more positions, for instance at position 24, 25 and/or 26, and/or

methyl or ethyl substituent in one or more positions, for instance at position 24, 26 and/or 27, and/or

halogen substituent(s) at one or more positions, for Instance perfluorated at positions 26 and / or 27 or difluorated at position 24, and/or esters derivatives or ether derivatives of one or more hydroxyl substituents mentioned above, and/or

changing one or more carbon atoms for an oxygen, nitrogen or sulfur atom, for instance at one or more of positions 22, 23 and 24, and/or

cyclized between the carbon atoms 26 and 27 by one bond (cyclopropane) or by the intermediacy of 1 to 4 carbon atoms, the resulting ring being saturated, unsaturated or aromatic and being optionally substituted at any possible position(s) with one or more substituents mentioned above, and/or

cyclized at one carbon or between two carbon atoms by 1 to 4 atoms to form a heterocyclic ring, including aromatic, which may be substituted at any possible position with one or more substituents mentioned above, and/or

unsaturated with one or more double or triple carbon-carbon bond(s), these unsaturated chains being optionally substituted at any possible position by one or more substituents mentioned above, and/or

epoxidized once or more between carbon atoms (preferably between 22 and 23, or between 23 and 24, or between 24 and 25, or between 25 and 26), the resulting epoxidized chain(s) being saturated or unsaturated and, when saturated, optionally substituted at any possible positions with one or more substituents mentioned above, and/or

two or more of the carbon atoms of said alkyl side-chain X being linked by a single bond or by the intermediacy of 1 to 5 carbon or oxygen or nitrogen or sulfur atoms to form a 3-7 membered saturated or unsaturated carbocyclic (including aromatic) or heterocyclic ring which may be substituted at any possible position(s) by one or more substituents mentioned above, and/or
substituted at one or more positions by one or more saturated or unsaturated, carbocyclic (including aromatic) or heterocyclic rings which can be substituted at any possible position(s) with one or more substituents mentioned above,
including all possible isomeric forms of said alkyl side-chain X.

In another particular embodiment, the invention relates to vitamin D analogues wherein R' is hydrogen and R is an alkyl group with only one carbon atom, and further wherein the OP group is in a configuration α at carbon 1. In another particular embodiment, the invention relates to vitamin D analogues wherein R' is hydrogen and R is an alkyl group with only one carbon atom, and further wherein the OP group is in a configuration α at carbon 3. In another particular embodiment, the invention relates to vitamin D analogues wherein R' is hydrogen and R is an alkyl group with only one carbon atom, and further wherein the OP group is in a configuration β at carbon 3. In another particular embodiment, the invention relates to vitamin D analogues wherein R' is hydrogen and R is an alkyl group with two carbon atoms.

In a specific embodiment the invention relates to compounds with the following structure:

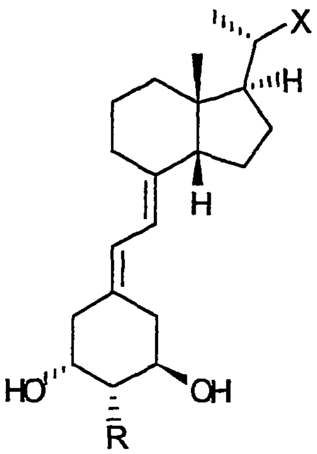

**105, 107, 113, 115**

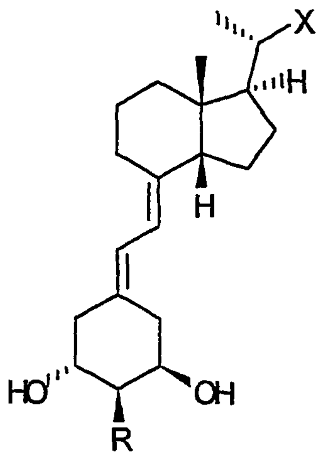

**106, 108, 114, 116**

**105** R = Me X = 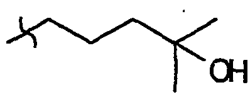

**106** R = Me X = 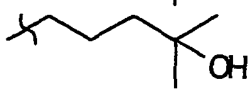

**107** R = Me X = 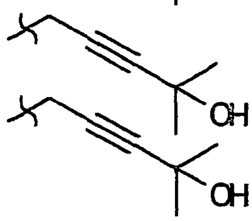

**108** R = Me X =

**113** R = Et X = 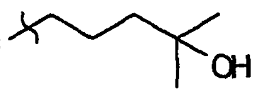

**114** R = Et X = 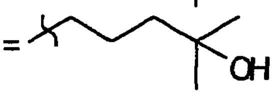

**115** R = Et X = 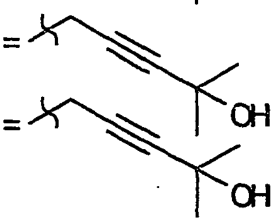

**116** R = Et X =

In a specific embodiment the invention relates to compounds with the following structure:

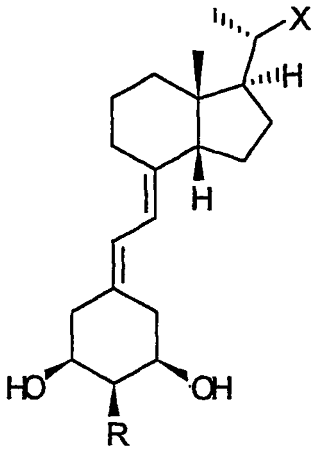

**117,119,120,121**

**117** R = Me X = 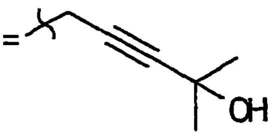

**119** R = Me X = 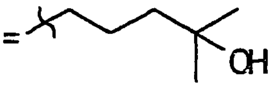

**120** R = Et X = 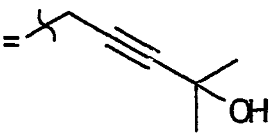

**121** R = Et X = 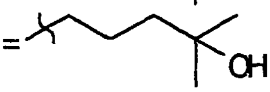

In a specific embodiment the invention relates to compounds with the following structure:

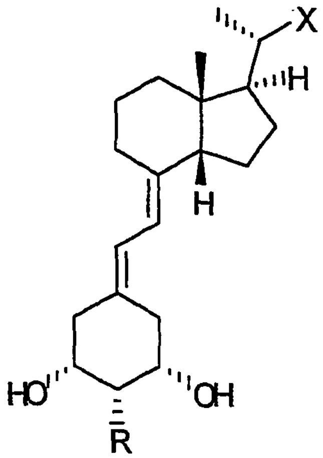

**118, 126, 127, 128**

**118** R = Me X = 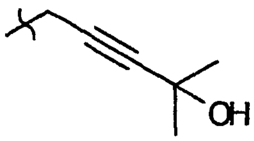

**126** R = Me X = 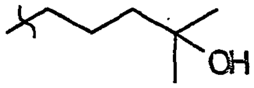

**127** R = Et X = 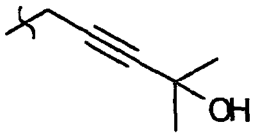

**128** R = Et X = 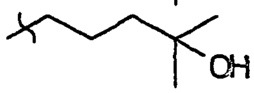

Another particular embodiment of the present invention relates to compounds with the structure (20S configuration)

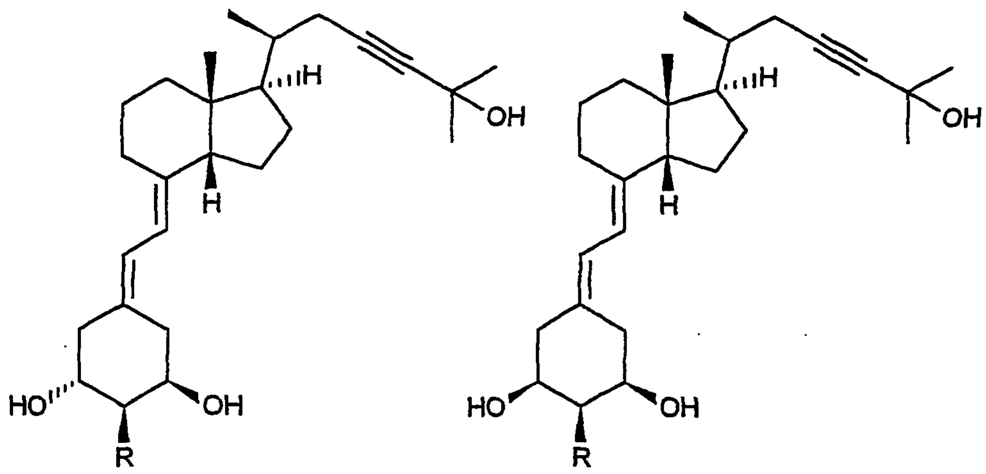

**141** R = Me　　　　**143** R = Me

**142** R = Et　　　　**144** R = Et

A second aspect of the invention relates to the compounds according to formula I for use as a medicine. The invention also relates to the compounds according to formula 1 for the treatment of hyperproliferative disorders or for the preparation of a medicament for the prevention or treatment of hyperproliferative disorders such as cancer and psoriasis and for the induction of cell differentiation. The invention also relates to the compounds according to formula I for the treatment of immunological disorders(such as allergy, asthma, auto-immune disorders, transplant rejection, etc.) or for the preparation of a medicament for the prevention or treatment of immunological disorders optionally in combination with an immune system interfering drug, inflammatory diseases (i.e. rheumatoid arthritis), skin disorders such as psoriasis and hyperproliferative disorders such as cancer. The invention further relates to use of a compound of formula 1 for the preparation of a medicament for improvement of the function of cells in which calcium is an essential regulating agent, for instance hormone secretion by endocrine glands, muscle cells and bone cells such as necessary in osteoporosis.

The invention also relates to the compound of formula I, for use as a pharmaceutically active ingredient, especially as an inhibitor of cell proliferation and/or an inductor of cell proliferation. Therefore, the invention also relates to the use of a compound of formula I for the manufacture of a medicine or a pharmaceutical composition having an inhibitory effect on cell proliferation and/or an inductor effect on cell differentiation for the prevention and/or treatment of hyperproliferative disorders such as cancer and psoriasis, immunological disorders, inflammatory disorders, calcium related diseases such as osteoporosis and for the induction of cell differentiation in humans and mammals. The invention also relates to a pharmaceutical preparation comprising a therapeutically effective amount of a compound of the invention and a pharmaceutically and/or veterinarily acceptable carrier or diluent. The compounds of the present invention can be used in a method of treatment of hyperproliferative disorders in a mammal, including a human, comprising administering to the mammal in need of such treatment a therapeutically effective amount of a compound of formula 1, optionally in a mixture with at least a pharmaceutically acceptable carrier.

Another aspect of the invention relates to methods for the preparation of compounds of formula 1, more particularly to methods for the preparation of the compounds specifically disclosed herein, to pharmaceutical compositions comprising them in admixture with at least a pharmaceutically acceptable carrier, the active ingredient optionally being in a concentration range of about 0.1-100% by weight, and to the use of these derivatives.

The compounds of the invention are employed for the treatment or prophylaxes of hyperproliferative disorders, immunological, inflammatory and calcium metabolism related disorders. The compounds of the invention have an inhibitory effect on cell proliferation such as in disorders like cancer and psoriasis. The compounds have also a positive effect on immunological and/or inflammatory diseases such as auto-immune disorders. The compounds of the invention have furthermore an activity on the calcium metabolism and are useful in disorders like osteoporosis.

The compounds may be present in a composition in an amount from about 0.1 μg/gm to about 100μg/gm of the composition.

The dosages envisaged within the context of the invention are in the range of 0.1μg/kg/day to 500μg/kg/day, particularly 0.5 μg/kg/day to 100μg/kg/day, more particularly 1.0 μg/kg/day to 10 μg/kg/day. Depending upon the pathologic condition to be treated and the patient's condition, the said effective amount may be divided into one or more subunits per day or may be administered at more than one day intervals.

According to a particular embodiment, the vitamin D analogues of the present invention are characterized by a higher specificity, thus allowing the treatment of a patient in need thereof with higher doses and/or more frequent administration and/or for a prolonged period of time without the occurrence of side-effects which, when are referred to as vitamin D toxicity. Vitamin D toxicity, mainly associated to its calcemic effect can cause symptoms such as but not limited to nausea, vomiting, poor appetite, constipation, weakness and weight loss. High levels of calcium can also cause changes in mental status (e.g. confusion) and heart rhythm abnormalities. Calcinosis, i.e. the deposition of calcium and phosphate in soft tissues like the kidney, can also be caused by vitamin D toxicity.

Administration of vitamin D analogues at a higher dose than currently being used in clinical settings (e.g. paricalcitol, calcitriol etc) are of interest because the calcemic effects of these analogues are dose-limiting, often resulting in a sub-optimal effect.

Prolonged administration (i.e. several months up to lifelong) of the analogues of the present invention are of particular interest in the treatment of metabolic vitamin D deficiencies, the treatment of persistant or structural (?) diseases such as cancer, autoimmune diseases, Alzheimer's and osteoporosis, or the administration to persons subjected to reduced sunlight for longer periods of time.

Moreover the analogues of the present invention are of particular use in the treatment of patients in need thereof which are susceptible to the calcemic side-effects of vitD, such as patients that are underweight or with metabolic deficiencies or in situations where administration of vitamin D is contraindicated such as in patients with severe renal failure, patients receiving therapy with cardiac glycosides (eg digoxin, digitalis) or patients with sarcoidosis.

As is conventional in the art, the evaluation of a synergistic effect in a drug combination may be made by analyzing the quantification of the interactions between individual drugs, using the median effect principle described by Chou et al. in Adv. Enzyme Reg. (1984) 22:27. Briefly, this principle states that interactions (synergism, additivity, antagonism) between two drugs can be quantified using the combination index (hereinafter referred as CI) defined by the following equation:

$$CI_x = \frac{ED_x^{1c}}{ED_x^{1a}} + \frac{ED_x^{2c}}{ED_x^{2a}}$$

wherein $ED_x$ is the dose of the first or respectively second drug used alone (1a, 2a), or in combination with the second or respectively first drug (1 c, 2c), which is needed to produce a given effect. The said first and second drug have synergistic or additive or antagonistic effects depending upon CI < 1, CI = 1, or CI > 1, respectively.

This principle may be applied to a combination of different drugs of the invention or to a combination of the drugs of the invention with other drugs that exhibit therapeutic effects on hyperproliferative disorders, immunological, inflammatory and calcium metabolism related disorders. The invention thus relates to a pharmaceutical composition or combined preparation having synergistic effects on hyperproliferative disorders, immunological, inflammatory and calcium metabolism related disorders and containing either

(A) a combination of:

(a) two or more of the compounds of the present invention, and
(b) optionally one or more pharmaceutical excipients or pharmaceutically acceptable carriers,
for simultaneous, separate or sequential use in the treatment or prevention of hyperproliferative disorders, immunological, inflammatory and calcium metabolism related disorders, or

(B) a combination of:

(c) one or more agents having an effect on hyperproliferative disorders (such as anti-cancer agents), immunological, inflammatory and calcium metabolism related disorders, and
(d) at least one of the compounds of the present invention, and
(e) optionally one or more pharmaceutical excipients or pharmaceutically acceptable carriers,

for simultaneous, separate or sequential use in the treatment or prevention of hyperproliferative disorders, immunological, inflammatory and calcium metabolism related disorders.

The pharmaceutical composition or combined preparation with synergistic activity against hyperproliferative disorders, immunological, inflammatory and calcium metabolism related disorders according to this invention may contain

the 19-nor-14-epi-1,25$(OH)_2D_3$ derivatives of the present invention over a broad content range depending on the contemplated use and the expected effect of the preparation. Generally, the content of the 19-nor-14-epi-1,25$(OH)_2D_3$ derivatives of the present invention of the combined preparation is within the range of 0.1 to 99.9% by weight, preferably from 1 to 99% by weight, more preferably from 5 to 95% by weight.

The present invention further provides veterinary compositions comprising at least one active ingredient as above defined together with a veterinary carrier therefor. Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

More generally, the invention relates to the compounds of formula I being useful as agents having biological activity or as diagnostic agents. Any of the uses mentioned with respect to the present invention may be restricted to a non-medical use, a non-therapeutic use, a non-diagnostic use, or exclusively an in vitro use, or a use related to cells remote from an animal.

Those of skill in the art will also recognize that some compounds of the invention may exist in different protonation states, depending on, among other things, the pH of their environment. While the structural formulae provided herein depict the compounds in only one of several possible protonation states, it will be understood that these structures are illustrative only, and that the invention is not limited to any particular protonation state, any and all protonated forms of the compounds are intended to fall within the scope of the invention.

The term "pharmaceutically acceptable salts" as used herein means the therapeutically active non-toxic salt forms which some of the compounds of formula I are able to form. Therefore, the compounds of this invention optionally comprise salts of the compounds herein, especially Pharmaceutical acceptable non-toxic salts containing, for example, Na+, Li+, K+, Ca+2 and Mg+2. Such salts may include those derived by combination of appropriate cations such as alkali and alkaline earth metal ions or ammonium and quaternary amino ions with an acid anion moiety, typically a carboxylic acid. The compounds of the invention may bear multiple positive or negative charges. The net charge of the compounds of the invention may be either positive or negative. Any associated counter ions are typically dictated by the synthesis and/or isolation methods by which the compounds are obtained. Typical counter ions include, but are not limited to ammonium, sodium, potassium, lithium, halides, acetate, trifluoroacetate, etc., and mixtures thereof. It will be understood that the identity of any associated counter ion is not a critical feature of the invention, and that the invention encompasses the compounds in association with any type of counter ion. Moreover, as the compounds can exist in a variety of different forms, the invention is intended to encompass not only forms of the compounds that are in association with counter ions (e.g., dry salts), but also forms that are not in association with counter ions (e.g., aqueous or organic solutions). Furthermore, this term also includes the solvates which the compounds of formula I as well as their salts are able to form, such as for example hydrates, alcoholates and the like. Finally, it is to be understood that the compositions herein comprise compounds of the invention in their unionized, as well as zwitterionic form, and combinations with stoichiometric amounts of water as in hydrates. Also included within the scope of this invention are the salts of some of the parental compounds with one or more amino acids, especially the naturally-occurring amino acids found as protein components. The amino acid typically is one bearing a side chain with a basic or acidic group, e.g., lysine, arginine or glutamic acid, or a neutral group such as glycine, serine, threonine, alanine, isoleucine, or leucine.

The compounds of the invention also include physiologically acceptable salts thereof. Examples of physiologically acceptable salts of the compounds of the invention include salts derived from an appropriate base, such as an alkali metal (for example, sodium), an alkaline earth (for example, magnesium), ammonium and NX4+ (wherein X is C1-C4 alkyl). Physiologically acceptable salts of a compound containing a hydroxy group include the anion of said compound in combination with a suitable cation such as Na+ and NX4+ (wherein X typically is independently selected from H or a C1-C4 alkyl group). However, salts of acids or bases which are not physiologically acceptable may also find use, for example, in the preparation or purification of a physiologically acceptable compound. All salts, whether or not derived form a physiologically acceptable acid or base, are within the scope of the present invention.

The terms cis and trans are used herein in accordance with Chemical Abstracts nomenclature and include reference to the position of the substituents on a ring moiety. The absolute stereochemical configuration of the compounds of formula (I) may easily be determined by those skilled in the art while using well-known methods such as, for example, X-ray diffraction.

The compounds of the invention may be formulated with conventional carriers and excipients, which will be selected in accord with ordinary practice. Tablets will contain excipients, glidants, fillers, binders and the like. Aqueous formulations are prepared in sterile form, and when intended for delivery by other than oral administration generally will be isotonic. Formulations optionally contain excipients such as those set forth in the "Handbook of Pharmaceutical Excipients" (1986) and include ascorbic acid and other antioxidants, chelating agents such as EDTA, carbohydrates such as dextrin, hydroxyalkylcellulose, hydroxyalkylmethylcellulose, stearic acid and the like.

Subsequently, the term "pharmaceutically acceptable carrier" as used herein means any material or substance with which the active ingredient is formulated in order to facilitate its application or dissemination to the locus to be

treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, suspensions, ointments, creams, tablets, pellets or powders.

Suitable pharmaceutical carriers for use in the said pharmaceutical compositions and their formulation are well known to those skilled in the art, and there is no particular restriction to their selection within the present invention. They may also include additives such as wetting agents, dispersing agents, stickers, adhesives, emulsifying agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, coating and/or grinding the active ingredients, in a one-step or multi-steps procedure, with the selected carrier material and, where appropriate, the other additives such as surface-active agents may also be prepared by inicronisation, for instance in view to obtain them in the form of microspheres usually having a diameter of about 1 to 10 gm, namely for the manufacture of microcapsules for controlled or sustained release of the active ingredients.

Suitable surface-active agents, also known as emulgent or emulsifier, to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids ($C_{10}$-$C_{22}$), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanylphosphatidyl-choline, dipalmitoylphoshatidyl-choline and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkytsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from I to 5 ethyleneglycol units per propyleneglycol unit. Representative examples of non-ionic surfactants are nonylphenol - polyethoxyethanol, castor oil polyglycolic ethers, polypropylene/polyethylene oxide adducts, tributylphenoxypolyethoxyethanol, polyethyleneglycol and octylphenoxypoly-ethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, particularly halides, having 4 hydrocarbon radicals optionally substituted with halo, phenyl, substituted phenyl or hydroxy; for instance quaternary ammonium salts containing as N-substituent at least one C8C22 alkyl radical (e.g. cetyl, lauryl, palmityl, myristyl, oleyl and the like) and, as further substituents, unsubstituted or halogenated lower alkyl, benzyl and/or hydroxy-lower alkyl radicals.

A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbucw', 2 d ed. (Hanser Verlag, Vienna, 1981) and "Encyclopaedia of Surfactants, (Chemical Publishing Co., New York, 1981).

Compounds of the invention and their physiologically acceptable salts (hereafter collectively referred to as the active ingredients) may be administered by any route appropriate to the condition to be treated, suitable routes including, oral, rectal, nasal, topical (including transdermally, ocular, buccal and sublingual), vaginal and parenteral (including

subcutaneous, intramuscular, intravenous, intra-arterially, intradermal, intrathecal and epidural). The preferred route of administration may vary with for example the condition of the recipient.

While it is possible for the active ingredients to be administered alone it is preferable to present them as pharmaceutical formulations. The formulations, both for veterinary and for human use, of the present invention comprise at least one active ingredient, as above described, together with one or more pharmaceutically acceptable carriers therefore and optionally other therapeutic ingredients. The carrier(s) optimally are "acceptable" in the sense of being compatible with the other ingredients of the formulation and not deleterious to the recipient thereof. The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein. The formulations of the invention are optionally applied as a topical ointment or cream (i.e. for psoriasis) containing the active ingredient(s) in an amount of, for example, 0.075 to 20% w/w (including active ingredient(s) in a range between 0.1% and 20% in increments of 0.1 % w/w such as 0.6% w/w, 0.7% w/w, etc), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base. If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol (including PEG400) and mixtures thereof. The topical formulations may desirably include a compound which enhances absorption or penetration of the active ingredient through the skin or other affected areas. Examples of such dermal penetration enhancers include dimethylsulfoxide and related analogs.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Optionally, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

The choice of suitable oils or fats for the formulation is based on achieving the desired cosmetic properties, since the solubility of the active compound in most oils likely to be used in pharmaceutical emulsion formulations is very low. Thus the cream should optionally be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate. Formulations suitable for nasal administration wherein the carrier is a solid include a coarse powder having a particle size for example in the range 20 to 500 microns (including particle sizes in a range between 20 and 500 microns in increments of 5 microns such as 30 microns, 35 microns, etc), which is administered in the manner in which snuff is taken, i.e. by rapid inhalation through the nasal passage from a container of the powder

held close up to the nose. Suitable formulations wherein the carrier is a liquid, for administration as for example a nasal spray or as nasal drops, include aqueous or oily solutions of the active ingredient. Formulations suitable for aerosol administration may be prepared according to conventional methods and may be delivered with other therapeutic agents.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavoring agents.

Compounds of the invention can be used to provide controlled release pharmaceutical formulations containing as active ingredient one or more compounds of the invention ("controlled release formulations") in which the release of the active ingredient can be controlled and regulated to allow less frequency dosing or to improve the pharmacokinetic or toxicity profile of a given invention compound. Controlled release formulations adapted for oral administration in which discrete units comprising one or more compounds of the invention can be prepared according to conventional methods.

Additional ingredients may be included in order to control the duration of action of the active ingredient in the composition. Control release compositions may thus be achieved by selecting appropriate polymer carriers such as for example polyesters, polyamino acids, polyvinyl pyrrolidone, pthylene-vinyl acetate copolymers, methylcellulose, carboxymethylcellufose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polyniethyl methacrylate and the other above-described polymers. Such methods include colloid drug delivery systems like liposomes, microspheres, microemulsions, nanoparticles, nanocapsules and so on. Depending on the route of administration, the pharmaceutical composition may require protective coatings. Pharmaceutical forms suitable for injectionable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol and the like and mixtures thereof.

In view of the fact that, when several active ingredients are used in combination, they do not necessarily bring out their joint therapeutic effect directly at the same time in the mammal to be treated, the corresponding composition may also be in the form of a medical kit or package containing the two ingredients in separate but adjacent repositories or compartments. In the latter context, each active ingredient may therefore be formulated in a way suitable for an administration route different from that of the other ingredient, e.g. one of them may be in the form of an oral or parenteral formulation whereas the other is in the form of an ampoule for intravenous injection or an aerosol.

The vitamin D analogues of the present invention can be prepared by several methods involving a series of chemical reactions, each of them being well known to those skilled in the art, altogether making up the process for preparing said compounds and exemplified further. The processes described further are only meant as examples and by no means are meant to limit the scope of the present invention. The synthesis of 14α derivatives is described herein to illustrate the synthesis of the claimed 14β-compounds.

A first method of synthesis of these analogues is very schematically shown in figure 1. This method synthesis is based on the so-called cyclovitamin approach which involves condensation of a vinylic bromide having the general formula III with a compound having the general formula IV. Vinylic bromides III may be obtained from known ketones II by methods well known to the skilled person. The synthesis of the respective diastereoisomeric bicyclic intermediates IV has been described e.g. in International patent publication WO 01/42251.

More specifically, a typical example of the synthesis of 14-epi-2β-methyl-1α,25-dihydroxyvitamin $D_3$ (compound **10-6**) is described in the figure 2. The synthesis of the vinylic bromide **15** may be performed based on the teachings of Trost et al. in J. Am. Chem. Soc. (1992) 114:9836-9844, and, as adapted for cis-fused hydrindanones, of Wu et al. in Eur. J. Org. Chem. (2001) 3779-3788 and Van Gool et al. in Eur. J. Org. Chem. (1998) 2241-2248. Reaction of the vinylic lithium derivative of **15** with the appropriate diastereoisomer of bicyclic A-ring precursor IV **17** (according to figure 1) led to intermediate **18** as a C-6 1:1 epimeric mixture by using the conditions of reaction step (a), i.e. using tBuLi in tetrahydrofuran (THF) at a temperature from about -70°C to - 10°C during about 1 hour. The subsequent acid catalyzed solvolysis, involving stereoselective attack of water, proceeds by way of two rotamers around the 5,6-bond in intermediate

**18.** The rotamer **18** shown in figure 2 is largely preferred (circa 9 : 1) and, using the conditions of reaction step (b), i.e. using *p*-toluenesutfonic acid (hereinafter PTSA) in a dioxane / $H_2O$ (1 : 1) mixture at a temperature of 55-60°C during 4 hours, led to intermediate **19** which, upon silyl ether deprotection using the conditions of reaction step (c), i.e. in THF at room temperature during about 72 hours, gave the required isomer **10-6.**

The other isomers (**102** to **114**) were obtained by the same general procedure, starting either from intermediates **15** or **16** or their respective 2-ethyl homologues and the appropriate diastereoisomer of the bicyclic A-ring precursor (IV in figure 1) of **17.**

The following 20-epi analogues were obtained similarly starting from 20-epi □.

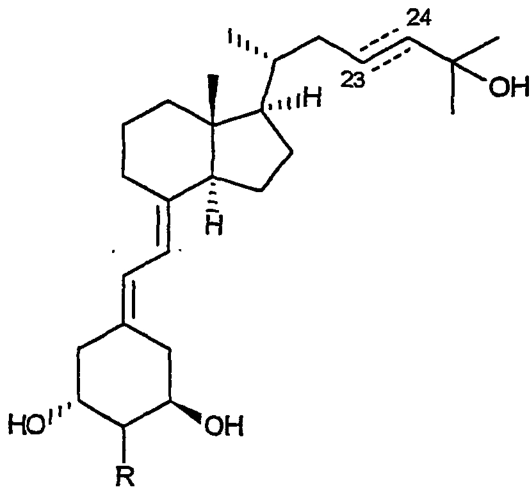

R = αMe, 23,24 - single bond
R = βMe, 23,24 - single bond
R = αMe, 23,24 - triple bond
R = βMe, 23,24 - triple bond
R = αEt, 23,24 - single bond
R = βEt, 23,24 - single bond
R = αEt, 23,24 - triple bond
R = βEt, 23,24 - triple bond

In the 19-*nor* series of vitamin D analogues, as shown in figure 3, the synthesis of compounds **101** and **102,** the corresponding 2-hydroxymethyl analogues and their 20-epimers may be performed via a Horner-Wittig reaction, also known as Lythgoe coupling, involving phosphine oxides. A 1:1 mixture of compounds **101** and **102** may also be obtained upon non-selective catalytic hydrogenation of 2-methylene-19-*nor*-1α,25-dihydroxyvitamin $D_3$ and then separated by reversed phase HPLC. Application of this strategy in a stereoselective synthesis involving a 2-substituted phosphine oxide (using steroid numbering) is however hampered by the fact that this particular A-ring precursor no longer possesses a pseudo $C_2$ axis of symmetry; consequently a control element for the 5,6-double bond geometry relative to the configuration at carbon 2 is required.

We herein provide a convergent synthesis of the compounds of the invention via the so-called cyclovitamin approach as an alternative to the more widely used Lythgoe coupling process. Again referring to figure 3, intermediate cyclopropyl alcohols such as **25, 26a** or **27a** are subjected to an acid-catalyzed stereoselective solvolysis, which may proceed via two rotamers around the 5,6-double bond, in equilibrium with each other. For example, rotamer **i** of **25** provides **1** with the desired 3α and 5,6-Z configurations, while rotamer **ii** leads to 5,6-E isomer **28.** Depending upon the substituents present and the applicable reaction conditions, the **1 : 28** isomer ratio observed is at best 4 : 1. This is of no consequence for the synthesis of 19-*nor* analogues such as compound **2** in which the A-ring has a pseudo-$C_2$ axis of symmetry, after introduction of a substituent at carbon 2 as in intermediate **27a** (starting from intermediate **44a,** see figure 8), a mixture of epimeric compounds **101** and **102** could occur. However, one rotamer was observed to be predominant especially during solvolysis directed towards 1- and/or 3-epimers of compound **2** where also this element

of symmetry is absent, leading to selectivities up to 18 : 1. Synthesis of the compounds of the invention was effected while taking into account that the presence of the 1α-hydroxy function is generally accepted as essential for receptor binding and biological activity and that these properties are influenced by the substitution pattern and the derived conformational behaviour of the A-ring.

Figure 4 illustrates the synthesis of bicyclo[3.1.0]cyclohexane A-ring precursors such as **44a** (R = Me; a-series) and its epimers and homologues (R = Et; b-series). This synthesis focuses around methyl *all-cis*-3,5-dihydroxy-4-methylcyclohexaneboxylate **29a** and its 4-ethyl homologue **29b** for which the enzyme-catalyzed asymmetrization is already known. According to figure 4, transesterification of **29a** with vinyl acetate in the presence of SAM II, a lipase from *Pseudomonas fluorescens,* afforded the pure enantiomer **30** in high yield. For the ethyl substituted series, intermediate **29b** was transformed via CCL- (a lipase from *Candida cylindracea*) or SAM II-mediated mono-hydrolysis to **31** in a completely enantioselective manner. An important feature of key intermediates **30** and **31** is the fact that they permit access to all stereoisomeric bicyclic A-ring precursors. Essential transformations are (i) inversions directed towards the required relative configuration(s) at carbon 1, carbon 2 and carbon 3 (steroid numbering) and (ii) cyclopropane ring formation via a leaving group at an oxy-substituent. Since intermediates **30** and **31** belong to different enantiomeric series, the order of the initial steps (a), (b) and (c) must be inverted (*e.g.* **30** → **32a** via (a), (b), (c); **31** → **32b** via (c), (b) and (a)) for the synthesis of homologues with identical absolute configuration. Preferred reaction conditions for performing step (a) include the Presence of a phosphine such as triphenylphosphine, a diazo compound such as diisopropyl azodicarboxylate (hereinafter DIAD), in a solvent such as tetrahydrofuran (hereinafter THF), during about 3 hours at room temperature. Preferred reaction conditions for performing step (b) include the presence of potassium carbonate in a solvent such as methanol at room temperature during about 6 hours. Preferred reaction conditions for performing step (c) include the presence of a triarylsilyl halide or trialkylsilyl halide or alkyldiarylsilyl halide such as *tert*-butyldiphenylsilyl (hereinafter TBDPS) chloride and the presence of a catalyst such as 4-(dimethylamino)pyridine (hereinafter DMAP), in a solvent such as dimethylformamide (hereinafter DMF), during about 10 hours at room temperature. If appropriate, the reaction time of each step may be decreased by a simultaneous increase in the reaction temperature.

When the hydroxy function is flanked by a *cis*-vicinal alkyl substituent, facile anti-elimination occurs in high yield, as is shown in figure 4 by the reaction of acetate **30** (91% yield of substituted cyclohexene) and the transformation of intermediate **32** into **33b.** We also observed that in the case of a vicinal trans-relation the Mitsunobu inversion proceeds normally, as demonstrated by the transformation of intermediates **34a,b** into intermediates **40a,b.**

In the next step (d), the easy transformation of cyclohexene intermediates was exploited. Hydroboration of intermediates **33a,b** in tetrahydrofuran as a solvent, at 0°C during 2-3 hours was non-selective and afforded a separable circa 1 : 1 mixture of intermediates **34a,b** and **35a,b.** Both diastereoisomers are suitable for further transformation into the corresponding mesylates **36a,b** and **37a,b** in step (e) through a reaction with mesityl chloride preferably in the presence of a catalyst such as triethylamine, in methylene chloride as a solvent, at 0°C during about 3 hours. The mesylate **37a,b** led, upon base-mediated cyclopropane formation in step (f) and subsequent conversion of the ester function of intermediate **38a,b** into a formyl group in step (g), to intermediates **39a,b** which are the precursors for the 2α-alkyl-19-*nor*-1α,25-dihydroxyvitamin $D_3$ analogues **102, 104, 106, 108, 110 112, 114** and **116.** Preferred reaction conditions for performing step (f) include the presence of potassium *tert*-butoxide, in a solvent such as a mixture of THF and *tert-butanol,* during about 30 minutes at about 50°C. Preferred reaction conditions for performing step (g) include for instance a reaction with lithium aluminum hydride in a solvent such as THF, during about 3 hours at about 0°C. On the other hand Mitsunobu inversion of the hydroxy group of intermediates **34a,b** is performed in step (h) and provides intermediates **41a,b** in high yield. Preferred reaction conditions for performing step (h) include for instance a reaction with *p*-nitrobenzoïc acid during about 24 hours in the presence of a phosphine such as triphenylphosphine, a diazo compound such as DIAD, in a solvent such as THF at room temperature. This opens, successively via intermediates **41a,b** through step (b), then intermediates **42a,b** through step (e), and then intermediates **43a,b** through step (f), a route to intermediates 44a,b which are the precursors for the epimeric 2β-alkyl vitamin $D_3$ analogues **101, 103,105, 107, 109, 111, 113** and **115.** Finally, mesylation of intermediates **34a,b** into intermediates **36a,b,** followed by cyclopropane formation in intermediates **45a,b** in step (f) and ester reduction in step (g) afforded intermediates **46a,b** which are the precursors for vitamin D analogues with a 1α, 2β, 3α configuration.

As shown at bottom of figure 4, the present invention also provides a stereoselective synthetic route via lactonic key intermediates. For example hydroboration of lactone **47** (available from the mono-acetate **30** through a succession of steps (a), (b) and (i)) gave intermediate **48** concomitantly with lactone reduction. Preferred reaction conditions for performing step (i) include the presence of PPTS at about 80°C during about 24 hours. After protection of the hydroxy function of intermediate **48** in step (c), the intermediate **49** is then transformed successively via step (b) into intermediate **50** and via step (e) into intermediate **51**, and many via step (1) into the already described A-ring precursor **44a.** Likewise, it is clear that after mesylation of intermediate **48,** the mesylate **52** is a useful intermediate for intermediate **37a** and hence for precursor **39a.** This represents an acceptable route for the synthesis of a particular vitamin $D_3$ analogue.

As shown in figure 5, the respective enantiomers (**63a,b, 65a,b** and **67a,b**) of the A-ring precursors **46a,b, 39a,b** and **44a,b** can be constructed via **56a,b** upon applying the same set of reactions previously described in figure

4. Key intermediates **56a,b,** i.e. the enantiomers of intermediates **33a,b,** were obtained from mono-esters **30** and **31b** by inverting the respective order of the 3-step sequence shown in figure 4. Hydroboration of intermediates **56a,b** afforded a separable mixture of intermediates **58a,b** (useful for making intermediates **67a,b**) and **60a,b.** The mesylate **61a,b** of the latter led to A-ring precursors **65a,b,** while initial Mitsunobu inversion of intermediates **60a,b** opens the route to intermediates **63a**,**b**. In figure 5, the preferred conditions for reaction steps (a), (b) and (c) are the same as for the corresponding steps in figure 4. The preferred conditions for reaction step (d) include the presence of *p*-nitrophenyl sulfonyl chloride, triethylamine, DMAP as a catalyst, during about 10 hours in methylene chloride as a solvent. The preferred conditions for reaction step (e) include the presence of cesium acetate, 18-crown-6-ether as a catalyst, during about 4 hours in toluene as a solvent.

As already mentioned above, an alternative for the Mitsunobu reaction when the hydroxy function is flanked by a *cis*-vicinal 4-alkyl substituent consists of inversion of the corresponding p-nitrophenylsulfonate with cesium propionate. Thus intermediate **54** is able, via intermediate **55,** to provide **57a,** an intermediate for the A-ring precursor **67a.**

The synthesis of the stereoisomers **69a,b** and **71a,b** is shown in figure 6 and does not require an inversion step. Thus intermediate **69a** was obtained starting from **30** via successively protection, hydrolysis, mesylation and cyclopropane formation; intermediate **69b** was similarly formed from **31** upon changing the order of the reaction sequence. Again adapting the same set of reactions led to enantiomers **71a,b.**

With the A-ring precursors in hand we turned our attention to the construction of the vitamin $D_3$ skeleton by the cyclovitamin D strategy described in figure 3. Reaction of the aldehydes, *e.g.* **44a** with the vinylic lithium derivative of **20** led to intermediate **27a** as a 1 : 1 epimeric mixture at carbon 6 (see figure 8). The subsequent acid catalyzed solvolysis, involving stereoselective attack of water, proceeds by way of two rotamers **i** and **ii** around the 5,6-bond (see also figure 3) in equilibrium with each other. It is clear that reaction via rotamer i of intermediate **27a** will lead to the introduction of an hydroxy group at carbon 3 (intermediate **72a** and finally compound **101),** while rotamer ii will introduce a hydroxy group at carbon 1 (intermediate **73a** and finally compound **102**). In order to be able to distinguish the two stereoisomers **101** and **102,** the TBDPS protective group was chosen since it is stable under solvolysis conditions. The major product **72a** arises from attack at carbon 3; intermediates **72a** and **73a** were formed in a 88 : 12 ratio. This ratio was somewhat more pronounced in the *cis*-CD ring (14-epi) series; the sequence starting from intermediates **44a** and **21** led to the 1 TPDPS ether of compound **105** and the 3-TPDPS ether of compound **106** in a 94 : 6 ratio. This difference between *trans*- and *cis*-fused hydrindanes was consistently found, independently from the configuration of the bicyclic A-ring fragment.

The structure of intermediate **72a** was proven by nuclear Overhauser effect (hereinafter NOE) and 2D correlation spectroscopy (hereinafter COSY-2D) experiments. The vinylic protons 6-H and 7-H (AB system; $\delta$ = 6.14 and $\beta$ = 5.46; *J* = 11.3 Hz) gave a NOE enhancement with respectively 10$\alpha$-H and 4$\alpha$,$\beta$-H. The assignment of these protons followed from the observation of a NOE enhancement with respectively 1-H ($\delta$ = 3.72) and 3-H ($\delta$ = 3.94). Localization of the latter protons follows from the trichloroacetate of intermediate **72a.** For 3-H a chemical shift from $\delta$ = 3.94 to $\delta$ = 5.17 was observed and COSY-2D experiments and NOE enhancements corroborated with those obtained for intermediate **72a.**

It is noteworthy that for the 1-OTBDPS ether series, 1-H is downfield relative to 3-H for the isomers with a 2$\alpha$-substituent while a reversed situation is observed for the 2$\beta$-substituted isomers. Identical observations have been made for the stereoisomers with varying carbon 1, carbon 2 and carbon 3 configurations in both the 2-methyl and 2-ethyl substituted series.

In a few cases in the 14-*epi* series a NOE enhancement between 7-H and an aromatic proton of the 1-OTBDPS substituent was also observed, which by itself is a structural proof. The most simple tool for structural identification in these series is provided by a consistent upfield chemical shift of 7-H in 1-OTBDPS ethers for which 6-H and 7-H give a $\Delta\delta$ = 0.5 - 0.7. This upfield shift is probably due to the anisotropy of a phenyl group of the DBDPS ether, which is in fact confirmed by the observed NOE enhancement (7-H, H-Ar). The regioisomers (3-OTBDPS) show the signals for 6-H and 7-H closer together, ($\Delta\delta$ = 0.1 - 0.3) with a small upfield shift for 6-H compared to the $\delta$-values observed for the unprotected title compounds.

Finally, TBDPS ether cleavage led to the desired 2-alkyl-substituted vitamin D analogues. In conclusion the above disclosed cyclovitamin route of synthesis provides good stereoselectivity.

The following examples are provided as an illustration of the invention and should in no way interpreted as limiting its scope.

All synthesis reactions were carried out under argon or nitrogen atmosphere with magnetic stirring. All solvents were purified or dried according to standard procedures. Solutions were dried over $MgSO_4$. The solvent was removed from the filtered solutions on a rotary evaporator. Column chromatography separations were performed on silica gel, eluents are given between brackets. HPLC separations were performed on a Knauer 64, a wasters 6000 A or a Kontron 420 delivery system with RI detection, eluents are given between brackets. Optical rotations were measured with a Perkin Elmer 421 polarimeter. IR spectra were recorded on a Perkin Elmer FTIR-1600 spectrometer and mass-spectra on a HP-5988 spectrometer. The $^{1}H$ NMR spectra were recorded at 500 MHz (WH-Bruker) and $^{13}C$ NMR spectra at

200 MHz (Varian-Gemini), the chemical shifts are expressed in ppm relative to TMS and coupling constants are in Hz.

Example 1 - preparation of (4*R*,6*R*)-6-*t*-butyldiphenylsilyloxv-1-methyl-4-methoxycarbonyl-1-cyclohexene (intermediate **33a**)

Reference is made to figure 4. To a stirred solution of intermediate **30** (1.3 g, 5.65 mmol) and $Ph_3P$ (4.5 g, 17.16 mmol in THF (15 mL) was added dropwise DIAD (3.6 mL, 17.16 mmol, 95% pure) at 0°C. After stirring for 3 hours at room temperature, the reaction solution was subjected to flash chromatography (using a pentane / $Et_2O$ 3 : 1 mixture) affording a colorless oil. It was dissolved in a dry MeOH (20 mL) and then was treated with $K_2CO_3$ (0.36 g, 2.61 mmol). After stirring for 6 hours, the solution was poured into $H_2O$-EtOAc, extracted (EtOAc), washed, dried and concentrated. Flash chromatography (using a isooctane / EtOAc 4 : 1 mixture) gave an intermediate as crystals from *n*-hexane-acetone [characterized as follows: m.p. 70 - 71 °C, $R_f$ = 0.21 (isooctane/EtOAc, 4:1). - $[\alpha]_D^{r.t}$= - 21.6 (c = 1.09, $CHCl_3$). - $C_9H_{14}O_3$ (170.21): calcd. C 63.51, H 8.29; found C 63.33, H 8.34]. TBDPSCI (1.55 mL, 98%, 5.99 mmol) was added to a solution of the intermediate (0.83 g, 4.88 mmol), imidazole (0.41 g, 6.02 mmol) and a cat. amount of DMAP in dry DMF (12 mL) at 0°C, The resulting mixture was stirred for 10 h at room temp. and was then poured into $H_2O$-EtOA separated, extracted (EtOAc), washed, dried and evaporated. Chromatography (isooctane/EtOAc, 95:5) gave **33a** (1.68 g, 73% overall) as a colourless oil characterized as follows: $R_f$ = 0.42 (isooctane/EtOAc, 9:1). - $[\alpha]_D^{r.t}$ = - 82.9 (c = 0.79, $CHCl_3$). - IR (film): *v* = 2952, 2857, 1738, 1589, 1472, 1428, 1362, 1308, 1247, 1169 $cm^{-1}$. - $^1H$ NMR ($CDCl_3$): δ = 7.73 - 7.37 (m, 10 H), 5.39 (m, 1 H), 4.24 (s, 1 H), 3.59 (s, 3 H), 2.39 (m, 1 H), 2.32 - 2.02 (m, 3 H), 1.75 (m, 1 H), 1.66 (s, 3 H), 1.07 (s, 9 H). -$^{13}C$ NMR ($CDCl_3$): δ= 175.2, 137.5, 136.1, 134.4, 133.7, 129.6, 127.6, 121.9, 71.4, 51.6, 38.9, 35.2, 27.9, 27.1, 20.2, 19.5. - MS (mlz): 408 ($M^+$, 1), 354 (8), 351 ($M^+$ - 57, 73), 299 (5), 273 (6), 213 (100), 183 (75), 137 (70). Elemental analysis: $C_{25}H_{32}O_3Si$ (408.61): calcd. C 73.49, H 7.89; found C 74.16, H 8.32.

Example 2 - preparation of the ethyl homologue **(33b)** of intermediate **(33a)**

Reference is made to figure 5. To a stirred solution of the monobutyrate **31** (1.9 g, 7.04 mmol), imidazole (1.43 g, 21.03 mmole) and DMAP (44 mg) in dryDMF (15 mL) was added dropwise TBDPS chloride (3.6 mL, 98% pure, 13.57 mmole). The mixture was stirred at room temp for 10 h. The solution was poured into $H_2O$-EtOAc, separated, extracted (EtOAc), washed, dried and concentrated. The residue was dissolved in a dry MeOH (80 mL) and $K_2CO_3$ (324 mg, 2.35 mmol) was added. The mixture was stirred at room temp. for 6 i h and then the solution was poured into $H_2O$-EtOc, separated, extracted (EtOAc), washed, dried and concentrated. The residue was purified by flash chromatography (isooctane/EtOAc, 4:1) affording the intermediate alcohol (2.5 g, 81%) as a colourless oil. It (1.9 g, 4.32 mmol) was taken up in dry THF ( 30 mL)containing $Ph_3P$ (5.77 g, 22.04 mmol). To this solution a DIAD (4.1 mL, 22.04 mmol) was dropwise added at 0°C, After stirring overnight at room temp., the solution was concentrated. The residue was purified by chromatography (isooctane/EtOAc, 95:5) affording **33b** (1.82 g, 92%) characterized as follows: $R_f$ = 0.28 (isooctane/EtOAc, 95:5). - $[\alpha]_D^{r.t}$ = - 74.8 (c = 0.81, $CHCl_3$ - IR (film): v = 2957, 2857, 1738, 1429, 1246, 1166, 1108, 1064, 935, 895, 821, 740, 704 $cm^{-1}$. -$^1H$NMR ($CDCl_3$): δ= 7.73 - 7.38 (m, 10 H), 5.36 (br.s, 1 H), 4.26 (br.s, 1 H), 3.58 (s, 3 H), 2.36 (m, 1 H), 2.32 (m, 1 H), 2.16(m, 2 H), 2.02 (m, 2 H), 1.75 (m, 1 H), 1.05 (s, 9 H), 0.87 (t, J = 7.4 Hz, 3 H). -$^{13}C$ NMR ($CDCl_3$: δ = 175.1, 136.7, 134.4, 133.5, 129.5, 127.1, 119.7, 70.2, 51.5, 38.7, 35.3, 27.7, 27.0, 25.3, 19.4, 12.3. - MS (m/z, %): 422 ($M^+$, 2), 401 (3), 365 (72), 333 (6), 267 (8), 255 (2), 227 (7), 213 (100), 201 (65), 163 (55), 137 (58), 105 (32), 79 (75), 41 (40). - Elemental analysis: $C_{26}H_{34}O_3Si$ (422.64): calcd. C 73.89, H 8.11; found C 73.72, H 8.27.

Example 3 - preparation of methyl (1*R*,3*S*,4*R*,5*R*)-5-*t*-butyldiphenylsilyloxy-3-hydroxy-4-methyl-cyclohexane carboxylate **(34a)**

Hydroboration of the intermediate of example 1 was effected as follows. To a stirred solution of **33a** (130 mg, 0.319 mmol), in THF (12 mL) was added dropwise a $BH_3$ solution (380 uL, 1M in THF, 0.38 mmol) at 0°C and stirring was continued at this temperature for 2.5 hours. Then $H_2O_2$(0.5 mL) and saturated $NaHCO_3$ (3 mL) were added. After stirring for 0.5 hour the reaction solution was poured into a $H_2O$-EtOAc mixture and the organic layer was separated. The aqueous layer was extracted by means of EtOAc. The combined organic extracts were washed, dried and concentrated. The residue was purified by chromatography (using isooctane / EtOAc mixtures ranging from 9 : 1 to 4 : 1), thus affording intermediate **34a** (48 mg, 35%) which was characterized as follows: $R_f$ = 0.21 (isooctane/EtOAc, 4:1). - $[\alpha]_D^{r.t}$ =- 51.8 (c = 0.55. $CHCl_3$ - IR (film): v = 3361, 2932, 2858, 1737, 1589, 1403, 1428, 1363, 1282, 1250, 1173, 1111 $cm^{-1}$ -$^1H$ NMR ($CDCl_3$): = 7.70 - 7.36 (m, 10 H), 3.59 (s, 3 H), 3.25 (m, 1 H), 3.09 (m, 1 H), 2.12 - 2.05 (m, 2 H), 1.91 (dt, J = 12.7, 4.3 Hz, 1 H), 1.62 (br.s, 1 H), 1.54 -1.38 (m, 3 H), 1.07 (d, J = 6.4 Hz, 3 H), 1.05 (s, 9 H). - $^{13}C$ NMR ($CDCl_3$: = 174.6, 135.9, 134.3, 133.6, 129.7, 127.5, 74.8, 73.0, 51.8, 47.9, 37.9, 37.1, 36.8, 27.1, 19.5, 14.5. - MS (m/z, %): 409 ($M^+$ - $H_2O$ - H, 1), 369 ($M^+$- 57, 10), 337 (25), 309 (5), 199 (75), 153 (25), 121 (15), 93 (100). Elemental analysis: $C_{25}H_{34}O_4Si$ (426.63): calcd. C 70.38, H 8.03; found C 70.16, H 8.14.

Example 4 - preparation of methyl (1*R*,3*R*,4*S*,5*R*)-5-*t*-butyldiphenylsilyloxy-3-hydroxy-4-methyl-cyclohexane carboxylate **(35a)**

Starting from the intermediate of example 2, intermediate **35a** (61 mg, 45%) was prepared following the same hydroboration procedure as described in example 3, and characterized as follows: $R_f$ = 0.19 (isooctane/EtOAc, 4:1. - $[\alpha]_D^{r.t}$ =-38.5 (c = 0.69, $CHCl_3$ IR (film): ν = 3442, 2954, 2893, 2857, 1735, 1715, 1589, 1463, 1427, 1378, 1273, 1196, 1111 $cm^{-1}$. $^1H$ NMR ($CDCl_3$): δ= 7.68 - 7.36 (m, 10 H), 4.17 (dt, J = 10.8, 4.7 Hz, 1 H), 3.89 (d, J = 3.1 Hz, 1 H), 3.63 (s, 3 H), 2.62 (tt, J = 11.8, 4.4 Hz, 1 H), 1.82 - 1.66 (m, 5 H), 1.59 (br.s, 1 H), 1.06 (s, 9 H), 0.96 (d, J = 7.2 Hz, 3 H). - $^{13}C$ NMR ($CDCl_3$): = 175.7, 135.8, 134.3, 129.6, 127.5, 72.0, 68.9, 51.7, 41.3, 36.6, 31.4, 29.6, 27.0, 19.3, 10.7. - MS (m/z, %): 369 ($M^+$ - 57, 100), 339 (5), 319 (4), 273 (6), 253 (10), 199 (85), 153 (65), 135. 40). - Elemental analysis: $C_{25}H_{34}O_4Si$ (426.63): calcd. C 70.38, H 8.03; found C 70.21, H 8.20.

Example 5 -preparation of the ethyl homologue (34b)

The ethyl homologue (34b) of intermediate (34a) was prepared in a similar manner and characterized as follows: $R_f$ = 0.12 (isooctane/EtOAc, 4:1). -$[\alpha]_D^{r.t}$ = - 51.1 (c = 0.52, $CHCl_3$). - IR (film): ν = 3421, 2956, 2857, 1736, 1508, 1458, 1428, 1363, 1272, 1242 $cm^{-1}$. $^1H$ NMR ($CDCl_3$): δ = 7.69 - 7.36 (m. 10 H), 3.59 (s, 3 H), 3.43 (dt, J = 10.6, 4.3 Hz, 1 H), 3.33 (dt, J = 10.6, 4.3 Hz, 1 H), 2.05 (m, 2 H), 1.92 (d, J =12.4 Hz, 1 H), 1.78 (m, 1 H), 1.70 (m, 1 H) 1.54 - 1.41 (m, 4 H), 1.09 (s, 9 H), 0.69 (t, J = 7.5 Hz, 3 H). -$^{13}C$ NMR ($CDCl_3$): δ = 175.2, 136.5, 134.8, 133.9, 130.3, 130.1, 128.2, 127.9, 71.3, 69.8, 52.8, 52.3, 38.3, 37.5, 37.3, 27.6, 19.9, 19.1, 9.4. - MS (m/z, %): 383 ($M^+$ - 57, 2), 351 (8), 305 (24), 273 (5), 213 (9), 199 (70), 183 (15), 153 (20), 135 (28), 107 (100), 79 (30). Elemental analysis: $C_{26}H_{36}O_4Si$ (440.65): calcd. C 70.87, H 8.23; found: C 70.50, H 8.33.

Example 6 - preparation of the ethyl homologue (35b)

The ethyl homologue (35b) of intermediate (35a) was prepared in a similar manner and characterized as follows: $R_f$= 0.17 (isooctane/EtOAc, 4:1), $[\alpha]_D^{r.t}$ = - 38.9 (c = 0.81, $CHCl_3$). - IR (film): *ν* = 3453, 2958, 2858, 1736, 1589, 1460, 1428, 1382, 1255, 1172, 1195, 1110 $cm^{-1}$. $^1H$ NMR ($CDCl_3$): δ = 7.67 - 7.37 (m, 10 H), 4.18 (dt, J = 11.7, 4.6 Hz, 1 H), 4.02 (d, J = 2.8 Hz, 1 H), 3.63 (s, 3 H), 2.60 (m, 1 H), 1.95 (m, 1 H), 1.80 (dt, J = 12.6, 4.1 Hz, 1 H), 1.65 - 1.45 (m, 5 H), 1.05 (s, 9 H), 0.87 (m, 3 H). - $^{13}C$ NMR ($CDCl_3$): δ = 176.2, 136.3, 134.8, 134.7, 130.1, 130.0, 128.0, 69.3, 52.1, 49.1, 36.8, 32.6, 30.0, 27.4, 19.7, 17.6, 13.3. - MS (m/z, %): 383 ($M^+$- 57, 90), 351 (6), 333 (5), 305 (4), 273 (10), 213 (50), 199 (100), 183 (58), 153 (60), 135 (65), 107 (48). - Elemental analysis: $C_{26}H_{36}O_4Si$ (440.65): calcd. C 70.87, H 8.23; found C 70.75, H 8.33.

Example 7 - preparation of methyl (1*R*,3*R*,4*R*,5*R*)-5-*t*-butyldiphenvisilyloxy-3-hydroxy-4-methyl-cyclohexane carboxylate (41a)

Reference is made again to figure 4. To a stirred solution of intermediate **34a** (0.55 g, 1.29 mmol), *p*-$NO_2PhCO_2H$ (0.26 g, 1.56 mmol) and $Ph_3P$ (0.41 g, 1.56 mmol) in THF (15 mL) at room temp. was added dropwise DIAD (323 uL, purity 95%, 1.56 mmole). After stirring for 24 hours, the solution was subjected to flash chromatography (using a isooctane / EtOAc 7 : 3 mixture). After concentration, the residue was purified by chromatography (using a isooctane/EtOAc 92 : 8 mixture) to give intermediate **40a** (0.60 g, 81%) [characterized as follows: $R_f$ = 0.24 (isooctane/EtOAc, 4:1). - $[\alpha]_D^{r.t}$ = - 61.4 (c = 0.30, $CHCl_3$). IR (film): ν = 2957, 2858, 1729, 1608, 1529, 1461, 1428, 1349, 1273, 1243 $cm^{-1}$. - $^1H$ NMR ($CDCl_3$): δ = 8.23 (dd, J = 8.8, 1.4 Hz, 2 H), 7.83 (dd, J = 8.8, 1.4 Hz, 2 H), 7.75 (dd, J = 8.0, 1.4 Hz, 2H), 7.71 (dd, J = 8.0. 1.4 Hz. 2 H). 7.52 - 7.40 (m, 6 H), 5.33 (d, J = 2.2 Hz, 1H). 3.73 (dt, J = 110.6,4.2 Hz, 1 H), 3.60 (s, 3 H), 2.46 (tt, J = 9.1, 3.5 Hz, 1 H), 2.17 (m, 2 H), 1.86 (m, 1 H), 1.73 (dt, J = 12.5, 2.2 Hz, 1H). 1.68 (q, J = 12.5 Hz, 1H), 1.08 (s, 9 H), 0.94 (d, J = 6.8 Hz, 3 H). - $^{13}C$ NMR ($CDCl_3$): δ = 174.6, 163.6, 150.4, 136.2, 135.4, 134.4, 133.3, 130.5, 129.7, 127.6, 127.5, 123.5, 75.9, 72.4, 51.9, 42.5, 37.3, 37.2, 30.1, 27.1, 19.4, 14.8. - MS (m/z, %): 544 ($M^+$ - 31, 1), 518 ($M^+$ - 57, 31), 488 (2), 442 (1), 409 (1), 348 (19), 302 (11), 273 (10), 213 (68), 183 (48), 150 (100). Elemental analysis: $C_{32}H_{37}O_7NSi$ (575.73): calcd. C 66.76, H 6.48, N 2.43; found C 66.39, H 6.50, N 2.43]. A mixture of intermediate **40a** (0.59 g, 1.03 mmol) and $K_2CO_3$ (71 mg, 0.51 mmol) in a dry MeOH (12 mL) was stirred for 6 hours at room temperature and then poured into $H_2O$-EtOAc, separated, extracted, washed, dried and concentrated. Flash chromatography (using a isooctane/EtOAc 4 : 1 mixture) gave the intermediate **41a** (0.42 g, 95%) as a colourless oil characterized as follows: $R_f$= 0.21 (isooctane/EtOAc, 4:1). - $[\alpha]_D^{r.t}$ = - 72.5 (c = 0.50, $CHCl_3$). - IR (film): *ν* = 3500, 3071, 2956, 2858, 1736, 1462, 1428, 1195, 1111, 1084 $cm^{-1}$. $^1H$ NMR ($CDCl_3$): δ = 7.71 - 7.35 (m, 10 H), 3.99 (d, J = 3.5 Hz, 1 H)3.75 (dt, J = 10.5, 4.3 Hz, 1 H), 3.58 (s, 3 H), 2.59 (tt, J = 12.2, 3.6 Hz, 1 H), 1.97 - 1.91 (m, 2 H), 1.66 - 1.57 (m, 2 H), 1.49 (m, 1 H), 1.05 (s, 9 H), 1.02 (d, J = 6.9 Hz, 3 H). - $^{13}C$ NMR ($CDCl_3$): δ = 175.6, 135.9, 134.7, 133.9, 129.5, 127.5, 72.3, 71.1, 51.6, 43.6, 37.2, 36.4, 35.1, 27.1, 19.5, 14.9. - MS (m/z): 395 ($M^+$- 31,4), 370 (24), 369 ($M^+$- 57, 100), 337 (10), 291 (6), 259

(8), 221 (4), 215 (74), 199 (84), 183 (64), 153 (71), 105 (58), 77 (72).

Example 8-preparation of the ethyl homologue **41b**

The ethyl homologue **41b** of intermediate **41a** was prepared starting from intermediate **34b** via **40b** as described in example 7 and characterized as follows: $R_f$ = 0.22 (isooctane/EtOAc, 4:1), - $[\alpha]_D^{r.t}$= - 63.4 (c = 0.71, $CHCl_3$). -IR (film): v = 3506, 3071, 2956, 2858, 1736, 1472, 1428, 1361, 1266, 1176, 1110 $cm^{-1}$. $^1H$ NMR ($CDCl_3$): δ = 7.67 - 7.35 (m, 10 H), 4.2 (d, J = 3.2 Hz, 1 H), 3.78 (m, 1 H), 3.58 (s, 3 H), 2.57 (m, 1 H), 1.94 (dt, J = 13.1, 3.6 Hz, 2 H), 1.60 - 1.50 (m, 2 H), 1.43 (br.s, 1 H), 1.33 (m, 1 H), 1.13 (m, 2 H), 1.05 (s, 9 H), 0.86 (t, J = 7.5 Hz, 3 H). - $^{13}$ C NMR ($CDCl_3$): δ = 175.6, 136.0, 129.6, 129.5, 127.5, 127.4, 71.5, 66.4, 51.6, 50.2, 37.2, 36.2, 35.0, 27.1, 19.7, 19.5, 11.3. - MS (m/z, %): 383 ($M^+$- 57, 100), 351 (6), 305 (4), 273 (8), 199 (90), 153 (45), 135 (50), 77 (65), 57 (68). - Elemental analysis: $C_{26}H_{36}O_4Si$ (440.65): calcd. C 70.87, H 8.23; found C 71.02, H 8.20.

Example 9 - preparation of (3a*R*,5a*R*)-1-methyl-4-oxo-5-oxa-bicyclo[3,2,1]-1-octene **47**

Reference is made to figure 4. A solution of the hydroxy intermediate prepared from **30** (0.36 g, 2.12 mmol) and PPTS (0.49 g, 1.95 mmol) in benzene (25 mL) was stirred at 80°C for 24 hours. The reaction mixture was cooled, diluted with $Et_2O$, washed (saturated $NaHCO_3$ and brine), dried and evaporated. The residue was purified by flash chromatography (using a isooctane / EtOAc 4 : 1 mixture) affording intermediate **47** (0.19 g, 85%) as colorless crystals in *n*-hexane/acetone, and characterized as follows: m.p = 47 - 48 °C. - $R_f$ = 0.21 (isooctane/EtOAc, 9:1). - $[\alpha]_D^{r.t}$ = + 130.1 (c = 0.89, $CHCl_3$). - IR (film): v = 2915, 2855, 1778, 1449, 1436, 1219, 1147, 1116 $cm^{-1}$. $^1H$ NMR ($CDCl_3$): δ = 5.43 (br.s, 1 H), 4.53 (d, J = 5.2 Hz, 1 H), 2.85 (t, J = 3.5 Hz, 1 H), 2.48 - 2.33 (m, 3 H), 2.06 (d, J = 11.1 Hz, 1 H), 1.81 (s, 3 H). $^{13}C$ NMR ($CDCl_3$): δ = 179.5, 137.5 ,122.2, 78.0, 37.6, 34.4, 28.4, 21.1. - MS (m/z, %): 165 ($M^+$+ 1, 1), 155 (1), 139 (4), 139 (23), 119 (4), 109 (10), 94 (30), 79 (100).

Example 10 - preparation of (1*S*,2*R*,3a*R*,5a*R*)-2-hydroxyl-1-methyl-4-oxo-5-oxa-bicyclo[3,2,1]octane **48**

This synthesis is effected through the hydroboration procedure of example 3 and achieves, with a yield of 42%, colourless crystals from *n*-hexane/acetone which were characterized as follows: M.p = 128 - 129 °C; $R_f$ = 0.21 (isooctane / EtOAc 1:1); $[\alpha]_D^{r.t}$ = - 109.3 (c = 0.97, $CHCl_3$). - IR (film): v = 3448, 2933, 1766, 1359, 1273, 1227 $cm^{-1}$. $^1H$ NMR ($CDCl_3$): δ = 4.56 (d, J = 5.9 Hz, 1 H), 3.57 (m, 1 H), 2.70 (br.s, 1 H), 2.41 (m, 1 H), 2.32 (m, 1 H), 2.16 (br.s, 1 H), 1.84 (d, J = 11.7 Hz, 1 H), 1.65 (m, 1 H), 1.58 (m, 1 H), 1.19 (d, J = 6.9 Hz, 3 H). $^{13}C$ NMR ($CDCl_3$): δ = 178.3, 82.8, 71.5, 42.4, 37.9, 37.6, 35.5, 16.1. - MS (m/z, %): 182 ($M^+$, 1), 161 (5), 154 (4), 128 (14), 113 (48), 97 (54), 67 (50), 55 (100). Elemental analysis: $C_8H_{12}O_3$ (156.18): calcd. C 61.52, H 7.74; found C 59.92, H 7.78.

Example 11 - preparation of ethyl (1*S*,3*R* 4*R*,5*R*)-3-*t*-butyldiphenylsilyloxy-6-hydroxy-5-methyl-cyclohexane carboxylate **50**

Reference is made to figure 4. Intermediate **50** was prepared in two steps from intermediate **48**, yield 86%, and characterized as follows: $R_f$ = 0.21 (isooctane/EtOAc, 4:1). - $[\alpha]_D^{r.t}$ = - 7.1 (c = 0.41, $CHCl_3$). - IR (film): v = 3445, 2953, 2858, 1734, 1684, 1653; 1559, 1540, 1428, 1362, 1256, 1195 $cm^{-1}$. $^1H$ NMR ($CDCl_3$): δ = 7.66 - 7.35 (m, 10 H), 4.25 (dt, J = 10.7, 6.7 Hz, 1 H), 3.94 (m, 1 H), 3.63 (s, 3 H), 2.92 (m, 1 H), 1.98 (m, 1 H), 1.93 (dt, J = 10.1, 6.5 Hz, 1 H), 1.68 - 1.60 (m, 3 H), 1.05 (s, 9 H), 0.77 (d, J = 7.3 Hz, 3 H). $^{13}C$ NMR ($CDCl_3$): δ = 176.0, 135.7, 133.9, 129.7, 127.6, 73.0, 68.3, 51.8, 41.1, 36.9, 31.4, 30.5, 26.9, 19.3, 10.5. - MS (m/z, %): 369 ($M^+$ - 57, 14), 337 (45), 291 (8), 259 (11), 199 (92), 169 (83), 137 (100), 93 (47). - Elemental analysis: $C_{25}H_{34}O_4Si$ (426.63): calcd. C 70.38, H 8.03; found C 69.93, H 8.22.

Example 12 - preparation of (4*S*,6*S*)-6-*t*-butyldiphenylsilyloxy-1-methyl methoxycarbonyl-1-cyclohexene **56a**

Reference is made to figure 5. Intermediate 56a was prepared in three steps from intermediate **30** via intermediates **53** and **54** as described before. Product was obtained with a yield of 81% and characterized as follows: $R_f$ = 0.30 (isooctane/EtOAc, 9:1); $[\alpha]_D^{r.t}$= + 81.9 (c =1.91, $CHCl_3$). - IR (film): v = 3070, 2952, 2855, 1738, 1472, 1433, 1428, 1362, 1308, 1247 $cm^{-1}$ - $^1H$ NMR ($CDCl_3$): δ = 7.73 - 7.36 (m, 10 H), 5.39 (t, J = 1.8 Hz, 1 H), 4.24 (br.s, 1 H), 3.59 (s, 3 H), 2.38 (m, 1 H), 2.22 - 2.04 (m, 3 H), 1.75 (m, 1 H), 1.66 (s, 3 H), 1.07 (s, 9 H). - $^{13}C$ NMR($CDCl_3$): δ = 175.1, 137.4, 134.4, 133.7, 129.6, 127.6, 121.9, 71.4, 51.6, 38.8, 36.2, 27.9, 27.1, 20.2, 19.5. - MS (m/z, %): 408 ($M^+$, 1), 387 (6), 351 ($M^+$- 57, 95), 319 (6), 299 (5), 273 (6), 227 (5), 213 (100), 183 (75), 137 (50), 77 (65).

Example 13 - preparation of the ethyl homologue **56b**

Using a procedure similar to that of example 12, the ethyl homologue of (isooctane/EtOAc, 9:1). $[\alpha]_D^{r.t}$ = + 74.2 (c = 1.09, $CHCl_3$). - IR (film): v = 2959, 2857, 1738, 1652, 1589, 1456, 1428, 1388, 1246 $cm^{-1}$. $^1H$ NMR ($CDCl_3$): δ = 7.72 - 7.38 (m, 10 H), 5.37 (m, 1 H), 4.26 (br.s, 1 H), 3.58 (s, 3 H), 2.35 (m, 1 H), 2.30 -1.98 (m, 5 H), 1.75 (m, 1 H), 1.05 (s, 9 H), 0.87 (t, J = 7.4 Hz, 3 H). - $^{13}C$. NMR ($CDCl_3$): δ = 175.1, 136.7, 134.4, 133.5, 129.5, 127.1, 119.7, 70.2, 51.5, 38.7, 35.3, 27.7, 27.0, 25.3,19.4, 12.3. - MS (m/z, %): 422 ($M^+$, 2), 401 (3), 365 (88), 333 (8), 287 (9), 255 (3), 227 (7), 213 (90), 183 (50), 137 (58), 107 (45), 79 (100).

Example 14 - preparation of ethyl (1*S*,35,4*R*,5*S*)-3-*t*-butyldiphenylsilyloxy-5-hydroxy-4-methylcyclohexane carboxylate **58a**

Intermediate **58a** was obtained by performing the hydroboration of the intermediate of example 12 under the same conditions as in example 3, and was characterized as follows: $R_f$ = 0.18 (isooctane/EtOAc, 4:1). - $[\alpha]_D^{r.t}$ = + 40.5 (c = 0.66, $CHCl_3$). - IR (film): v = 3452, 3070, 2953, 2857, 1735, 1717, 1427, 1379, 1272, 1195 $cm^{-1}$. - $^1H$ NMR ($CDCl_3$): δ = 7.68 - 7.36 (m, 10 H), 4.17 (dt, J = 10.8, 4.7 Hz, 1 H), 3.89 (d, J = 2.7 Hz, 1 H), 3.63 (s, 3 H), 2.62 (m, 1 H), 1.81 - 1.67 (m, 5 H), 1.56 (br.s, 1 H), 1.06 (s, 9 H), 0.96 (d, J = 7.2 Hz, 3 H). - $^{13}C$ NMR ($CDCl_3$): δ = 175.7, 135.8, 134.4, 129.7, 127.6, 72.0, 68.9, 56.9, 51.7, 41.3, 36.6, 31.4, 29.6, 27.0, 19.3, 10.6. - MS (m/z, %): 425 ($M^+$-1, 1),385 (2), 369 ($M^+$- 57, 100), 337 (5), 291 (4), 259 (10), 221 (4), 199 (85), 153 (65).

Example 15 - preparation of ethyl (1*S*,3*S*,4*S*,5*R*)-3-*t*-butyldiphenylsilyloxy-5 hydroxy-4-methyl-cyclohexane carboxylate **60a**

Reference is made to figure 5 and the general procedure described herein-above. Intermediate **60a** was characterized as follows: $R_f$ = 0.15 (isooctane/EtOAc, 4:1). - $[\alpha]_D^{r.t}$= + 51.2 (c = 0.53, $CHCl_3$). - IR (film): v = 3444, 3070, 2952, 2857, 1736, 1459, 1427, 1361, 1282, 1249, 1172 $cm^{-1}$. - $^1H$ NMR ($CDCl_3$): δ = 7.69 - 7.36 (m, 10 H), 3.59 (s, 3 H), 3.25 (m, 1 H), 3.08 (m, 1 H), 2.11 - 2.05 (m, 2 H), 1.92 (d, J = 13.0 Hz, 1 H), 1.53 -1.44 (m, 4 H), 1.08 (d, J = 6.4 Hz, 3 H), 1.05 (s, 9 H). - $^{13}C$ NMR ($CDCl_3$): δ = 174.5, 135.9, 134.3, 133.6, 129.7, 129.6, 127.6, 127.5, 74.8, 73.0, 51.8, 47.9, 37.9, 37.1, 36.8, 30.1, 27.0, 19.4, 14.5. - MS (m/z, %): 369 ($M^+$- 57, 5), 337 (8), 291 (45), 259 (4), 247 (3), 199 (72), 153 (28), 121 (25), 93 (100).

Example 16 - preparation of the ethyl homologue **58b**

Using a procedure similar to that of example 14, the ethyl homologue of intermediate **58a** was made and characterized as follows: $R_f$ = 0.17 (isooctane/EtOAc, 4:1). - $[\alpha]_D^{r.t}$ = + 38.0 (c = 1.13, $CHCl_3$). - IR (film): v = 3453, 2958, 2858, 1736, 1589, 1460, 1428, 1382, 1255 $cm^{-1}$. - $^1H$ NMR ($CDCl_3$): δ = 7.67-7.37 (m, 10 H), 4.18 (dt, J = 11.7,4.6 Hz, 1 H), 4.02 (d, J = 2.8 Hz, 1 H), 3.63 (s, 3 H), 2.60 (m, 1 H), 1.95 (m, 1 H), 1.80 (dt, J = 12.6, 4.1 Hz, 1 H), 1.65 - 1.45 (m, 6 H), 1.05 (s, 9 H), 0.87 (m, 3 H). - $^{13}C$ NMR ($CDCl_3$): δ = 176.2, 136.3, 134.8, 134.7, 130.1, 130.0, 128.0, 69.3, 52.1, 49.1, 36.8, 32.6, 30.0, 27.4, 19.7, 17.6, 13.3. - MS (m/z, %): 383 ($M^+$ - 57, 100), 351 (4), 287 (6), 273 (7), 213 (55), 199 (95), 183 (55), 153 (50), 107 (35), 55 (86).

Example 17 - preparation of the ethyl homologue **60b**

Using a procedure similar to that of example 15, the ethyl homologue of intermediate **60a** was made and characterized as follows: $R_f$ = 0.12 (isooctane/EtOAc, 4:1). - $[\alpha]_D^{rt}$ = + 50.7 (c = 0.52, $CHCl_3$). - IR (film): v = 3421, 2956, 2857, 1736, 1508, 1458, 1428, 1363, 1272, 1242, 1169, 1110, 1040, 1007, 866, 822, 740, 703, 612 $cm^{-1}$. $^1H$ NMR ($CDCl_3$): δ = 7.69 - 7.36 (m, 10 H), 3.59 (s, 3 H), 3.43 (dt, J = 10.6, 4.3 Hz, 1 H), 3.33 (dt, J = 10.6, 4.3 Hz, 1 H), 2.05 (m, 2 H), 1.92 (d, J = 12.4 Hz, 1 H), 1.78 (m, 1 H), 1.70 (m, 1 H) 1.54 -1.41 (m, 3 H), 1.09 (s, 9 H), 0.89 (m, 1 H), 0.69 (t, J = 7.5 Hz, 3 H). $^{13}C$ NMR ($CDCl_3$): δ = 175.2, 136.5, 134.8, 133.9, 130.3, 130.1, 128.2, 127.9, 71.3, 69.8, 52.8, 52.3, 38.3, 37.5, 37.3, 27.6, 19.9, 19.1, 9.4. - MS (m/z, %): 383 ($M^+$ 57,3), 351 (9), 305 (32), 273 (6), 199 (64), 183 (15), 153 (18), 135 (35), 107 (100), 79 (35).

Example 18 - alternative preparation of methyl (1*S*,3*S*,4*R*,5*S*)-3-t butyldiphenylsilyloxy-4-methyl-cyclohexane carboxylate **58a**

Reference is made to figure 5. To a stirred solution of intermediate **54** (0.21 g, 0.49 mmol), $Et_3N$ (172 uL, 1.23 mmol) and DMAP (6 mg, 0.05 mmol) in $CH_2Cl_2$ (8 mL) was added *p*-nitrobenzenesulfonyl chloride (136 mg, 0.61 mmol) at 0°C and stirring was continued for 10 hours. The solution was submitted to flash chromatography (using a isooctane

/ EtOAc 9 : 1 mixture) to give intermediate **55** (0.27 g, 90%) characterized as follows: $R_f$ = 0.27 (isooctane/EtOAc, 4:1). - $[\alpha]_D^{r.t}$ = - 3.2 (c = 0.40, $CHCl_3$). - IR (film): ν = 3072, 2955, 2858, 1737, 1608, 1535, 1428, 1351, 1288, 1251, 1187 $cm^{-1}$. $^1H$ NMR ($CDCl_3$): δ = 8.35 (dd, J = 7.1, 1.7 Hz, 2 H), 7.99 (dd, J = 7.1, 1.7 Hz, 2 H), 7.60 - 7.35 (m, 10 H), 4.39 (dt, J = 12.1, 4.7 Hz, 1 H), 3.63 (s, 3 H), 3.62 (m, 1 H), 2.17 (m, 1 H), 2.05 (dt, J = 12.9, 1.7 Hz, 1 H), 1.88 (m, 1 H), 1.79 (m, 1 H), 1.73 (m, 1 H), 1.66 (m, 1 H), 1.03 (s, 9 H), 1.00 (d, J = 6.9 Hz, 3 H). - $^{13}C$ NMR ($CDCl_3$): δ = 173.4, 150.5, 142.9, 135.6, 133.4, 129.9, 128.8, 127.7, 124.4, 81.9, 70.6, 52.1, 39.5, 37.4. 30.1, 27.8, 26.8, 19.1, 5.4. - MS (m/z, %): 554 ($M^+$ 57, 21), 494 (2), 416 (3), 384 (57), 351 (32), 304 (9), 258 (7), 213 (67), 153 (49), 93 (100). - Elemental analysis: $C_{31}H_{37}O_8SiNS$ (611.79): calcd. C 60.86, H 6.09, N 2.29, found C 61.09, H 6.30, N 2.29.

To a stirred solution of intermediate **55** (0.22 g, 0.36 mmole) and 18-crown-6-ether (476 mg, 1.80 mmole) was added freshly prepared $EtCO_2Cs$ (371 mg, 1.80 mmole). The mixture was stirred at 110°C for 2.5 hours and then cooled to room temperature. The mixture was diluted with EtOAc, washed and concentrated. Column chromatography (using a isooctane / EtOAc 100 : 4 mixture) afforded intermediate **57a** (95 mg, 55%) characterized as follows: $R_f$ = 0.26 (isooctane:EtOAc, 4:1). - $[\alpha]_D^{r.t}$ = + 30.7 (c = 0.79, $CHCl_3$). - IR (film): ν = 3049, 2954, 2858, 1738, 1463, 1428, 1274, 1189, 1112 $cm^{-1}$. $^1H$ NMR ($CDCl_3$): δ = 7.67 - 7.34 (m, 10 H), 4.90 (d, J = 2.9 Hz, 1 H), 3.98 (dt, J = 11.6, 4.7 Hz, 1 H), 3.65 (s, 3 H), 2.48 (m, 1 H), 2.14 - 1.99 (m, 2 H), 1.93 (m, 1 H), 1.87 (dt, J = 12.8, 4.2 Hz, 1 H), 1.93 - 1.81 (m, 3 H), 1.06 (s, 9 H), 1.04 (d, J = 7.3 Hz, 3 H), 0.97 (t, J = 7.6 Hz, 3 H). - $^{13}C$ NMR ($CDCl_3$): δ = 175.1, 173.1, 135.8, 133.9, 129.7, 127.5, 74.1, 69.1, 51.8, 38.0, 37.3, 30.9, 27.7, 26.9, 26.7, 19.2, 9.9, 9.0. - MS (m/z): 451 ($M^+$ - 31, 2), 425 ($M^+$ - 57, 21), 386 (1), 351 (15), 291 (3), 255 (27), 199 (29), 183 (19), 135 (21), 93 (26). Elemental analysis: $C_{28}H_{38}O_5Si$ (482.69): calcd. C 69.67, H 7.94; found C 69.82, H 7.82.

Finally, the methanolysis of intermediate **57a** provided intermediate **58a** in a 98% yield.

Example 19 - mesylate formation

The following general procedure was used for mesylate formation: to a stirred solution of a hydroxy compound and $Et_3N$ (1.5 equivalent) in $CH_2Cl_2$ (0.04 - 0.05 mmol/mL) was added dropwise mesityl chloride (1.2 equivalent) at 0°C and stirring was continued for 3 hours. The resulting solution was subjected to flash chromatography (using a isooctane / EtOAc 7 : 3 mixture). The residue was purified by HPLC (using a isooctane / EtOAc 9 : 1 mixture) to give the corresponding mesylate (yield circa 95%).

Example 20 -formation of 3a-carbomethoxy-bicyclo[3,1,0]hexane

The following general procedurewas used for the formation of 3a-carbomethoxy-bicyclo[3,1,0]hexane: to a stirred solution of a mesylate obtained for instance according to example 19 (0.03 - 0.05 mmol/mL) in a tBuOH-THF mixture (3 : 2) was added dropwise tBuOK (1 M in tBuOH, 1.2 equivalent) at 45- 50°C and stirring was continued for 0.5 hour. The solution was poured into $H_2O$-EtOAc and then the organic layer was separated. The aqueous layer was extracted with EtOAc. The combined organic extracts were washed, dried and concentrated. The residue was purified by flash chromatography (using a isooctane / EtOAc 100 : 3 mixture) affording the bicyclic product with a yield of about 70%.

Example 21 - preparation of (1*S*,2*R*,3a*S*,4a*S*)-2-*t*-butyldiphenylsilyloxy-3a-carbomethoxy-1-methyl-bicyclo[3.1.0] hexane **38a** and its enantiomer **66a**

Reference is made to figure 4. Using the above described general procedures, intermediate **38a** was made starting from intermediate **37a** and characterized as follows: $R_f$ = 0.24 (isooctane / EtOAc 97 : 3 mixture) ; $[\alpha]_D^{r.t}$ = 53.8 (c = 0.65, $CHCl_3$). - IR (film): ν = 2957, 2858, 1724, 1472, 1428, 1367, 1292, 1222, 1149 $cm^{-1}$. $^1H$ NMR ($CDCl_3$): δ = 7.65 - 7.36 (m, 10 H), 3.84 (m, 1 H), 3.64 (s, 3 H), 2.24 (dd, J = 12.5, 8.5 Hz, 1 H), 2.03 (dd, J = 13.5, 6.6 Hz, 1 H). 1.93 (dd. J = 12.7, 7.2 Hz, 1 H), 1.59 (m, 1 H), 1.18 (dd, J = 8.4, 4.8 Hz, 1 H), 1.05 (d, J = 6.9 Hz, 3 H), 1.05 (s, 9 H), 0.56 (t, J = 5.2 Hz, 1 H). - $^{13}C$ NMR ($CDCl_3$): δ = 174.9, 135.7, 133.9, 129.6, 127.6, 72.9, 51.8, 37.2, 34.1, 33.3, 27.2, 27.0, 19.4, 19.5, 15.1. - MS (m/z, %): 408 ($M^+$, 1), 353 ($M^+$- 57, 21), 351 (87), 296 (13), 237 (8), 213 (100), 183 (58), 135 (61), 77 (67). Elemental analysis: $C_{25}H_{32}O_3Si$ (408.61): calcd. C 73.49, H 7.89; found C 73.57, H 8.04.

Referrring to figure 5, the corresponding enantiomer **66a** was similarly made from intermediate **59a** and characterized as follows: - $[\alpha]_D^{r.t}$ = + 52.2 (c = 0.76, $CHCl_3$).

Example 22 - preparation of the ethyl homologues **38b** and **66b**

Using a similar procedure (figure 4), the ethyl homologue of intermediate **38a** was made and characterized as follows: $R_f$ = 0.28 (isooctane / EtOAc 95 : 5 mixture). - $[\alpha]_D^{r.t}$ = - 36.2 (c = 1.04, $CHCl_3$). - IR (film): ν = 2958, 1724, 1428, 1366, 1233, 1158 $cm^{-1}$. - $^1H$ NMR ($CDCl_3$): δ = 7.67 - 7.38 (m, 10 H), 3.87 (1 H, q, J = 7.2 Hz), 3.64 (s, 3 H), 2.18 (m,

1 H), 1.92 (m, 1 H), 1.75 (m, 1 H), 1.56 (s, 2 H), 1.22 (m, 2 H), 1.05 (s, 9 H), 0.96 (t, J = 7.4 Hz, 3 H), 0.55 (t, J = 5.1 Hz, 1 H). - $^{13}C$ NMR ($CDCl_3$): δ = 175.1, 135.8, 135.7, 134.0, 129.8, 127.7, 73.1, 51.8, 44.1, 34.3, 31.4, 27.5, 27.0, 21.3, 19.3, 18.5, 12.0. - MS (m/z, %): 422 ($M^+$, 27), 365 ($M^+$ - 57, 26), 337 (3), 287 (5), 213 (40), 199 (52), 153 (22), 135 (100), 79 (35), 57 (25). Elemental analysis: $C_{26}H_{34}O_3Si$ (422.63): calcd. C 73.89, H 8.11; found C 73.72, H 8.31.

Referrring to figure 5, the corresponding enantiomer **66b** was similarly made and characterized as follows: - $[\alpha]_D^{rt}$ = + 36.5 (c = 1.07, $CHCl_3$).

Example 23 - preparation of (1*R*,2*R*,3a*S*,4a*S*)-2-*t*-butyldiphenylsilyloxy-3a-carbomethoxy-1-methyl-bicyclo [3.1.0] hexane **43a** and its enantiomer **62a**

Reference is made to figure 4. Using the above described general procedures, intermediate **43a** was made starting from intermediate **42a** and characterized as follows: $R_f$ = 0.21 (isooctane / EtOAc 100 : 3 mixture). - $[\alpha]_D^{r.t}$ = - 100.4 (c = 1.15, $CHCl_3$). - IR (film): ν = 3048, 2956, 2858, 1727, 1589, 1472, 1428, 1369, 1346, 1259, 1199 $cm^{-1}$. - $^1H$ NMR ($CDCl_3$): δ = 7.65 - 7.35 (m, 10 H), 3.63 (s, 3 H), 3.30 (dd, J = 12.3, 7.4 Hz, 1 H), 2.27 - 2.19 (m, 2 H), 1.99 (dd. J = 12.8. 7.1 Hz. 1 H). 1.78 (dt. J = 8.6.5.2 Hz. 1 H), 1.07 (m, 1 H), 1.04 (s, 9 H), 0.87 (d, J = 6.6 Hz, 3 H), 0.45 (t, J = 5.1 Hz, 1 H). - $^{13}C$ NMR (CDCl): δ = 174.8, 135.9, 134.0, 129.6, 127.5, 77.4, 51.6, 41.7, 36.4, 32.3, 26.9, 26.7, 19.2, 15.9, 15.5. - MS (m/z, %): 408 ($M^+$, 5), 377 (6), 351 ($M^+$ - 57, 65), 317 (6), 273 (5), 225 (4), 213 (100), 183 (43), 135 (40), 84 (72). - Elemental analysis: $C_{25}H_{32}O_3Si$ (408.61): calcd. C 73.49, H 7.89; found C 73.32, H 8.01.

Referrring to figure 5, the corresponding enantiomer **62a** was similarly made from intermediate **61a** and characterized as follows: - $[\alpha]_D^{r.t}$ = + 99.5 (c = 0.72, $CHCl_3$). Elemental analysis : $C_{25}H_{32}O_3Si$ (408.61): calcd. C 73.49, H 7.89; found C 73.47, H 8.0.6.

Example 24 - preparation of the ethyl homologues **58b** and **62b**

Using a procedure (figure 4) similar to that of example 23, the ethyl homologue of intermediate **43a** was made and characterized as follows: $R_f$ = 0.28 (isooctane/EtOAc, 95:5). - $[\alpha]_D^{r.t}$ = - 102.9 (c = 0.65, $CHCl_3$). - IR (film): ν = 2957, 2857, 1725, 1589, 1461, 1428, 1371, 1327, 1262, 1197, 1154 $cm^{-1}$. - $^1H$ NMR ($CDCl_3$): δ = 7.68 - 7.35 (m, 10 H), 3.63 (s, 3 H), 3.37 (q, J = 8.1 Hz, 1 H), 2.21 (m, 2 H), 2.09 (m, 1 H), 1.98 (dd, J = 12.8, 7.1 Hz, 1 H), 1.86 (dt, J = 8.6, 5.1 Hz, 1 H), 1.08 (q, J = 5.0 Hz, 2 H), 1.05 (s, 9 H), 0.90 (t, J = 7.1 Hz, 3 H), 0.45 (t, J = 5.1 Hz, 1 H). - $^{13}C$ NMR ($CDCl_3$): δ = 175.2, 136.2, 134.3, 129.9, 127.8, 127.7, 76.4, 51.9, 49.3, 36.6, 30.4, 27.3, 26.9, 24.2, 19.5, 16.5, 13.0. - MS (m/z, %): 422 ($M^+$, 1), 407 (2), 365 ($M^+$- 57, 75), 309 (4), 213 (90), 199 (20), 183 (35), 135 (30), 77 (45), 41 (48). Elemental analysis: Calcd. for $C_{26}H_{34}O_3Si$ (422.63): C 73.89; H 8.11; found C 73.70, H 8.25.

Referrring to figure 5, the corresponding enantiomer **62b** was similarly made from intermediate **61b** and characterized as follows: - $[\alpha]_D^{r.t}$ = + 103.5 (c = 0.72, $CHCl_3$).

Example 25 - preparation of (1*R*,2*R*,3a*R*,4a*R*)-2-*t*-Butyldiphenylsilyloxy-3a-carbomethoxy-1-methyl-bicyclo [3.1.0] hexane **45a** and its enantiomer **64a**

Reference is made to figure 4. Using the above described general procedures, intermediate **45a** was made starting from intermediate **36a** and characterized as follows: $R_f$ = 0.21 (isooctane / EtOAc 100 : 3 mixture). - $[\alpha]_D^{r.t}$ = + 7.1 (c = 0.58, $CHCl_3$). - IR (film): ν = 3071, 2956, 2858, 1724, 1589, 1472, 1428,1390,1366, 1292, 1222, 1190, 1151, 1112 $cm^{-1}$. $^1H$ NMR ($CDCl_3$): δ = 7.62 - 7.36 (m, 10 H), 3.89 (d, J = 6.3 Hz, 1 H), 3.64 (s, 3 H), 2.45 (dd, J = 14.3, 6.2 Hz, 1 H), 2.03 (m, 1 H), 1.94 (d, J = 14.3 Hz, 1 H), 1.69 (d, J = 1.3 Hz, 1 H), 1.66 (t, J = 7.7 Hz, 1 H), 1.47 (m, 1 H), 1.05 (s, 9 H), 0.66 (d, J = 7.4 Hz, 3 H). - $^{13}C$ NMR ($CDCl_3$): δ = 175.4, 135.9, 134.2, 129.8, 127.8, 80.7, 51.8, 44.7, 36.7, 35.9, 30.9, 27.1, 20.9, 19.4, 19.0. - MS (m/z, %): 408 ($M^+$, 4), 377 (7), 351 ($M^+$ - 57, 43), 319 (7), 273 (8), 245 (16), 199 (47), 153 (41), 121 (100), 77 (58). Elemental analysis: $C_{25}H_{32}O_3Si$ (408.61): calcd. C 73.49, H 7.89; found C 73.36, H 8.01.

Referrring to figure 5, the corresponding enantiomer **64a** was similarly made from intermediate **61a** and characterized as follows: - $[\alpha]_D^{r.t}$ = - 7.4 (c = 0.70, $CHCl_3$). Elemental analysis : $C_{25}H_{32}O_3Si$ (408.61): calcd. C 73.49, H 7.89; found C 73.67, H 8.02.

Example 26 - preparation of the ethyl homologues **45b** and **64b**

Using a similar procedure (figure 4) to that of example 25, the ethyl homologue of intermediate **45a** was made and characterized as follows: $R_f$ = 0.26 (isooctane / EtOAc 95 : 5 mixture). - $[\alpha]_D^{r.t}$ = + 16.1 (c = 1.05, $CHCl_3$). - IR (film): ν = 2959, 2858,1723, 1560, 1428, 1365, 1297, 1274, 1220, 1150 $cm^{-1}$.-$^1H$ NMR ($CDCl_3$): δ = 7.67 - 7.37 (m, 10 H), 3.96 (d, J = 6.6 Hz, 1 H), 3.64 (s, 3 H), 2.37 (m, 1 H), 1.94 (d, J = 14.2 Hz, 1 H), 1.83 (t, J = 7.3 Hz, 1 H), 1.73 (m, 1 H), 1.68 (t, J = 5.2 Hz, 1 H), 1.48 (m, 1 H), 1.03 (s, 9 H), 0.94 (m, 2 H), 0.63 (t, J = 7.5 Hz, 3 H). - $^{13}C$ NMR ($CDCl_3$): δ = 174.8, 135.5, 135.4, 129.2, 127.2, 127.1, 78.5, 51.2, 35.9, 34.2, 30.2, 29.6, 26.5, 26.1, 20.3, 18.5, 11.3. - MS (m/z, %): 422

($M^+$, 2), 365 ($M^+$ - 57, 82), 213 (100), 199 (20), 183 (40), 153 (10), 135 (60), 77 (42), 49 (70). Elemental analysis: $C_{26}H_{34}O_3Si$ (422.63): calcd. C 73.89, H 8.11; found C 73.77, H 8.19.

Referrring to figure 5, the corresponding enantiomer **64b** was similarly made from intermediate **61b** and characterized as follows: - $[\alpha]_D^{r.t}$ = -15.9 (c = 0.70, $CHCl_3$).

Example 27 - preparation of (1*R*,2*S*,3a*S*,4a*S*)-2-*t*-butyldiphenylsilyloxy-3a carbomethoxy-1-methyl-bicyclo [3.1.0] hexane **68a** and its enantiomer **70a**

Referring to figure 6, intermediate **68a** was made from intermediate **30** and characterized as follows: $R_f$ = 0.33 (isooctane / EtOAc 9 : 1 mixture). - $[\alpha]_D^{r.t}$ = -13.2 (c = 1.61 $CHCl_3$). - IR (film): ν = 2931, 1724, 1428, 1288, 1224, 1147 $cm^{-1}$. - $^1H$ NMR ($CDCl_s$): δ = 7.63 - 7.36 (m, 10 H), 4.19 (t, J = 6.0 Hz, 1 H), 3.62 (s, 3 H), 2.30 (m, 2 H), 1.97 (d, J = 14.2 Hz, 1 H), 1.85 (m, 1 H), 1.64 (t, J = 4.6 Hz, 1 H), 1.35 (dd, J = 8.7, 3.9 Hz, 1 H), 1.09 (s, 9 H), 0.99 (d, J = 6.9 Hz, 3 H). - $^{13}C$ NMR (CDCl3): δ = 175.1, 136.0, 134.1, 133.6, 129.5, 127.5, 75.0, 51.4, 40.4, 38.1, 35.4, 29.5, 27.0, 19.2, 18.4, 12.9. - MS (m/z, %): 408 ($M^+$), 377, 351, 319, 273, 245, 199, 158, 153, 121 (100), 77, 57.

The corresponding enantiomer **70a** was also made and characterized as follows: - $[\alpha]_D^{r.t}$ = + 13.9 (c = 0.65, $CHCl_3$). Elemental analysis: $C_{25}H_{32}O_3Si$ (408.61): calcd. C 73.49, H 7.89; found C 73.41, H 8.13.

Example 28 - preparation of the ethyl homologues **68b** and **70b**

Using a similar procedure (figure 6) to that of example 27, the ethyl homologue of intermediate **68a** was made and characterized as follows: $R_f$ = 0.28 (cyclohexane / EtOAc 94 : 6 mixture). - $[\alpha]_D^{r.t}$= - 29.4 (c = 0.61, $CHCl_3$). - IR (film): ν = 2958, 1723, 1427, 1366, 1298, 1224, 1148 $cm^{-1}$. - $^1H$ NMR ($CDCl_3$): δ = 7.63 - 7.35 (m, 10 H), 4.18 (t, J = 5.9 Hz, 1 H), 3.61 (s, 3 H), 2.26 (m, 1 H), 2.06 (m, 1 H), 1.98 (d, J = 14.3 Hz, 1 H), 1.92 (m, 1 H), 1.67 (t, J = 4.3 Hz, 1 H), 1.48 (m, 2 H), 1.36 (dd, J = 12.1, 3.9Hz, 1 H), 1.05 (s, 9 H), 0.89 (t, J = 7.4 Hz, 3 H). - $^{13}C$ NMR ($CDCl_3$): δ = 175.3, 136.1, 134.3, 133.5, 129.6, 127.5, 74.6, 51.6, 48.3, 37.8, 33.3, 29.9, 27.1, 20.9, 19.3, 18.5, 13.3. - MS (m/z, %): 422 ($M^+$, 2), 391 (4), 365 ($M^+$- 57, 40), 337 (8), 287 (12), 259 (10), 225 (8), 199 (65), 135 (100), 105 (38).

The corresponding enantiomer **70b** was also made and characterized as follows - $[\alpha]_D^{r.t}$ = + 28.4 (c = 0.75, $CHCl_3$).

Example 29 - preparation of (1*S*,2*S*,3a*S*,4a*S*)-2-t butyldiphenylsilyloxy-3a-formyl-1-methyl-bicyclo[3.1.0]hexane **39a**

Reference is made to figure 4. To a solution of intermediate **38a** (132 mg, 0.324 mmole) in THF (10 mL) at 0°C was added dropwise $LiAlH_4$ (485 uL, 0.485 mmol, 1 M in THF). The mixture was stirred at 0°C for 1.5 hour and then EtOAc (3 mL) was added. After stirring for 0.5 hour, the reaction was quenched with $H_2O$. The mixture was filtrated through Celite and filtrate was dried, concentrated. The residue was purified by flash chromatography (using a isooctane / EtOAc 4 : 1 mixture) to afford the primary alcohol (120 mg, 97%) as a colourless oil being characterized as follows: [- $R_f$ = 0.21 (isooctane/EtOAc, 4:1). - $[\alpha]_D^{r.t}$ = - 4.6 (c = 0.35, $CHCl_3$). - IR (film): ν = 3332, 3070, 2930, 2858, 1472, 1428, 1390, 1219, 1112, 1008 $cm^{-1}$- $^1H$ NMR ($CDCl_s$): δ = 7.66 - 7.35 (m, 10 H), 3.91 (m, 1 H), 3.52 (d, J = 2.0 Hz, 2 H), 2.03 (m, 1 H), 1.91 - 1.82 (m, 2 H), 1.45 (br.s, 1 H), 1.05 (s, 9 H), 1.04 (d, J = 7.7 Hz, 3 H), 0.92 (m, 1 H), 0.27 (m, 2 H). - $^{13}C$ NMR ($CDCl_3$): δ = 137.6, 134.2, 129.6, 127.5, 73.4, 68.6, 37.5, 35.0, 29.9, 27.9, 26.9, 19.2, 15.3, 13.5. - MS (m/z, %): 380 ($M^+$, 1), 363 (1), 323 ($M^+$ - 57, 11), 305 (4), 267 (2), 245 (21), 227 (9), 199 (100), 152 (4), 135 (12), 107 (85). - Elemental analysis: $C_{24}H_{32}O_2Si$ (380.60): calcd. C 75.74, H 8.47; found C 75.34, H 8.62]. Said oil (100 mg, 0.263 mmol) was taken up into $CH_2Cl_2$ (10 mL) containing NMO (48 mg, 97%, 0.397 mmol) and 4A MS (130 mg) and then TPAP (9.5 mg, 97%, 0.03 mmol) was added. The mixture was stirred at room temperature for 0.5 hour and then was subjected to flash chromatography (using a isooctane / EtOAc 4 : 1 mixture). Evaporation and column chromatography (using a isooctane / EtOAc 9 : 1 mixture) afforded intermediate **39a** (93 mg, 95%) as a colourless oil being characterized as follows: $R_f$ = 0.22 (isooctane/EtOAc, 95:5). - $[\alpha]_D^{r.t}$ = - 49.4 (c = 0.99, $CHCl_3$). IR (film): ν = 2931, 2858, 1702, 1459, 1427, 1389, 1219 $cm^{-1}$. - $^1H$ NMR ($CDCl_3$): δ = 8.80 (s, 1 H), 7.65 - 7.37 (m, 10 H), 3.93 (m, 1 H), 2.24 (dd, J = 12.9, 9.3 Hz, 1 H), 2.13 (m, 1 H), 1.81 (dd, J = 12.9, 7.2 Hz, 1 H), 1.68 (dd, J = 8.9, 5.6 Hz, 1 H), 1.20 (dd, J = 8.8, 6.0 Hz, 1 H), 1.05 (s, 9 H), 1.03 (d, J = 8.1 Hz, 3 H), 0.86 (t, J = 5.6Hz, 1 H). - $^{13}C$ NMR ($CDCl_3$): δ = 200.1, 135.7, 133.8, 129.7, 127.6, 72.7, 37.9, 36.7, 32.7, 30.6, 26.8, 19.2, 17.6, 14.7. - MS (m/z, %): 321 ($M^+$- 57, 86), 305 (7), 279 (14), 243 (20), 199 (100), 181 (35), 139 (68), 105 (58), 77 (64).

Example 30 - preparation of the ethyl homologue **39b**

Using a similar procedure (figure 4) to that of example 29, the ethyl homologue of intermediate **39a** was made and characterized as follows: $R_f$ = 0.28 (isooctane / EtOAc 9 : 1 mixture). - $[\alpha]_D^{r.t}$ = - 33.3 (c = 0.57, $CHCl_3$). - IR (film): ν = 3070, 2960, 2857, 2711, 1700, 1472, 1428, 1389, 1270, 1217, 1175, 1111, 1027 $cm^{-1}$. - $^1H$ NMR ($CDCl_3$): δ = 8.81

(s, 1 H), 7.64 - 7.35 (m, 10 H), 3.95 (q, J = 7.8 Hz, 1 H), 2.19 (m, 1 H), 1.91 - 1.78 (m, 4 H), 1.25 (m, 1 H), 1.17 (m, 1 H), 1.05 (s, 9 H), 0.95 (t, J = 7.2 Hz, 3 H), 0.84 (t, J = 5.6 Hz, 1 H).-$^{13}$C NMR ($CDCl_3$): δ = 200.0, 135.7, 135.5, 133.7, 129.7, 129.6, 127.6, 73.1, 43.6, 38.2, 31.7, 30.1, 26.8, 20.9, 19.1, 17.6, 11.7. - MS (m/z, %): 335 ($M^+$ - 57, 55), 280 (10), 257 (18), 227 (15), 199 (100), 181 (30), 139 (50), 105 (45), 77 (65). Elemental analysis: $C_{25}H_{32}O_2Si$ (392.60): calcd. C 76.48, H 8.21; found C 76.32, H 8.40.

Example 31 - preparation of (1*R*,2*R*,3a*S*,4a*S*)-2-f-butyldiphenylsilyloxy-3a-formyl-1-methyl-bicyclo[3.1.0]hexane **44a**

Referring to figure 4, intermediate 44a was made from intermediate **43a** (in a manner as described previously for intermediate **39a**) and obtained in a yield of 93% as a colourless oil being characterized as follows: $R_f$ = 0.38 (isooctane / EtOAc 4:1).- $[\alpha]_D^{r.t}$ = - 109.3 (c =0.16, $CHCl_s$). - IR (film): v = 3048, 2958, 2858, 1702, 1589, 1472, 1428, 1389, 1362, 1252, 1182, 1112, 1086 $cm^{-1}$. - $^1$H NMR ($CDCl_3$): δ = 8.78 (s, 1 H), 7.65 - 7.35 (m, 10 H), 3.39 (dd, J = 15.5, 7.8 Hz, 1 H), 2.26 - 2.22 (m, 2 H), 1.88 (m, 2 H), 1.08 (m, 1 H), 1.05 (s, 9 H), 0.93 (d, J = 7.7 Hz, 3 H), 0.75 (t, J = 5.5 Hz, 1 H). $^{13}$C NMR ($CDCl_3$): δ = 199.9, 135.9, 133.8, 129.7, 127.6, 77.3, 41.5, 37.4, 33.7, 30.8, 26.9, 19.1, 15.7, 15.2. - MS (m/z, %): 321 ($M^+$ - 57, 48), 319 (8), 309 (11), 259 (24), 199 (100), 181 (40), 153 (20).

Example 32 - preparation of the ethyl homologue **44b**

Using a similar procedure (figure 4) to that of example 31, the ethyl homologue of intermediate **44a** was made and characterized as follows: $R_f$ = 0.34 (isooctane/EtOAc, 9:1). - $[\alpha]_D^{r.t}$ = - 110.9 (c = 0.53, $CHCl_3$). - IR (film): v = 3070, 2959, 2857, 2710, 1704, 1462, 1428, 1257 $cm^{-1}$. -$^1$H NMR ($CDCl_3$): δ = 8.80 (s, 1 H), 7.64 - 7.35 (m, 10 H), 3.46 (q, J = 7.3 Hz, 1 H), 2.21 (t, J = 11.3 Hz, 1 H), 2.09 (br.s, 1 H), 1.96 (d, J = 5.1 Hz, 1 H), 1.87 (m, 1 H), 1.62 (m, 2 H), 1.12 (s, 1 H), 1.04 (s, 9 H), 0.90 (t, J = 7.2 Hz, 3 H), 0.74 (t, J = 5.6 Hz, 1 H). $^{13}$C NMR ($CDCl_3$): δ = 199.6, 135.8, 133.8, 129.6, 127.4, 75.9, 48.7, 37.2, 33.6, 28.6, 26.8, 23.9, 19.1, 15.3, 12.6. - MS (m/z, %): 335 ($M^+$- 57, 50), 279 (4), 227 (10), 199 (100), 181 (20), 139 (45), 105 (32), 77 (60).

Illustrative Example 33 - preparation of 2β-methyl-19-nor-1α,25-dihydroxyvitamin $D_3$ (compound **101**)

Reference is made to figure 8. To a stirred solution of intermediate **20** shown in figure 8 (65 mg, 0.151 mmole) in THF (2.0 mL) was added dropwise *t*BuLi (197 uL, 1.7 M in pentane, 0.334 mmol) at -78°C. After stirring at -78°C for 1 hour, the reaction was warmed to -10°C and was kept on stirring at this temperature for 0.5 hour. The solution was cooled down to -78°C again and then a solution of intermediate **44a** (80 mg, 0.212 mmol) in THF (1.5 mL) was added dropwise. Stirring was continued at this temperature for 1 hour and then the reaction was quenched by addition of saturated $NH_4Cl$. The solution was diluted with $Et_2O$, washed (saturated $NaHCO_3$ and brine), dried and concentrated. The residue was purified by flash chromatography (using a isooctane / EtOAc 94: mixture) to afford intermediate shown as **27a** in figure 8 (53 mg, 48%) as a colorless oil. The latter (53 mg, 0.073 mmole) was taken up into 4 mL of a dioxane / $H_2O$ 3 : 1 mixture containing *p*-toluenesulfonic acid (hereinafter PTSA) (4 mg, 0.023 mmol). The mixture was stirred in the dark at 55 - 60°C for 4 hours and then the solution was diluted with $Et_2O$ washed (saturated $NaHCO_3$ and brine), dried and concentrated. The residue was purified by HPLC (using a isooctane / EtOAc 4 : 1 mixture) affording in a yield of 88% the intermediates shown as **72a** (38 mg) and **73a** (5 mg) in figure 8. These intermediates were characterized as follows:

Intermediate **72a :** $R_f$ = 0.22 (isooctane / EtOAc 4 : 1). - $[\alpha]_D^{r.t}$ = - 15.7 (c = 0.76, $CHCl_3$). - IR (film): ν = 3394, 2942, 1616, 1471, 1428, 1377, 1110 $cm^{-1}$. -$^1$H NMR ($CDCl_3$): δ = 7.71 - 7.35 (m, 10 H), 6.14 (d, J = 11.3 Hz, 1 H), 5.46 (d, J = 11.3 Hz, 1 H), 3.94 (m, 1 H), 3.72 (td, J = 8.9, 4.2 Hz, 1 H), 2.72 (m, 1 H), 2.66 (dd, J = 13.3, 4.0 Hz, 1 H), 2.37 - 2.44 (m, 2 H), 2.25 (dd, J = 13.6, 5.2 Hz, 1 H), 2.05 - 1.85 (m, 4 H), 1.80 (m, 1 H), 1.70 - 1.60 (m, 3 H), 1.50 - 1.24 (m, 13 H), 1.23 (s, 6 H), 1.07 (s, 9 H), 1.03 (d, J = 6.9 Hz, 3 H), 0.93 (d, J = 6.5 Hz, 3 H), 0.45 (s, 3 H). -$^{13}$C NMR ($CDCl_3$): δ = 142.2, 135.9, 1334.8, 134.2, 132.1, 129.6, 127.5, 122.9, 115.4, 73.9, 71.4, 71.1, 56.5, 56.2, 45.6, 44.4, 43.3, 40.4, 36.5, 36.4, 36.1, 30.1, 29.2, 28.8, 27.7, 27.1, 25.5, 22.5, 22.3, 20.8, 19.4, 18.8, 14.0, 11.9. - MS (m/z, %): 656 ($M^+$, 1), 638 ($M^+$ - 18, 1), 600 ($M^+$ - *t*Bu + H, 1), 581 (3), 563 (2), 503 (3), 472 (1), 400 (4), 365 (6), 321 (9), 239 (11), 199 (100), 149 (19), 135 (52), 59 (88).

Intermediate **73a:** $R_f$ = 0.19 (isooctane / EtOAc 4 : 1). - $[\alpha]_D^{r.t}$ = + 18.3 (c = 0.31, $CHCl_3$). - IR (film): ν = 3405, 3071, 2958, 2879, 1459, 1429, 1376, 1217, 1147, 1076, 1053 $cm^{-1}$. - $^1$H NMR ($CDCl_3$): δ = 7.71 - 7.36 (m, 10 H), 5.99 (d, J = 11.2 Hz, 1 H), 5.77 (d, J = 11.2 Hz, 1 H), 4.00 (m, 1 H), 3.71 (td, J 8.1, 4.2 Hz, 1 H), 2.68 (dd, J = 13.8, 5.1 Hz, 1 H), 2.58 (dd, J = 13.7, 5.9 Hz, 1H), 2.29 (dd, J = 13.7, 3.7 Hz, 1 H), 2.23 (dd, J = 13.1, 3.9 Hz, 1 H), 2.07 - 1.82 (m, 5 H), 1.68 - 1.23 (m, 17 H), 1.22 (s, 6 H), 1.05 (s, 9 H), 1.03 (d, J = 6.7 Hz, 3 H), 0.93 (d, J = 6.5 Hz, 3 H), 0.51 (s, 3 H). - $^{13}$C NMR ($CDCl_3$): δ = 142.3, 135.9, 134.9, 133.0, 129.5, 127.5, 123.4, 115.4, 74.4, 71.3, 71.1, 56.5, 56.2, 4.5. 7, 44.4, 44.0, 40.5, 36.4, 36.1, 34.8, 30.1, 29.4, 28.8, 27.6, 27.0, 25.4, 23.4, 22.2, 20.8, 19.4, 18.8, 13.6, 12.0. - MS (m/z, %): 656 ($M^+$, 1), 599 ($M^+$ - *t*Bu, 1), 581 (3), 521 (1), 468 (1), 400 (1), 365 (6), 325 (5), 245 (8), 199 (61), 183 (18), 135 (42),

59 (100).

A solution of intermediate **72a** (27 mg, 0.041 mmol) in THF (0.5 mL) was treated with tetrabutylammonium fluoride (hereinafter TBAF) (3.5 mL, 1 M in THF). After stirring at room temperature in the dark for 72 hours, the solution was subjected to flash chromatography (using a isooctane / EtOAc 1 : 1 mixture). The residue was purified by HPLC (using a isooctane / EtOAc 3 : 2 mixture) affording the vitamin D compound **101** (11 mg, 82%) which was characterized as follows: $R_f$ = 0.21 (isooctane / EtOAc 1 : 1). - IR (KBr): ν = 3422, 2946, 1618, 1452, 1376, 1350, 1150, 1056 $cm^{-1}$. - $^1H$ NMR ($CDCl_3$): δ = 6.26 (d, J = 11.2 Hz, 1 H), 5.87 (d, J = 11.2 Hz, 1 H), 3.51 (td, J = 10.1, 4.7 Hz. 1 H). 3.08 (dd. J = 12.9. 4.0 Hz. 1 H). 2.79 (dd. J = 12.9, 4.0 Hz, 1 H), 2.44 (d, J = 13.1 Hz, 1 H), 2.37 (m, 1 H), 2.04 -1.98 (m, 3 H), 1.90 (t, J = 10.7 Hz, 2H), 1.80-1.23 (m, 18 H), 1.22 (s, 6 H), 1.14 (d, J = 6.8 Hz, 3 H), 0.94 (d, J = 6.5 Hz, 3 H), 0.55 (s, 3 H). - MS (m/z, %): 418 ($M^+$, 9), 400 (6), 385 (4), 357 (5), 317 (2), 289 (6), 245 (8), 203 (4), 189 (6), 149 (27), 135 (41), 84 (58), 59 (100).

Illustrative Example 34 - preparation of 2a-methyl-19-nor-1α 25-dihydroxyvitamin $D_3$ (compound **102)**

Reference is made to figure 2. The same procedure was used as in example 33, except the starting material was **15.** The resulting vitamin D analogue **102** was characterized as follows: $R_f$ = 0.18 (isooctane / EtOAc 1 : 1). - $[\alpha]_D^{r.t}$ = + 26.6 (c = 0.14, $CHCl_3$). - IR (film): ν = 3354, 2958, 1454, 1054 $cm^{-1}$ -$^1H$ NMR ($CDCl_3$): δ = 6.37 (d, J = 11.3 Hz, 1 H), 5.83 (d, J = 11.3 Hz, 1 H), 3.96 (m, 1 H), 3.61 (td, J = 9.4, 4.6 Hz, 1 H), 3.20 (m, 2 H), 3.02 (dt, J = 12.0, 4.8 Hz, 2 H), 2.80 (dd, J = 13.6, 4.3 Hz, 2 H), 2.60 (dd, J = 12.9, 4.4Hz, 1H), 2.23 (d, J = 12.6 Hz, 1 H), 2.14 (t, J = 10.3 Hz, 1 H), 2.06 - 1.20 (m, 18 H), 1.22 (s, 6 H), 1.14 (d, J = 6.9 Hz, 3 H), 0.98 (d, J = 7.4 Hz, 3 H), 0.54 (s, 3 H). - MS (m/z , %): 418 ($M^+$, 1), 400 ($M^+$ - $H_2O$ 18), 382 (6), 340 (22), 295 (12), 271 (9), 233 (32), 191 (22), 149 (85), 135 (25), 92 (100).

Illustrative Example 35 - preparation of the ethyl homologue **110**

Using the same procedure as in example 33, the vitamin D analogue **110** was made and characterized as follows: $R_f$ = 0.18 (isooctane : EtOAc, 3 : 2); $[\alpha]_D^{r.t}$ = + 28.0 (c = 0.21, $CHCl_3$). - IR (film): ν=3389, 2958, 1454, 1188, 1045c $m^{-1}$. - $^1H$ NMR ($CDCl_3$): δ = 6.38 (d, J = 11.3 Hz, 1 H), 5.83 (d, J = 11.3 Hz, 1 H), 4.14 (m, 1 H), 3.63 (m, 1 H), 2.87 (dd, J = 13.9, 4.0 Hz, 1 H), 2.80 (dd, J = 12.7, 4.5 Hz, 1 H), 2.61 (dd, J = 12.5, 4.1 Hz, 1 H), 2.20 - 1.22 (m, 24 H), 1.21 (s, 6 H), 1.00 (t, J = 7.4 Hz, 3 H), 0.91 (d, J = 6.7 Hz, 3H), 0.53 (s, 3 H). - $^{13}C$ NMR ($CDCl_3$): δ = 143.3, 131.5, 124.1, 115.4, 71.7, 71.3, 68.0, 56.7, 56.5, 50.9, 45.9, 45.5, 44.6, 40.6, 36.5, 36.2, 35.7, 30.2, 29.5, 29.1, 27.8, 25.6, 23.6. 22.4, 20.9, 18.9, 12.2, 11.8. - MS (m/z, %): 432 ($M^+$, 10), 414 (6), 371 (2), 303 (5), 267 (6), 245 (8), 208 (6), 173 (10), 149 (30), 133 (40), 81 (65), 55 (100).

Illustrative Example 36 - Preparation of the ethyl homologue **109**

Using the same procedure as in example 33, the vitamin D analogue **109** was made and characterized as follows: $R_f$= 0.22 (isooctane / EtOAc 3 : 2); $[\alpha]_D^{r.t}$ = + 42.1 (c = 0.32, $CHCl_3$). - IR (film): ν = 3378, 2959, 1454, 1378 $cm^{-1}$. - $^1H$ NMR ($CDCl_3$): δ = 6.26 (d, J = 11.3 Hz, 1 H), 5.87 (d, J = 11.3 Hz, 1 H), 4.09 (m, 1 H), 3.54 (m, 1 H), 3.10 (dd, J = 12.7, 4.3 Hz, 1 H), 2.80 (dd, J = 12.8, 4.5 Hz, 1 H), 2.38 (m, 2 H), 2.05 -1.25 (m, 25 H), 1.22 (s, 6 H), 1.00 (t, J = 7.4 Hz, 3 H), 0.94 (d, J = 6.5 Hz, 3 H), 0.54 (s, 3 H). - $^{13}C$ NMR ($CDCl_3$): δ = 143.1, 131.4, 123.5, 115.4, 71.2, 67.7, 56.6, 56.4, 51.5, 45.9, 44.5, 44.2, 40.6, 37.9, 36.5, 36.2, 31.0, 29.5, 29.3, 29.1, 27.8, 23.6, 22.4, 20.9, 20.3, 18.9, 12.2, 11.7. - MS (m/z, %): 432 ($M^+$, 10), 414 (5), 303 (8), 267 (8), 245 (15), 208 (5), 173 (5), 135 (30), 105 (35), 81 (70), 59 (100).

Example 37 - preparation of 14-*epi*-2α-methyl-19-nor-1α,25-dihydroxyvitamin $D_3$ compound **106**)

Using the same procedure as in example 33, the vitamin D analogue **106** was made and characterized as follows: $R_f$ = 0.20 (isooctane / EtOAc 1 : 1) ; $[\alpha]_D^{r.t}$ = + 61.1 (c = 0.34, $CHCl_3$). - IR (film): ν = 3384, 2960, 1455, 1379, 1147, 1043 $cm^{-1}$. - $^1H$ NMR ($CDCl_3$): δ = 6.31 (d, J = 11.2 Hz, 1 H), 6.01 (d, J = 11.2 Hz, 1 H), 3.97 (dd, J = 5.6, 2.4 Hz, 1 H), 3.59 (td, J = 9.5, 4.6 Hz, 1 H), 2.84 (dd, J =13.9, 4.6 Hz, 1 H), 2.59 (dd, J = 12.8, 4.4 Hz, 1 H), 2.47(dt, J = 14.6, 5.1 Hz, 1 H), 2.21 (d, J = 13.7 Hz, 1 H), 2.15 - 2.08 (m, 2 H), 1.88 - 1.21 (m, 22 H), 1.22 (s, 6 H), 1.14 (d, J = 6.9 Hz, 3 H), 0.92 (s, 3 H), 0.88 (d, J = 6.7 Hz, 3H). $^{13}C$ NMR ($CDCl_3$): δ = 143.4, 131.6, 124.2, 118.5, 72.4, 71.6, 71.1, 57.8, 54.6, 45.3, 44.9, 44.4, 43.9, 37.9, 35.5, 34.5, 34.0, 29.9, 29.4, 29.2, 26.8, 24.8, 22.3, 21.9, 21.7, 19.8, 13.6. - MS (m/z , %): 418 ($M^+$, 1), 400 ($M^+$-$H_2O$, 22), 387 (7), 357 (4), 340 (5), 289 (14), 271 (21), 245 (19), 191 (17), 147 (29), 133 (38), 81 (59), 59 (100).

Example 38 - preparation of the ethyl homologue **114**

Using the same procedure as in example 33, the vitamin D analogue **114** was made and characterized as

follows: $R_f$ = 0.20 (isooctane / EtOAc, 3:2); [α] $_D^{r.t}$ = + 34.4 (c = 0.48, $CHCl_3$). - IR (film): ν = 3372, 2958, 1464, 1378, 1190, 1044 $cm^{-1}$. - $^1H$ NMR ($CDCl_3$): δ = 6.32 (d, J =11.3 Hz, 1 H), 6.01 (d, J = 11.3 MHz, 1 H), 4.15 (br.s, 1 H), 3.64 (m, 1 H), 2.92 (m, 1 H), 2.59 (m, 1 H), 2.47 (m, 1 H), 2.18 - 2.05 (m, 3 H), 1.86 - 1.24 (m, 23H), 1.22 (s, 6 H), 1.00 (t, J = 7.4 Hz, 3 H), 0.89 (d, J = 6.8 Hz, 3 H), 0.86 (s, 3 H). - $^{13}C$ NMR ($CDCl_3$): δ = 143.5, 131.6, 124.2, 118.7, 71.6, 71.2, 68.0, 58.0, 54.7, 51.0, 45.6, 45.4, 44.5, 38.0, 35.7, 34.7, 34.1, 30.0, 29.5, 29.3, 27.0, 24. 9, 22. 3, 22.0, 21.8, 20.0, 119.8, 11.8. - MS (m/z, %): 432 ($M^+$, 2), 414 (15), 386 (4), 265 (5), 245 (10), 199 (20), 161 (15), 135 (30), 81 (50), 55 (100).

Example 39 - preparation of 14-*epi*-2-methyl-19-nor-1 α,25-dihydroxyvitamin $D_3$ (compound **105**)

Using the same procedure as in example 33, the vitamin D analogue **105** was made and characterized as follows: $R_f$ = 0.18 (isooctane / EtOAc 1 : 1) ; [a]$_D^{r.t}$ = + 38.7 (c = 0.40, $CHCl_3$). - IR (film): ν = 3382, 2958, 1455, 1377, 1212, 1045 $cm^{-1}$. - $^1H$ NMR ($CDCl_3$): δ = 6.19 (d, J = 11.2 Hz, 1 H), 6.05 (d, J = 11.2 Hz, 1 H), 3.90 (dd, J = 6.2, 3.0 Hz, 1 H), 3.52 (td, J = 10.1, 4.6 Hz, 1 H), 3.08 (dd, J = 12.9, 4.4 Hz, 1 H), 2.41 - 2.48 (m, 2 H), 2.33 (dd, J = 13.2, 4.2 Hz, 1 H), 2.15 - 2.03 (m, 2 H), 1.94 - 1.80 (m, 2 H), 1.72 - 1.23 (m, 19 H), 1.22 (s, 6 H), 1.13 (d, J = 6.9 Hz, 3 H), 0.92 (s, 3 H), 0.88 (d, J = 6.7Hz, 3 H). - $^{13}C$ NMR ($CDCl_3$): δ = 143.2, 131.6, 123.7, 118.6, 71.9, 71.7, 71.1, 57.9, 54.6, 45.4, 44.4, 44.1, 43.9, 37.9, 37.4, 34.5, 34.0, 29.8, 29.4, 29.2, 26.8, 24.8, 22.4, 21.8, 21.6, 19.8, 14.0. - MS (m/z , %): 418 ($M^+$, 1), 401 ($M^+$ - $H_2O$ + H, 1), 387 (2), 357 (4), 370 (1), 293 (1), 292 (5), 260 (2), 199 (35), 183 (11), 153 (25), 111 (28), 93 (100).

Example 40 - preparation of the ethyl homologue **113**

Using the same procedure as in example 33, the vitamin D analogue **113** was made and characterized as follows: $R_f$= 0.21 (isooctane / EtOAc 3 : 2) ; [ α]$_D^{r.t}$ = + 17.0 (c = 0.15, $CHCl_3$). - IR (film): ν = 3369, 2958, 1455, 1378, 1190, 1044 $cm^{-1}$. - $^1H$ NMR ($CDCl_3$): δ = 6.19 (1 H, d, J = 11.2 Hz), 6.06 (d, J = 11.3 Hz, 1 H), 4.09 (m, 1 H), 3.50 (m, 1 H), 3.11 (dd, J = 12.7, 4.2 Hz, 1 H), 2.97 (m. 1 H). 2.47 (m. 1 H). 2.38 (m. 1 H). 2.18 - 1.25 (m, 25 H), 1.22 (s, 6 H), 0.99 (t, J = 7.4 Hz, 3 H), 0.90 (d, J = 6.7 Hz, 3 H), 0.88 (s, 3 H). - MS (m/z, %): 432 ($M^+$, 2), 414 (28), 381 (4), 301 (4), 267 (8), 245 (10), 199 (30), 149 (30), 105 (50), 81 (70), 59 (100).

Illustrative Examples 41 - preparation of 2α-methyl-19-nor-23-yn-1α,25-dihydroxy-vitamin $D_3$ (compound **104**)

Using the same procedure as in example 33, the vitamin D analogue **113** was made and characterized as follows: $R_f$ = 0.18 (isooctane / EtOAc 1 : 1) ; α]$_D^{r.t}$ = + 42.7 (c = 0.11, $CHCl_3$). - IR (film): 3368, 2929, 1614, 1454, 1377, 1261, 1166, 1024 $cm^{-1}$. - $^1H$ NMR ($CDCl_3$): δ = 6.36 (d, J =11.1 Hz, 1 H), 5.82 (d, J = 11.1 Hz, 1 H), 3.96 (br.s, 1 H), 3.61 (m, 1 H), 2.80 (d, J = 14.1 Hz, 2 H), 2.60 (d, J = 12.8 Hz, 1 H), 2.28 -1.52 (m, 17 H), 1.51 (s, 6 H), 1.38 - 1.25 (m, 3 H), 1.13 (d, J = 6.7 Hz, 3 H), 1.06 (d, J = 6.3 Hz, 3 H), 0.54 (s, 3 H). - MS (m/z , %): 414 ($M^+$, 14), 396 ($M^+$- $H_2O$, 8), 381 (7), 353 (4), 317 (12), 267 (3), 241 (9), 199 (13), 185 (16), 161 (21), 105 (37), 84 (52), 43 (100).

Illustrative Example 42 - preparation of the ethyl homologue **112**

Using the same procedure as in example 33, the vitamin D analogue **112** was made and characterized as follows: $R_f$= 0.18 (isooctane / EtOAc 3 : 2); [α ]$_D^{r.t}$ = + 23.1 (c = 0.26, $CHCl_3$). - IR (firm): ν = 3367, 2958, 2238, 1455, 1378, 1167 $cm^{-1}$. - $^1H$ NMR ($CDCl_3$): δ = 6.38 (d, J = 11.2 Hz, 1 H), 5.83 (d, J = 11.1 Hz, 1 H), 4.14 (m, 1 H), 3.64 (td, J = 9.8, 4.8 Hz, 1 H), 2.97 (m, 1 H), 2.87 (dd, J = 13.9, 4.3 Hz, 1 H), 2.80 (dd, J = 12.8, 4.5 Hz, 1 H), 2.30 - 1.51 (m, 17 H), 1.50 (s, 6 H), 1.46 -1.25 (m, 5 H), 1.06 (d, J = 6.6 Hz, 3 H), 0.98 (t, J = 7.4 Hz, 3 H), 0.54 (s, 3 H). - MS (m/z, %): 428 ($M^+$, 2), 410 (10), 370 (8), 331 (5), 313 (8), 295 (4), 241 (3), 199 (8), 161 (20), 149 (40), 91 (40), 43 (100).

Illustrative Example 43 - preparation of 2β-methyl-19-nor-23-yn-1α,25-dihydroxy-vitamin $D_3$ (compound **103**)

Using the same procedure as in example 33, the vitamin D analogue **113** was made and characterized as follows: $R_f$ = 0.18 (isooctane / EtOAc 1 : 1) ; [α]$_D^{r.t}$ = + 28.2 (c = 0.37, $CHCl_3$). - IR (film): ν = 3380, 2930, 1455, 1377, 1346, 1166 1041 $cm^{-1}$ - $^1H$ NMR ($DCCl_3$): δ = 644 (d. J = 11.3 Hz. 1 H). 6.04 (d. J =11.3 Hz, 1 H), 4.07 (dd, J = 4.1, 3.0 Hz, 1 H), 3.68 (td, J =10.1, 4.8 Hz, 1 H), 3.24 (dd, J = 12.9, 3.8Hz, 1 H), 2.97 (dd, J = 12.9, 4.4 Hz, 1 H), 2.60 (d, J = 3.6 Hz, 1 H), 2.51, (dd, J =13.8, 3.4 Hz, 1 H), 2.44 (dd, J = 16.6, 3.4 Hz, 1 H), 2.26 - 2.16 (m, 5 H), 2.07 (m, 2 H), 1.68 (s, 6 H), 1.88 - 1.66 (m, 8 H), 1.52 - 1.41 (m, 3 H), 1.31 (d, J = 6.8 Hz, 3 H), 1.24 (d, J =6.5 Hz, 3 H), 0.72 (s, 3 H). - MS (m/z , %): 414 ($M^+$, 18), 396 ($M^+$ - $H_2O$, 8), 376 (7), 356 (4), 353 (1). 317 (15), 267 (4), 241 (9), 199 (21), 173 (23), 161 (25), 105 (42), 91 (53), 43 (100).

Illustrative Example 44 - preparation of the ethyl homologue **112**

Using the same procedure as in example 33, the vitamin D analogue **112** was made and characterized as follows: $R_f$= 0.19 (isooctane / EtOAc 3 : 2); $[\alpha]_D^{r.t}$ = + 24.9 (c = 0.54, $CHCl_3$). -IR (film): 3378, 2930, 1454, 1166, 1039 $cm^{-1}$. - $^1H$ NMR ($CDCl_3$): δ = 6.26 (d, J = 11.2 Hz, 1 H), 5.87 (d, J = 11.2 Hz, 1 H), 4.10 (m, 1 H), 3.55 (m, 1 H), 3.10 (dd, J = 12.9, 4.1 Hz, 1 H), 2.80 (dd, J = 12.5, 4.2 Hz, 1 H), 2.40 - 1.53 (m, 18 H), 1.52 (s, 6 H), 1.50 - 1.24 ( m, 5 H), 1.07 (d, J = 6.5 Hz, 3 H), 1.00 (t, J = 7.4 Hz, 3 H), 0.56 (s, 3 H). - $^{13}C$ NMR ($CDCl_3$): δ = 142.6, 131.6, 123.4, 115.5, 86.1, 81.3, 71.2, 67.6, 65.4, 56.3, 55.7, 51.0, 45.7, 44.1, 40.4, 37.9, 36.0, 31.9, 30.1, 29.0, 27.7, 25.7, 23.5, 22.3, 20.2, 19.2, 12.2, 11.6. -MS (m/z, %): 428 ($M^+$, 10), 410 (2), 370 (4), 331 (3), 267 (3), 241 (4), 199 (8), 173 (8), 149 (20), 105 (30), 91 (45), 43 (100).

Example 45 - preparation of 14-*epi*-2α-methyl-19-nor-23-yn-1α,25-dihydroxy vitamin $D_3$ (compound **108**)

Using the same procedure as in example 33, the vitamin D analogue **108** was made and characterized as follows: $R_f$ = 0.19 (isooctane / EtOAc 1 : 1) ; $[\alpha]_D^{r.t}$ = + 55.1 (c = 0.11, $CHCl_3$). - IR (film): ν = 3362, 2959, 2929, 1450, 1376, 1329, 1243, 1175, 1127 $cm^{-1}$ . - $^1H$ NMR ($CDCl_3$): δ = 6.30 (d, J = 11.3 Hz, 1 H), 6.02 (d, J = 11.3 Hz, 1 H), 3.98 (m, 1 H), 3.60 (td, J = 9.4, 4.6 Hz, 1 H), 2.83 (dd, J = 13.9, 4.6 Hz, 1 H), 2.59 (dd, J = 12.8, 4.3 Hz, 1 H), 2.41 (dt, J = 12.5, 3.8 Hz, 1 H), 2.27 - 1.99 (m, 5 H), 1.87 (m, 1 H), 1.76 - 1.52 (m, 11 H), 1.49 (s, 6 H), 1.33 - 1.25 (m, 3 H), 1.13 (d, J = 6.7 Hz, 3 H), 1.02 (d, J = 6.5 Hz, 3 H), 0.95 (s, 3 H). $^{13}C$ NMR ($CDCl_3$): δ = 142.9, 131.9, 124.1, 118.5, 86.1, 81.7, 72.5, 71.6, 65.4, 57.7, 52.6, 44.9, 44.9, 43.9, 37.7, 35.5, 33.9, 31.7, 31.7, 29.4, 27.9, 25.2, 24.5, 22.5, 21.8, 20.0, 13.6. - MS (m/z , %): 653 ($M^+$ + H, 1), 634 ($M^+$ - $H_2O$, 5), 597 ($M^+$ - 57 + H, 2), 459 (1), 385 (4), 361 (3), 335 (3), 267 (4), 199 (90), 183 (38), 135 (75), 43 (100).

Example 46 - preparation of the ethyl homologue **116**

Using the same procedure as in example 33, the vitamin D analogue **116** was made and characterized as follows: $R_f$= 0.24 (isooctane / EtOAc 3 : 2); $[\alpha]_D^{r.t}$ = + 22.3 (c = 0.38, $CHCl_3$). - IR (film): ν = 3370, 2958, 2874, 2233, 1731, 1614, 1462, 1378, 1337, 1240, 1167 $cm^{-1}$ - 1H NMR ($CDCl_3$): δ = 6.31 (d, J = 11.3 Hz, 1 H), 6.03 (d, J =11.3 Hz, 1 H), 4.15 (m, 1 H), 3.64 (m, 1 H), 2.89 (m, 1 H), 2.60 (dd, J = 12.8, 4.3 Hz, 1 H), 2.45 -1.51 (m, 18 H), 1.50 (s, 6 H), 1.40 - 1.25 (m, 5 H), 1.02 (t, J = 7.4 Hz, 3 H), 0.91 (d, J = 6.9 Hz, 3 H), 0.88 (s, 3 H). - $^{13}C$ NMR ($CDCl_3$): δ = 143.1, 132.1, 124.2, 118.7, 86.2, 81.8, 71.7, 68.0, 57.9, 53.3, 52.8, 51.0, 45.6, 45.1, 37.9, 35.8, 34.2, 31.9, 30.2, 29.5, 28.2, 25.6, 25.3, 24.7, 22.7, 21.9, 20.2, 11.9. - MS (m/z, %): 428 ($M^+$, 2), 410 ($M^+$ - 18, 8), 370 (5), 313 (5), 277 (6), 199 (30), 149 (35), 142 (30), 91 (50), 43 (100).

Example 47 - preparation of 14-*epi*-2β-methyl-19-nor-23-yn-1α,25-dihydroxyvitamin $D_3$ (compound 107)

Using the same procedure as in example 33, the vitamin D analogue 107 was made and characterized as follows: $R_f$ = 0.18 (isooctane / EtOAc 1:1); $[\alpha]_D^{r.t}$ = + 43.8 (c = 0.21, $CHCl_3$). - IR (film): ν = 3358, 2929, 1455, 1377, 1338, 1239, 1166 $cm^{-1}$. - $^1H$ NMR ($CDCl_3$): δ = 6.19 (d, J = 11.4 Hz, 1 H), 6.06 (d, J = 11.4 Hz, 1 H), 3.89 (dd, J = 6.3, 3.2 Hz, 1 H), 3.54 (td, J = 10.1, 4.6 Hz, 1 H), 3.07 (dd, J = 12.9, 4.2 Hz, 1 H), ), 3.01 (m, 1 H), 2.46 - 2.31 (m, 3 H), 2.23 (dd, J = 16.7, 3.5 Hz, 2 H), 2.15 - 2.01 (m, 3 H), 1.94 - 1.86 (m, 2 H), 1.78 - 1.50 (m, 8 H), 1.49 (s, 6 H), 1.35 - 1.25 (m, 3 H), 1.14 (d, J = 6.7 Hz, 3 H), 1.03 (d, J = 6.6 Hz, 3 H), 0.88 (s, 3 H). - MS (m/z , %): 652 ($M^+$, 1), 634 ($M^+$- $H_2O$ + 1, 6), 594 ($M^+$ - 57 + H, 2), 537 (3), 459 (2), 396 (1), 378 (4), 321 (5), 261 (6), 199 (100), 183 (27), 135 (72).

Example 48 - Preparation of the ethyl homologue **115**

Using the same procedure as in example 33, the vitamin D analogue **115** was made and characterized as follows: $R_f$= 0.22 (isooctane / EtOAc 3 : 2); $[\alpha]_D^{r.t}$ = + 8.9 (c = 0.69, $CHCl_3$). - IR (film): ν = 3381, 2958, 2233, 1454, 1383, 1166 $cm^{-1}$. - $^1H$ NMR ($CDCl_3$): δ = 6.19 (d, J = 11.3 Hz, 1 H), 6.07 (d, J = 11.3 Hz, 1 H), 4.10 (m, 1 H), 3.57 (m, 1 H), 3.10 (dd, J = 12.8, 4.3 Hz, 1 H), 2.97 (m, 1 H), 2.38 (m, 2 H), 2.22 (m, 2 H), 2.15 - 2.00 (m, 3 H), 1.94 - 1.86 (m, 2 H), 1.84 -1.51 (m, 9 H), 1.50 (s, 6 H), 1.45 - 1.24 (m, 5 H), 0.98 (t, J = 7.5 Hz, 3 H), 0.90 (d, J = 6.7 Hz, 3 H), 0.88 (s, 3 H). - $^{13}C$ NMR ($CDCl_3$): δ = 142.8, 132.1, 123.5, 118.3, 86.3, 81.6, 71.0, 67.7, 57.8, 52.0, 51.0, 45.1, 44.1, 37.9, 37.8, 34.0, 31.9, 31.7, 30.1, 28.8, 28.2, 25.5, 24.8, 22.6, 22.0, 20.2, 19.9, 11.6. - MS (m/z, %): 428 ($M^+$, 2), 410 (6), 313 (4), 277 (4), 241 (4), 199 (15), 173 (10), 149 (30), 105 (25), 91 (40), 43 (100).

Example 49 - preparation of 3,14-*bis*-*epi*-2α-methyl-19-nor-23-yn-1α,25-dihydroxyvitamin-$D_3$

Using the same procedure as in example 33, this vitamin D analogue was made and characterized as follows:

$R_f$= 0.21 (isooctane / EtOAc 3 : 2). - $[\alpha]_D^{r.t}$ +53.11 (c = 0.31, $CHCl_3$). - IR (film)= 3345, 2928, 1455, 1362, 1232, 1167, 1103, 1067, 1038, 944, 874, 750 $cm^{-1}$. - $^1H$ NMR (500 MHz, $CDCl_3$): □ = 6.31 (d, J = 11.3 Hz, 1 H), 6.08 (d, J = 11.3 Hz, 1 H), 3.95 (d, J = 2.1 Hz, 1 H), 3.90 (d, J = 2.9 Hz, 1 H), 3.05 (d, J = 14.1 Hz, 1 H), 2.49-2.40 (m, 4 H), 2.26-1.50 (m, 15 H), 1.49 (s, 6 H), 1.31-1.23 m, 3 H), 1.19 (d, J = 7.2 Hz, 3 H), 1.01 (d, J = 6.7 Hz, 3 H), 0.96 (s, 3 H).- $^{13}C$, NMR (50 Mhz, $CDCl_3$): □ = 142.2, 129.8, 125.0, 118.7, 86.0, 81.8, 73.3, 73.1, 65.4, 57.4, 52.6, 44.9, 44.9, 38.9, 37.6, 36.5, 33.9, 31.7, 31.7, 29.3, 27.8, 25.1, 24.4, 22.4, 21.8, 20.1, 14.5. - MS (m/z, %): 414 ($M^+$, 1), 396, ($M^+$-$H_2O$, 9), 381 (5), 363 (7), 356 (4), 299 (7), 267 (8), 241 (9), 213 (12), 185 (14), 147 (21), 107 (21), 107 (26), 91 (45), 43 (100).

Examples 50 - preparation of 1,14-*bis-epi*-2β-methyl-19-nor-23-yn-1α,25-dihydroxyvitamin-$D_3$

Using the same procedure as in example 33, this vitamin D analogue was made and characterized as follows: $R_f$ = 0.21 (isooctane / EtOAc 4 : 1); $[\alpha]_D^{r.t}$ + 20.42 (c = 0.31, $CHCl_3$). - IR (film)= 3353, 2930, 1611, 1455, 1376, 1169, 1070, 1028, 988, 948, 882, 729 $cm^{-1}$. - $^1H$ NMR (500 MHz, $CDCl_3$): □ = 6.32 (d, J = 11.3 Hz, 1 H), 6.08 (d, J = 11.3 Hz, 1 H), 3.96 (br.s, 1 H), 3.90 (br.s, 1 H), 3.06 (dd, J = 14.1, 3.2 Hz, 1 H), 2.49-2.38 (m, 3 H), 2.25-1.50(m, 16 H), 1.49 (s, 6 H), 1.32-1.24 (m, 3 H), 1.18 (d, J = 7.2 Hz, 3 H), 1.02 (d, J = 6.6 Hz, 3 H), 0.95 (s, 3 H). - MS (m/z, %): 396 ($M^+$-$H_2O$, 24), 378 (8), 335 (2), 299 (13), 267 (12), 241 (13), 199 (18), 185 (20), 145 (21), 105 (51), 91 (74), 43 (100).

In the following examples dealing with biological tests on the claimed 14β-compounds, results are also given for 14α-compounds for the purposes of comparison.

Example 51 - Binding properties of the 1α,$25(OH)_2D_3$ analogues

*a) Affinity for vitamin D receptor (VDR)*

The methods used to evaluate the binding properties of the new analogues are examples of the state of the art techniques used for steroid hormone (including vitamin D) binding assays as described previously (Verstuyf A. et al. J Bone Mineral Res 13: 549-558, 1998).

The affinity of the C2-substituted analogues of 1α,$25(OH)_2D_3$ to the vitamin D receptor was evaluated by their ability to compete with [$^3H$]1α,$25(OH)_2D_3$ for binding to high speed supernatant from intestinal mucosa homogenates obtained from normal pigs. The incubation was performed at 4°C for 20 h and phase separation was obtained by addition of dextran-coated charcoal. The relative affinity of the analogues was calculated from their concentration needed to displace 50% of [$^3H$]1α,$25(OH)_2D_3$ from its receptor compared with the activity of 1α,$25(OH)_2D_3$ (assigned a value of 100 %).

Results

All the 19-nor-1α,$25(OH)_2D_3$ analogues (with the natural or 23-yne side chain; with or without 14-epimerisation) with a 2α-methyl (**102,104,106,108,117**) or 2α-ethyl (**110,112,114,116**) substituted A-ring possess higher affinity for the vitamin D receptor compared to their 2β-methyl (**101,103,105,107,118**) or 2β-ethyl (**109,111,113,115**) counterparts (Table 1 and 2).

The C2-methyl substituted 19-nor-1α,$25(OH)_2D_3$ analogues (**101-108, 141**) have mostly a higher binding affinity for the VDR than their C2-ethyl substituted 19-nor-1α,$25(OH)_2D_3$ counterparts (**109-116,142**).

The binding affinity for the VDR is always higher for the 2α-methyl analogues with the natural side chain of 1α,$25(OH)_2D_3$ (**102,106**) compared to their 2α-methyl counterparts with the 23-yne side chain (**104,108**). This observation is even more pronounced for the 2β-methyl analogues with the natural side chain of 1α,$25(OH)_2D_3$ (**101,105**) compared to the 2β-methyl analogues with the 23-yne side chain (**103,107**). The introduction of the 23-yne side chain in the 2α-ethyl (**112**) or 2β-ethyl (**111**) analogues also decreased the affinity of VDR compared to the 2-ethyl (**110**) or 2β-ethyl (**109**) analogues with the natural side chain of 1α,$25(OH)_2D_3$.

The 2α-methyl-1α,$25(OH)_2D_3$ analogue with (**106**) or without 14-epimerisation (102) together with the 2α-ethyl-1α,25 $(OH)_2D_3$ analogue (**110**) displayed the highest affinity for the vitamin D receptor [90% compared to 1α,$25(OH)_2D_3$ (=100 % binding)].

*b) Affinity for human DBP*

Binding of 1α,$25(OH)_2D_3$ analogues to hDBP was performed at 4°C essentially as described previously [18]. [$^3H$]1α,$25(OH)_2D_3$ and 1α,$25(OH)_2D_3$ or its analogues were added in 5 μl ethanol into glass, tubes and incubated with hDBP (0.18 μM) in a final volume of 1 ml (0.01 M Tris-HCl buffer and 0.154 M NaCl, pH 7.4) for 3 h at 4°C. Phase separation was then obtained by the addition of 0.5 ml of cold dextran-coated charcoal.

Results (Table 1 and 2)

All the investigated C2 substituted analogues have a binding affinity for DBP equivalent or lower than 10 % compared to 1α,25$(OH)_2D_3$ (=100% affinity), except for compounds **114,102** and **110** demonstrating 40, 50 and 20 % affinity, respectively.
The 2α-methyl (**102,104,106,108**) or 2α-ethyl (**110,112,114**) 19-nor-1α,25$(OH)_2D_3$ analogues have higher affinity for DBP compared to their 2β-methyl (**101,103,105,107**) or 2β-ethyl (**109,111,113**) counterparts.
The C2-methyl substituted 19-nor-1α,25$(OH)_2D_3$ analogues have always a higher binding affinity for DBP than their C2-ethyl substituted 19-nor-1α,25$(OH)_2D_3$ counterparts, except for 2α-methyl-14-epi-19-nor-1α,25$(OH)_2D_3$ [9 % affinity compared to 1α,25$(OH)_2D_3$ (=100 % binding), **compound 106]** versus 2α-ethyl-14-epi-19-nor-1α,25$(OH)_2D_3$ [40 % affinity compared to 1α,25$(OH)_2D_3$ (=100 % binding), **compound 114]**.
The binding affinity for DBP is always higher for the 2α-methyl or 2α-ethyl analogues with the natural side chain of 1α, 25$(OH)_2D_3$ compared to their 2α-methyl respectively 2α-ethyl counterparts with the 23-yne side chain.
When the *trans* oriented C14 hydrogen of the analogues is changed into a *cis* oriented C14 hydrogen the affinity for DBP decreased except for compound **114**.

Example 52 - Effects of the C2-substituted 1α,25$(OH)_2D_3$ analogues on cell proliferation and cell differentiation.

*a) Breast carcinoma cells MCF-7*

To evaluate the effect of on cell proliferation, malignant MCF-7 cells were cultured in DMEM/nut. mix. F12 (HAM) medium supplemented with 10% heat inactivated FCS, glutamine (2 mM), penicillin (100 units/ml) and streptomycin (0.1 mg/ml). Cultures were maintained at 37°C in a humidified atmosphere of 5% $CO_2$ in air. MCF-7 cells were seeded at 5 x $10^3$ cells/well in the above described medium in 96-well microtiter plates in a final volume of 0.2 ml per well. Triplicate cultures were performed. After 24 h, 1α,25$(OH)_2D_3$ or analogues were added in the appropriate concentrations for an incubation period of 72 h. Then, 1 μCi [$^3$H]thymidine was added to each well and cells were harvested after 4 h incubation with a Packard harvester and measured by the Packard Topcount System (Packard, Meriden, USA).

*b) Promyelocytic leukemia cells HL-60*

To evaluate the effect on cell differentiation HL-60 cells were seeded at 4 x 104 cells/ml in 25 $cm^2$ Falcon tissue chambers using RPMI 1640 medium supplemented with 20% FCS and gentamycin (50 μg/ml) in a final volume of 5 ml. Cultures were maintained at 37°C in a humidified atmosphere of 5% CO2 in air. One day later, 1α,25(OH)2D3 or analogues were added to the cell culture in ethanol (final concentration < 0.2%). After 4 days of culture, the dishes were shaken to lose adherent cells. Cells were washed twice in RPMI, counted and assayed for differentiation markers (NBT reduction assay). Superoxide production was measured as NBT reducing activity as described previously (Ostrem V.K et al. Proc Natl Acad Sci USA 84: 2610-2614, 1987). HL-60 cells at 1 x 106/ml were mixed with an equal volume of freshly prepared solution of phorbol 12-myristate 13-acetate (200 ng/ml) and NBT (2 μg/ml) and incubated for 30 min at 37°C. The percentage of cells containing black formazan deposits was determined using a hemacytometer.

Results (Table 1 and 2; Figure 9 and 10)

In general, the 2α-methyl-19-nor-1α,25$(OH)_2D_3$ analogues (**compounds 102**, **104, 106, 108, 141**) and most of the 2α-ethyl-19-nor-1α,25$(OH)_2D_3$ analogues (**compounds 110, 112, 116, 117, 142**) had very potent antiproliferative and prodifferentiating effects in contrast to the 2β-substituted analogues that were less potent than their 2α counterparts. The 2α-methyl-19-nor analogue with the natural side chain of 1α,25$(OH)_2D_3$ (**102**) showed an antiproliferative and prodifferentiating activity of 4 (MCF-7) to 10 (HL60) times that of 1α,25$(OH)_2D_3$. The introduction of the 23-yne side chain in this analogue **102** strongly increased (26 times) the capacity to inhibit the proliferation of MCF-7 cells **(compound 104)** and is 100 times more active than 1α,25$(OH)_2D_3$. When the *trans* oriented C14 hydrogen of the analogue 2α-methyl-19-nor-23-yne-1α,25$(OH)_2D_3$ **(104)** was reoriented into the *cis* configuration [2α-methyl-14-epi-19-nor-23-yne-1α,25$(OH)_2D_3$ (**compound 108**)] again the antiproliferative activity increased. The 14-epi-19-nor-1α,25$(OH)_2D_3$ analogue is half as potent as the parent compound to differentiate HL 60 cells and 3 times less active to inhibit MCF-7 cell proliferation (Table 1). Introduction of a 2α-methyl **(106)** or 2α-ethyl **(114)** group in this 14-epi-19-nor-1α,25$(OH)_2D_3$ analog increased the biological effects on HL 60 cells and MCF-7 cells.
Of all the investigated analogues compound **108** was the most potent analogue to induce cell differentiation (40 times that of 1α,25$(OH)_2D_3$) and to inhibit cell proliferation [120 times that of 1α,25$(OH)_2D_3$]. As described above the intrinsic activity of the 19-nor-14-epi-23-yne-1α,25$(OH)_2D_3$ analogue (SDB 112/TX522) increased strongly when it was substituted with a 2α-methyl A-ring (compound **108)** but when TX 522 was substituted with a 2α-ethyl A-ring **(116)** the biological

profile was not further enhanced. On the other hand the introduction of a 2β-methyl **(107)** or 2β-ethyl **(115)** A-ring in TX 522 decreased its *in vitro* activity. 3-Epimerisation **(117)** of compound **108** markedly decreased the prodifferentiative activity and 20-epimerisation **(141)** of compound **108** strongly decreased the inhibition of MCF-7 cell proliferation. 1-Epimerisation **(118)** of compound **107** was not active at all.
The 2β-methyl-19-nor analogue with the natural side chain of 1α,25$(OH)_2D_3$ **(101)** was less potent than 1α,25$(OH)_2D_3$ to induce differentiation or inhibit proliferation. The introduction of 2β-methyl **(105)** in the 14-epi-19-nor-1α,25$(OH)_2D_3$ analog increased the prodifferentiating effects on HL 60 cells but decreased the antiproliferative effects on MCF-7 cells. The opposite was seen when 2β-ethyl **(113)** was introduced in 14-epi-19-nor1α,25$(OH)_2D_3$. The 2β-methyl-19-nor analogue with the 23-yne side chain **(103)** and its 14-epi counterpart **(107)** were 3 to 9 times more potent than 1α,25$(OH)_2D_3$.
The substitution of a 2α-ethyl group in place of a 2α-methyl group on the A-ring of 19-nor-1α,25$(OH)_2D_3$ **(110)** decreased its prodifferentiating activity on HL60 cells. Nevertheless its activity was still 2 to 6 times more potent than 1α,25$(OH)_2D_3$. The 2β-ethyl counterpart of compound 24 **(109)** lost potency and was less active than 1α,25$(OH)_2D_3$. The introduction of the 23-yne side chain in the analogue 2α-ethyl-19-nor-1α,25$(OH)_2D_3$ **(112)** enhanced the antiproliferative (3-fold) effects on MCF-7 cells. The 2β-ethyl-19-nor-23-yne-1α,25$(OH)_2D_3$ (**111**) was again less active than 2α-efhyl-19-nor-23-yne-1α,25$(OH)_2D_3$ **(112).**

Examples 53 - In vivo activity of the 1α,25$(OH)_2D_3$ analogues

Eight weeks old, male NMRI mice were obtained from the Proefdierencentrum of Leuven (Belgium) and fed a vitamin D-replete diet (0.2% calcium, 1% phosphate, 2000 U vitamin D/kg; Hope Farms, Woerden, The Netherlands). Groups of six mice were subcutaneously injected daily during 7 consecutive days with different doses of 1α,25$(OH)_2D_3$ (0.1, 0.2 and 0.4 μg/kg/day) or analogues. The control group was injected with vehicle (arachis oil). The average weight of each group of 6 mice was determined at the beginning and at the end of the experiment. The following parameters were evaluated: serum calcium, serum osteocalcin, femur calcium and urinary calcium. Serum and urinary calcium were measured by a microcolorimetric assay (Sigma, St. Louis, MO). Femurs were removed and femur calcium content was measured in HCl-dissolved bone ash (obtained by heating for 24h in an oven at 100°C), using the same technique as for serum and urinary calcium. Serum osteocalcin was determined by an in-house radioimmunoassay that used mouse osteocalcin as standard and a polyclonal guinea pig anti-mouse osteocalcin antiserum (Bouillon R. et al. Clin Chem 38: 2055-60, 1992).

Results (Table 1-3)

The 2α-methyl-19-nor analogue with the natural side chain of 1α,25$(OH)_2D_3$ **(102)** was 2.5 times less calcemic than 1α,25$(OH)_2D_3$. Further reduction of calcemic activity (80-fold) could be obtained by introduction of a 2β-methyl A-ring **(101).** The introduction of the 23-yne side chain **(104)** or 14-epimerisation **(106)** or a combination of both **(108)** reduced the calcemic activity of 2α-methyl-19-nor-1α,25$(OH)_2D_3$ **(102).** The calcemic effects of 2β-methyl-19-nor-1α,25$(OH)_2D_3$ was not further reduced by introduction of unsaturation into the side chain (23-yne, **103),** 14-epimerisation **(105)** or a combination of both **(107).**
The substitution of a 2α-ethyl group in the A-ring of 19-nor-1α,25$(OH)_2D_3$ **(110)** was 7.6 times less calcemic than 1α,25$(OH)_2D_3$ and its 2β counterpart **(109)** further decreased the calcemic activity 16-fold. Again the introduction of the 23-yne side chain **(112)** or 14-epimerisation **(114)** or a combination of both **(116)** reduced the calcemic effects of 2α-ethyl-19-nor-1α,25$(OH)_2D_3$ **(110).** Only the combination of 14-epimerisation with the 23-yne side chain **(115)** reduced the calcemic effects of 2β-ethyl-19-nor-1α,25$(OH)_2D_3$ **(109).** Some of these novel compounds exhibited a very interesting pattern of biological activity with low effects on serum calcium levels together with anabolic actions on bone. For example, the mice treated during 7 consecutive days (i.p. 1μg/kg/d) with the analogue 2α-methyl-14-epi-19-nor-23-yne-1α,25$(OH)_2D_3$ **(108)** have low serum calcium levels compared to the mice treated with 1,25$(OH)_2D_3$ (0.1; 0.2 ; 0.4μg/kg/d; Table 3). Beside little or no effect on serum calcium levels this compound at a dose of 1μg/kg/d increased calcium levels in bone (10% increase compared to vehicle treated mice and 18%, 14%, 20% increase compared to 0.1, 0.2 and 0.4 μg/kg/d 1,25$(OH)_2D_3$ respectively; Table 3). Because of their preferential activity on bone; these compounds are ideal candidates for the treatment of bone disorders such as osteoporosis, osteomalacia and renal osteodystrophy.

Example 54 - Selectivity profile of the most potent C2-substituted 19-nor-1α,25$(OH_2D_3$ analogues

Table 4 represents the selectivity profile of the most potent C2-substituted 19-nor-1α,25$(OH)_2D_3$ analogues based on data obtained *in vitro* on MCF-7 cells compared with their actual *in vivo* calcemic effects in mice (serum calcium levels after 7 days treatment). All activities are calculated as percent activity compared with 1α,25$(OH)_2D_3$. The selectivity profile of is therefore 1. Although the C2α-methyl substituted 14-epi-19-nor-1α,25$(OH)_2D_3$ analogue with the 23-yne side chain **(108)** has the most potent intrinsic effect on cell differentiation and proliferation, the C2-ethyl substituted (2α

as well as 2β) 14-epi-19-nor-23-yne-1α,25$(OH)_2D_3$ analogues **(116** and **115)** show the best dissociation ratio between antiproliferative and calcemic effects. The selectivity profile of the analogues **116, 115** and **108** exceeds several fold that of the best analogues of 1α,25$(OH)_2D_3$ yet published when measured with the same methods in the same laboratory (Table 4) and such analogues might be useful in the treatment of hyperproliferative disorders (cancer, psoriasis) and autoimmune diseases.

Example 55 - Testing of compounds in an in vivo model for osteoporosis

*a) Primary Prevention of osteoporosis by vitamin D analogues*

'12 week old C3H female mice are subjected to bilateral ovariectomy or sham surgery. The animals are treated with the analog or vehicle by oral gavage or intraperitoneally. Dosing is started 3 days after sugery and continued for 8-9 weeks.

Before the first treatment *in vivo* measurements are performed to determine bone mineral density (BMD), bone mineral content (BMC) of total body and spine by dual-energy X-ray absorptiometry (DXA). Urine and serum is collected to measure calcium levels together with collagen cross-links in urine and osteocalcin in serum. The animals are weighed regularly during the experimental period. After 4 weeks treatment urine and serum is again collected and biochemical parameters are determined. At the end of the experiment (8-9 weeks) urine is collected and DXA measurement is performed *in vivo* to determine BMD and BMC. After killing the animals tibiae and femora are dissected The following biochemical parameters are investigated: serum calcium, serum osteocalcin, urine calcium, urine collagen cross-links, femur calcium.

Tibiae are used for histomorphometric analysis and femurs for measurement of cortical and trabecular volumetric density and geometry by peripheral quantitative computed tomography (pQCT) *ex vivo.*

In vivo administration of the analogues of the present invention, more particularly of compound **108** results in an increase in BMD, BMC, and femur calcium while serum calcium or urinary calcium levels are not significantly increased after at least four weeks, more particularly after 8 weeks of treatment.

*b) Secondary Prevention or treatment of osteoporosis by vitamin D analogues*

12 week old C3H female mice are subjected to bilateral ovariectomy or sham surgery. The animals are treated with the analog or vehicle by oral gavage or intraperitoneally. Dosing is started 4 weeks after sugery and continued for 4-10 weeks.

Before the first treatment *in vivo* measurements are performed to determine bone mineral density (BMD), bone mineral content (BMC) of total body and spine by dual-energy X-ray absorptiometry (DXA). Urine and serum is collected to measure calcium levels together with collagen cross-links in urine and osteocalcin in serum. The animals are weighed regularly during the experimental period. After 4 weeks treatment urine and serum is again collected and biochemical parameters are determined and urine is collected and DXA measurement is performed *in vivo* to determine BMD and BMC. After killing the animals tibiae and femora are dissected The following biochemical parameters are investigated: serum calcium, serum osteocalcin, urine calcium, urine collagen cross-links, femur calcium.

Tibiae are used for histomorphometric analysis and femurs for measurement of cortical and trabecular volumetric density and geometry by peripheral quantitative computed tomography (pQCT) *ex vivo.*

In vivo administration of the analogues of the present invention, more particularly of compound 108 results in an increase in BMD, BMC and femur calcium concentration as compared to the levels observed prior to treatment while serum calcium or urinary calcium levels are not significantly increased after four weeks, more particularly after 8 weeks of treatment. More particularly it is observed that the levels of BMD, BMC, and femur calcium are higher than the levels of sham operated control animals while serum calcium or urinary calcium levels are not significantly higher.

TABLE 1: 14-epi-19-nor-1α,25$(OH)_2$-$D_3$ analogues substituted on carbon 2

| |
|---|
| *All results are expressed as percentage activity (at 50% of the dose response) in comparison with 1α;25$(OH)_2D_3$ (=100% activity). The antiproliferative action of 1α,25$(OH)_2D_3$ or analogues on MCF-7 breast cancer cells was measured by [$^3$H]thymidine incorporation and the potency to differentiate HL 60 cells was determined by a nitroblue tetrazolium assay. The calcemic effects were determined in mice by a daily intraperitoneal injection of 1α, 25$(OH)_2D_3$ or analogues during 7 consecutive days.* |

(continued)

| COMPOUND | AFFINITY STUDIES | | IN VITRO STUDIES | | IN VIVO STUDIES | | |
|---|---|---|---|---|---|---|---|
| | VDR | DBP | HL 60 | MCF-7 | Calcium serum | Calcium urine | Osteocalcin serum |
| 1α,25$(OH)_2D_3$ | 100 | 100 | 100 | 100 | 100 | 100 | 100 |
| 14-epi-19-nor-1α,25$(OH)_2D_3$ | 19 | 15 | 50 | 30 | 0.3 | 0.4 | |
| **106** | 90 | 9 | 600 | 900 | 13 | 6 | 13 |
| **105** | 60 | 7 | 75 | 20 | 0.4 | 0.4 | 0.8 |
| **108** | 80 | 0.3 | 4000 | 12000 | 2 | 4 | 4 |
| **107** | 10 | 0 | 400 | 350 | 0.4 | 1.6 | 0.8 |
| **114** | 30 | 40 | 80 | 150 | 1 | 1 | 1 |
| **113** | 8 | 3 | 10 | 75 | 0.4 | 0.4 | 0.4 |
| **116** | 65 | 0.1 | 550 | 3000 | 0.2 | | 0.2 |
| **115** | 9 | 0.2 | 80 | 600 | << 0.1 | < 0.1 | 0.1 |
| **117** | 40 | 0 | 400 | | < 0.25 | < 0.25 | |
| **118** | 6 | 3 | 0 | | < 0.25 | < 0.25 | |
| **141** | 9 | | 1500 | 4500 | | | |
| **142** | 10 | | 400 | 1500 | | | |

TABLE 2: 19-nor-1α.25$(OH)_2D_3$ analogues substituted on carbon 2

*All results are expressed as percentage activity (at 50% of the dose response) in comparison with 1α,25$(OH)_2D_3$ (=100% activity). The antiproliferative action of 1α,25$(OH)_2D_3$ or analogues on MCF-7 breast cancer cells was measured by [$^3$H]thymidine incorporation and the potency to differentiate HL 60 cells was determined by a nitroblue tetrazolium assay. The calcemic effects were determined in mice by a daily intraperitoneal injection of 1α,25$(OH)_2D_3$ or analogues during 7 consecutive days.*

| COMPOUND | AFFINITY STUDIES | | IN VITRO STUDIES | | IN VIVO STUDIES | | |
|---|---|---|---|---|---|---|---|
| | VDR | DBP | HL 60 | MCF-7 | Calcium serum | Calcium urine | Osteocalcin serum |
| **102** | 90 | 50 | 1000 | 400 | 40 | | 15 |
| **101** | 75 | 9 | 70 | 80 | 0.5 | | |
| **104** | 70 | 4 | 1500 | 10500 | 10 | | 4 |
| **103** | 20 | 1 | 100 | 900 | 0.4 | 1.6 | 0.8 |
| **110** | 90 | 20 | 200 | 650 | 13 | 13 | 13 |
| **109** | 40 | 9 | 10 | 90 | 0.4 | 0.8 | 0.8 |
| **112** | 55 | 0.9 | 300 | 2000 | 1.6 | 0.8 | 3 |
| **111** | 9 | 0.5 | 80 | 700 | 0.4 | 0.8 | 0.8 |

TABLE 3: Calcemic effects of 2α-methyl-14,epi-19-nor-23-yne-1α,25$(OH)_2D_3$ (compound 8) in NMRI mice

| *In vivo biological activity of compound 8 determined by measuring serum and femur calcium levels in mice after intraperitoneally injections after 7 consecutive days. Mice were injected with vehicle (arachis oil), 1α,25-$(OH)_2$-$D_3$ (0.1, 0.2 or 0.4 μg/kg/d) or analogue (0.4 or 1 μg/kg/d).* | | | |
|---|---|---|---|
| **Compound** | **Dose μg/kg/d** | **Serum calcium (mg/dl)** | **Femur calcium (mg)** |
| Vehicle | | 10.14±0.22$ | 9.12±0.33 |
| 1,25$(OH)_2D_3$ | 0.1 | 11.79±0.58* | 8.9±0.50 |
| 1,25$(OH)_2D_3$ | 0.2 | 13.02±0.55* | 9.23±0.31 |
| 1,25$(OH)_2D_3$ | 0.4 | 14.81±0.57* | 8.75±0.15 |
| Compound **108** | 0.4 | 9.76±0.18$ | 9.57±0.26 |
| Compound **108** | 1 | 10.10±0.23$ | 10.52±0.43*,$ |
| Significantly different from vehicle treated group (p < 0.001)*<br>Significantly different from 1,25$(OH)_2D_3$ treated group at different doses (p < 0.001) $ | | | |

TABLE 4

**COMPARISON BETWEEN ANTIPROLIFERATIVE AND CALCEMIC ACTIVITY OF C2 SUBSTITUTED 19-NOR-1α,25$(OH)_2D_3$ ANALOGUES AND KNOWN SUPERAGONISTS OF 1α,25$(OH)_2D_3$.**

| Compounds | Antiproliferative activity on MCF-7 cells (%) | Serum calcium in mice (%) | Specificity ratio |
|---|---|---|---|
| 1α,25$(OH)_2D_3$ | 100 | 100 | 1 |
| 2α-ethyl-14-epi-19-nor-23-yne-1a,25$(OH)_2D_3$ (**116**) | 3000 | 0.2 | 15000 |
| 2β-ethyl-14-epi-19-nor-23-yne-1α,25$(OH)_2D_3$ (**115**) | 600 | <<0.1 | > 6000 |
| 2α-methyl-14-epi-19-nor-23-yne-1α,25$(OH)_2D_3$ (108) | 12000 | 2 | 6000 |
| 2β-methyl-19-nor-23-yne-1α,25$(OH)_2D_3$ (**103**) | 900 | 0.4 | 2250 |
| 2β-ethyl-19-nor-23-yne-1α,25$(OH)_2D_3$ (**111**) | 700 | 0.4 | 1750 |
| OCT* | 100-150 | 0.3 | 330-500 |
| MC 903* | 100-200 | 1 | 200 |
| KH 1060* | 10000-30000 | 500 | 20-60 |
| EB 1089* | 1000-10000 | 120 | 8-80 |

*All results are expressed as percentage activity (at 50% of the dose response) with 1α,25$(OH)_2D_3$ (=100% activity). The anti proliferative action of 1α,25$(OH)_2D_3$ on MCF-7 breast cancer cells was measured by [$^3$H]thymidine incorporation. The calcemic effects (serum calcium levels) were determined in mice by a daily intraperitoneal injection of 1α,25$(OH)_2D_3$ or analogues during 7 conssecutive days. * Data published in J Bone Mineral Res 15 (2): 237-252 (2000).*

## Claims

1. A 2-substituted-14-epi-19-nor-vitamin D analogue having the general formula (I)

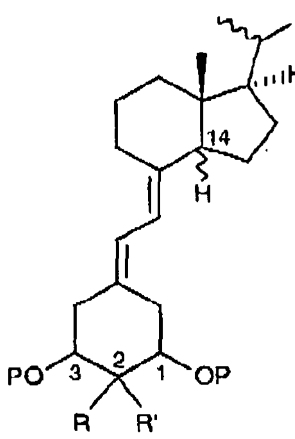

wherein:

- P is selected from the group consisting of hydrogen, alkyl, cycloalkyl, acyl, and other protecting groups;
- carbon atom 20 may have either the R or S configuration;
- the hydrogen atom at carbon atom 14 is in the β configuration;
- R' is hydrogen;
- R is selected from the group consisting of normal or branched alkyl groups having from 1 to 5 carbon atoms and being optionally substituted with one or more functional atoms or groups selected from the group consisting of fluoro, chloro, hydroxy, sulfhydryl and amino; provided that when R is an alkyl group with only one carbon atom, said group is in a configuration α at carbon 2;
- X represents an alkyl side-chain consisting of 2 to 15 carbon atom which can be substituted or functionalised as follows:

  • hydroxy substituent at one or more positions, for instance at position 24, 25 and/or 26, and/or
  • methyl or ethyl substituent in one or more positions, for instance at position 24, 26 and/or 27, and/or
  • halogen substituent(s) at one or more positions, for instance perfluorated at positions 26 and/or 27 or difluorated at position 24, and/or
  • esters derivatives or ether derivatives of one or more hydroxyl substituents mentioned above, and/or
  • changing one or more carbon atoms for an oxygen, nitrogen or sulfur atom, for instance at one or more of positions 22, 23 and 24, and/or
  • cyclized between the carbon atoms 26 and 27 by one bond (cyclopropane) or by the intermediacy of 1 to 4 carbon atoms, the resulting ring being saturated, unsaturated or aromatic and being optionally substituted at any possible position(s) with one or more substituents mentioned above, and/or
  • cyclized at one carbon or between two carbon atoms by 1 to 4 atoms to form a heterocyclic ring, including aromatic, which may be substituted at any possible position with one or more substituents mentioned above, and/or
  • unsaturated with one or more double or triple C-C bond(s), these unsaturated chains being optionally substituted at any possible position by one or more substituents mentioned above, and/or
  • epoxidized once or more between carbon atoms (preferably between 22 and 23, or between 23 and 24, or between 24 and 25, or between 25 and 26), the resulting epoxidized chain(s) being saturated or unsaturated and, when saturated, optionally substituted at any possible positions with one or more substituents mentioned above, and/or
  • two or more of the carbon atoms of said alkyl side-chain X being linked by a single bond or by the intermediacy of 1 to 5 carbon or oxygen or nitrogen or sulfur atoms to form a 3-7 membered saturated or unsaturated carbocyclic (including aromatic) or heterocyclic ring which may be substituted at any possible position(s) by one or more substituents mentioned above, and/or
  • substituted at one or more positions by one or more saturated or unsaturated, carbocyclic (including aromatic) or heterocyclic rings which can be substituted at any possible position(s) with one or more substituents mentioned above,
  • including all possible isomeric forms of said alkyl side-chain X.

2. A 2-substituted-14-epi-19-nor-vitamin D analogue according to claim 1, wherein R is an alkyl group with one carbon atom.

3. A 2-substituted-14-epi-19-nor-vitamin D analogue according to claim 1 or claim 2, wherein said group R is in a configuration α at carbon 2.

4. A 2-substituted-14-epi-19-nor-vitamin D analogue according to claim 1, wherein R is an alkyl group having 2 carbon atoms.

5. A 2-substituted-14-epi-19-nor-vitamin D analogue according to claim 2, wherein the OP group is in a configuration α at carbon 1.

6. A 2-substituted-14-epi-19-nor-vitamin D analogue according to claim 2, wherein the OP group is in a configuration α at carbon 3.

7. A 2-substituted-14-epi-19-nor-vitamin D analogue according to claim 2, wherein the OP group is in a configuration β at carbon 3.

8. A 2-substituted-14-epi-19-nor-vitamin D analogue having one of the following structures:

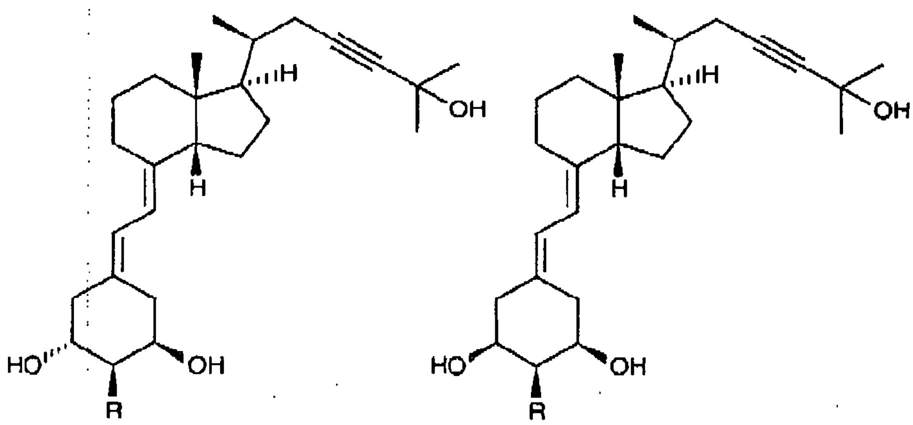

**141** R = Me
**142** R = Et

**143** R = Me
**144** R = Et

9. A 2-substituted-14-epi-19-nor-vitamin D analogue according to claim 1, having one of the following structures:

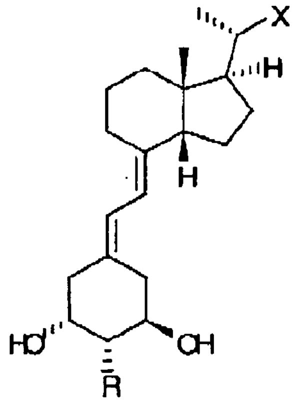

**105, 107, 113, 115**

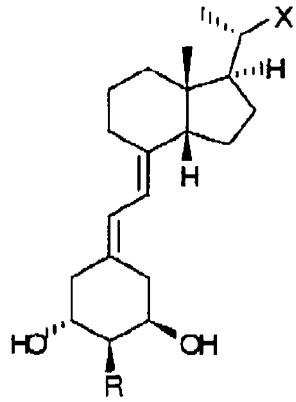

**106, 108, 114, 116**

**105** R = Me X = 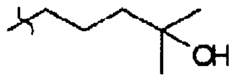

**106** R = Me X = 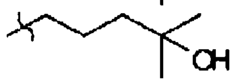

**107** R = Me X = 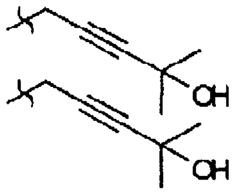

**108** R = Me X =

**113** R = Et X = 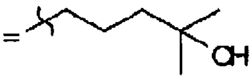

**114** R = Et X = 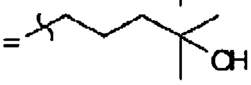

**115** R = Et X = 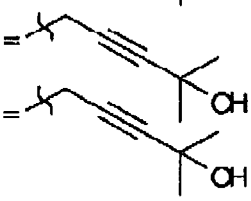

**116** R = Et X =

**10.** A 2-substituted-14-epi-19-nor-vitamin D analogue according to claim 1, having one of the following structures:

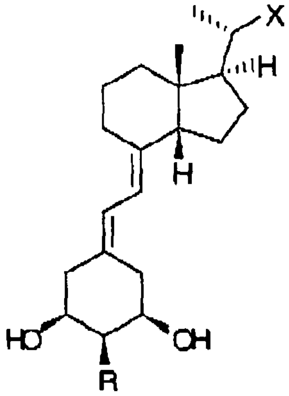

**117,119,120,121**

**117** R = Me X = 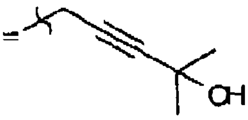

**119** R = Me X = 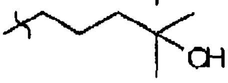

**120** R =Et X = 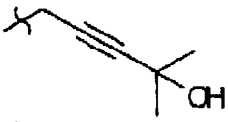

**121** R =Et X = 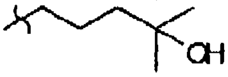

**11.** A 2-substituted-14-epi-19-nor-vitamin D analogue according to claim 1, having one of the following structures:

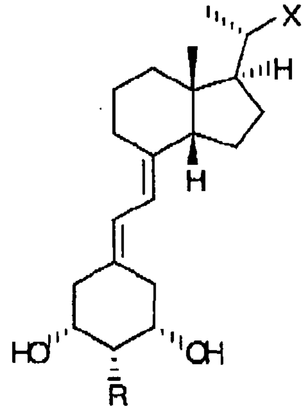

**118, 126, 127, 128**

**118** R = Me X = 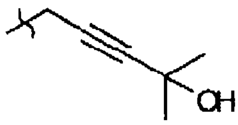

**126** R = Me X = 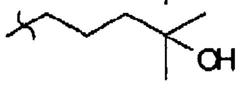

**127** R = Et X = 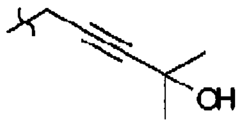

**128** R = Et X = 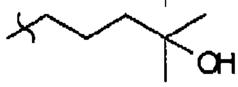

**12.** A pharmaceutically acceptable salt of a 2-substituted-14-epi-19-nor-vitamin D analogue according to any of claims 1 to 11.

**13.** A solvate of a 2-substituted-14-epi-19-nor-vitamin D analogue according to any of claims 1 to 11 or a salt thereof.

**14.** A Pharmaceutical preparation comprising a therapeutically effective amount of a 2-substituted-14-epi-19-nor-vitamin D analogue according to claim 8, a pharmaceutically acceptable salt or a solvate thereof, and one or more pharmaceutically and/or veterinarily acceptable carriers or diluents.

**15.** A pharmaceutical preparation comprising a therapeutically effective amount of a 2-substituted-14-epi-19-nor-vitamin D analogue according to any of claims 1 to 7 and 9 to 11, a pharmaceutically acceptable salt or a solvate thereof, and one or more pharmaceutically and/or veterinarily acceptable carriers or diluents.

**16.** A pharmaceutical preparation according to claim 15, further comprising an effective amount of an immuno-modulating agent.

**17.** A pharmaceutical preparation according to claim 15, further comprising an effective amount of an anti-cancer agent,

**18.** Use of a 2-substituted-14-epi-19-nor-vitamin D analogue according to any of claims 1 to 11, a pharmaceutically acceptable salt or a solvate thereof, for the preparation of a medicament for inhibiting cell proliferation and/or induction of cell differentiation.

**19.** Use of a 2-substituted-14-epi-19-nor-vitamin D analogue according to any of claims 1 to 11, a pharmaceutically acceptable salt or a solvate thereof, for the preparation of a medicament for therapy and/or prevention of immu nodisorders.

**20.** Use of a 2-substituted-14-epi-19-nor-vitamin D analogue according to any of claims 1 to 11, a pharmaceutically acceptable salt or a solvate thereof, for the preparation of a medicament for therapy and/or prevention of inflammatory diseases; skin disorders; hyperproliferative disorders or cancer.

**21.** Use of a 2-substituted-14-epi-19-nor-vitamin D analogue according to any of claims 1 to 11, a pharmaceutically acceptable salt or a solvate thereof, for the preparation of a medicament for treating a metabolic bone disease.

**22.** Use according to claim 21, wherein said metabolic bone disease is osteoporosis.

**Patentansprüche**

**1.** 2-substituiertes-14-epi-19-nor-vitamin D-Analog mit der allgemeinen Formel (I):

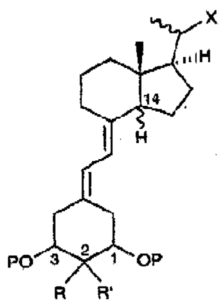

wobei:

- P ausgewählt ist aus der Gruppe betstehend aus Wasserstoff, Alkyl, Cycloalkyl, Acyl und anderen Schutzgruppen;
- das Kohlenstoffatom 20 entweder die R- oder S-Konfiguration haben könnte;
- das Wasserstoffatom beim Kohlenstoffatom 14 in der β-Konfiguration ist;
- R' Wasserstoff ist;
- R ausgewählt ist aus der Gruppe bestehend aus normalen oder verzweigten Alkylgruppen mit 1 bis 5 Kohlenstoffatomen und wahlweise mit einem bzw. einer oder mehreren funktionellen Atom(en) oder Gruppe(n) substituiert ist, die ausgewählt sind aus der Gruppe bestehend aus Fluoro, Chloro, Hydroxy, Sulfhydryl und Amino; vorausgesetzt, dass, wenn R eine Alyklgruppe mit nur einem Kohlenstoffatom ist, die Gruppe in einer α-Konfiguration bei Kohlenstoff 2 ist;

  - X eine Alkylseitenkette darstellt, die aus 2 bis 15 Kohlenstoffatomen besteht, die wie folgt substituiert oder funktionalisiert sein kann:
  - Hydroxylsubstituent an einer oder mehreren Position(en), zum Beispiel an Position 24, 25 und/oder 26, und/oder
  - Methyl- oder Ethylsubstituent an einer oder mehreren Pcsition(en), zum Beispiel an Position 24, 26 und/oder 27, und/oder
  - Halogensubsituent(en) an einer oder mehreren Position(en), zum Beispiel perfluoriert an Positionen 26 und/oder 27 oder difluoriert an Position 24, und/oder
  - Esterderivate oder Etherderivate eines oder mehrerer der oben genannten Hydroxylsubstituenten, und/oder
  - Ändern eines oder mehrerer Kohlenstoffatome für ein Sauerstoff-, Stickstoff- oder Schwefelatom, zum Beispiel an einer oder mehreren der Positionen 22, 23 und 24, und/oder
  - cyclisiert zwischen den Kohlenstoffatomen 26 und 27 durch eine Bindung (Cyclopropan) oder durch dazwischen liegende 1 bis 4 Kohlenstoffatome, wobei der erhaltene Ring gesättigt, ungesättigt oder aromatisch ist und wahlweise an (einer) möglichen Position(an) mit einem oder mehreren der oben genannten Substituenten substitutiert ist, und/oder
  - cyclisiert an einem Kohlenstoff oder zwischen zwei Kohlenstoffatomen durch 1 bis 4 atome zur Bildung eines heterocyclischen, einschließlich aromatischen, Rings, der an einer möglichen Position mit einem oder mehreren der oben genannten Substituenten substituiert sein kann, und/oder

- ungesättigt mit einer oder mehreren doppelten oder dreifachen C-C-Bindung(en), wobei diese ungesättigten Ketten wahlweise an einer möglichen Position durch einen oder mehrere der oben genannten Substituenten substituiert sein können, und/oder
- einmal oder mehr zwischen Kohlenstoffatomen (vorzugsweise zwischen 22 und 23 oder zwischen 23 und 24 oder zwischen 24 und 25 oder zwischen 25 und 26) epoxidiert, wobei die erhaltene(n) epoxidierte(n) Kette(n) gesättigt oder ungesättigt ist bzw, sind und, falls gesättigt, wahlweise an einer möglichen Position mit einem oder mehreren der oben genannten Substituenten substituiert sein kann bzw. können, und/oder
- zwei oder mehr der Kohlenstoffatome der Alkylseitenkette X durch eine einzige Bindung oder durch dazwischen liegende 1 bis 5 Kohlenstoff- oder Sauerstoff- oder Stickstoff- oder Schwefelatome verbunden sind, um einen 3-7-gliedrigen gesättigen oder ungesättigten carbocyclischen (einschließlich aromatischen) oder heterocyclischen Ring zu bilden, der an einer möglichen Position mit einem oder mehreren der oben genannten Substituenten substituiert sein kann, und/oder
- Substituiert an einer oder mehreren Position(en) durch einen oder mehrere gesättigte(n) oder ungesättigten (n), carbocyclische(n) (einschließlich aromatische(n)) oder heterocyclische(n) Ring(e), der bzw. die an einer oder mehreren möglichen Position(en) mit einem oder mehreren der oben genannten Substituenten substituiert sein kann bzw. können,
- einschließlich aller möglichen isomeren Formen der Alkylseitenkette X.

**2.** 2-substituiertes-14-epi-19-nox-Vitamin D-Analog nach Anspruch 1, wobei R eine Alkylgruppe mit einem Kohlenstoffatom ist.

**3.** 2-substituiertes-14-epi-19-nor-Vitamin D-Analog nach Anspruch 1 oder Anspruch 2, wobei die Gruppe R bei Kohlenstoff 2 in einer α-Konfiguration ist.

**4.** 2-substituiertes-14-epi-19-nor-Vitamin D-Analog nach Anspruch 1, wobei R eine Alkylgruppe mit 2 Kohlenstoffatomen ist.

**5.** 2-substituiertes-14-epi-19-nor-Vitamin D-Analog nach Anspruch 2, wobei die OP-Gruppe bei Kohlenstoff 1 in einer α-Konfiguration ist.

**6.** 2-substituiertes-14-epi-19-nor-Vitamin D-Analog nach Anspruch 2, wobei die OP-Gruppe bei Kohlenstoff 3 in einer α-Konfiguration ist.

**7.** 2-substituzertes-14-epi-19-nor-Vitamin D-Analog nach Anspruch 2, wobei die OP-Gruppe bei Kohlenstoff 3 in einer β-Konfiguration ist.

**8.** 2-substituiertes-14-epi-19-nor-Vitamin D-Analog mit einer der folgenden Strukturen:

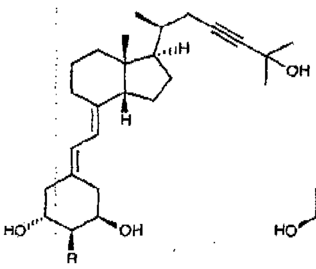

141 R = Me
142 R = Et

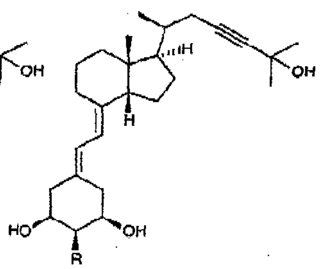

143 R = Me
144 R = Et

**9.** 2-substituiertes-14-epi-19-nor-Vitamin D-Analog nach Anspruch 1 mit einer der folgenden Strukturen:

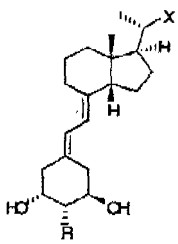

105, 107, 113, 115

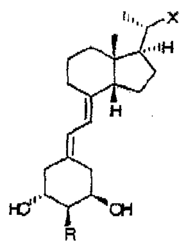

106, 108, 114, 116

105 R = Me X = / 106 R = Me X = / 107 R = Me X = / 108 R = Me X =

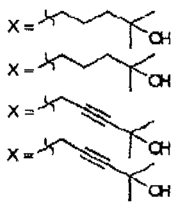

113 R = Et X = / 114 R = Et X = / 115 R = Et X = / 116 R = Et X =

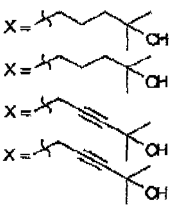

**10.** 2-substituiertes-14-epi-19-nor-Vitamin D-Analog nach Anspruch 1 mit einer der folgenden Strukturen:

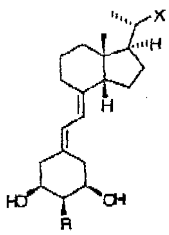

117,119,120,121

117 R = Me X =

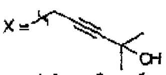

119 R = Me X =

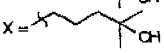

120 R =Et X =

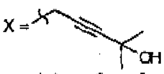

121 R =Et X =

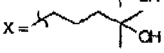

**11.** 2-Substituiertes-14-epi-19-nor-Vitamin D-Analog nach Anspruch 1 mit einer der folgenden Strukturen:

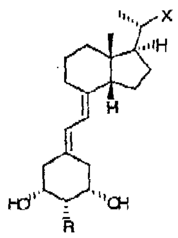

118, 126, 127, 128

118 R = Me X = 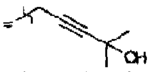

126 R = Me X = 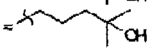

127 R = Et X = 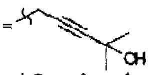

128 R = Et X = 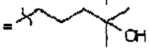

**12.** pharmazeutisch annehmbares Salz eines 2-substituierten-14-epi-19-nor-Vitainin D-Analogs nach einem der Ansprüche 1 bis 11.

**13.** Solvat eines 2-substituierten-14-epi-19-nor-Vitamin D-Analogs nach einem der Ansprüche 1 bis 11 oder ein Salz davon.

**14.** Pharmazeutische Zubereitung, umfassend eine therapeutisch wirksame Menge eines 2-substituierten-14-epi-19-nor-Vitamin D-Analogs nach Anspruch 8, eines pharmazeutisch annehmbaren Salzes oder eines Solvates davon, und ein(en) oder mehrere pharmazeutisch und/oder veterinärisch annehmbare(n) Träger oder annehmbare(s) Verdünnungsmittel.

**15.** Pharmazeutische Zubereitung, umfassend eine therapeutisch wirksame Menge eines 2-substituierten-14-epi-19-nor-Vitamin D-Analogs nach einem der Ansprüche 1 bis 7 und 9 bis 11, eines pharmazeutisch annehmbaren Salzes oder eines Solvates davon, und ein(en) oder mehrere pharmazeutisch und/oder veterinärisch annehmbare(n) Träger oder annehmbare(s) Verdünnungsmittel.

**16.** Pharmazeutische Zubereitung nach Anspruch 15, des Weiteren umfassend eine wirksame Menge eines immunmodulierenden Mittels.

**17.** Pharmazeutische Zubereitung nach Anspruch 15, des Weiteren umfassend eine wirksame Menge eines Antikrebsmittels.

**18.** Verwendung eines 2-substituierten-14-epi-19-nor-Vitamin D-Analogs nach einem der Ansprüche 1 bis 11, eines pharmazeutisch annehmbaren Salzes oder eines Solvates davon für die Herstellung eines Medikaments zur Hemmung einer Zellproliferation und/oder Auslösung einer zelldifferenzierung.

**19.** Verwendung eines 2-substituierten-14-epi-19-nor-Vitamin D-Analogs nach einem der Ansprüche 1 bis 11, eines pharmazeutisch annehmbaren Salzes oder eines Solvates davon für die Herstellung eines Medikaments zur Therapie und/oder Prävention von Immunstörungen.

**20.** Verwendung eines 2-substituierten-14-epi-19-nor-Vitamin D-Analogs nach einem der Ansprüche 1 bis 11, eines pharmazeutisch annehmbaren Salzes oder eines Solvates davon für die Herstellung eines Medikaments zur Therapie und/oder Prävention von Entzündungserkankungen; Hautkrankheiten, hyperproliferativer Erkrankungen oder Krebs.

**21.** Verwendung eines 2-substitulerten-14-epi-19-nor-Vitanin D-Analogs nach einem der Ansprüche 1 bis 11, eines pharmazeutisch annehmbaren Salzes oder eines Solvates davon für die Herstellung eines Medikaments zur Behandlung einer metabolischen Knuchenkrankheit.

**22.** Verwendung nach Anspruch 21, Möbel die metabolische Knochenkrankheit Osteoporose ist.

## Revendications

**1.** Un analogue de vitamine D 2-substituée 14-epi 19-nor ayant la formule général (1):

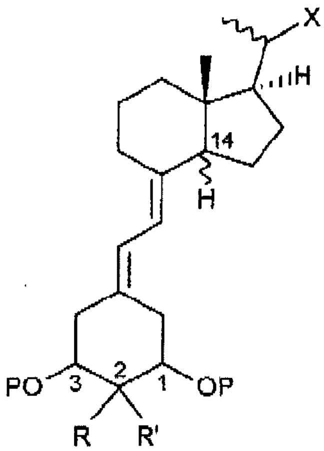

dans laquelle :

- P est sélectionné parmi le groupe constitué de hydrogène alcoyle cycloalcoyle, acyle, et d'autres groupes protecteurs ;
- l' atome de carbone 20 peut avoir la configuration soit R soit S ;
- l'atome d'hydrogène situé à l'atome de carbone 14 est dans la configuration β ;
- R' est l'hydrogène;
- R est sélectionné parmi le groupe constitué de groupes alcoyles normaux ou branchés ayant de 1 à 5 atomes de carbone et étant le cas échéant substitués avec un ou plusieurs atomes ou groupes fonctionnels sélectionnés parmi le groupe constitué de fluoro, chloro, hydroxyle , sulfhydryle et amino ; à condition que lorsque R est un groupe alcoyle avec un seul atome de carbone , ledit groupe est dans une configuration α au carbone 2 ;
- X représente une chaîne latérale alcoyle constituée de 2 à 15 atomes de carbone qui peut être substituée ou fonctionnalisée comme suit :

substituant hydroxyde à une ou plusieurs positions , par exemple à la position 24 , 25 et / ou 26 , et / ou substituant méthyle ou éthyle à une ou plusieurs positions , par exemple à la position 24 , 26 et / ou 27 , et / ou substituant(s) halogène à une ou plusieurs positions par exemple perfluorés aux positions 25 et / ou 27 ou difluorés à la position 24, et / ou

• des dérivés esters ou des dérivés éther d' un ou plusieurs substituants hydroxyle mentionnés ci-dessus , et 1 ou

• changer un ou plusieurs atomes de carbone pour un atome d'oxygène, d' azote ou de soufre, par exemple à une ou plusieurs des positions 22 , 23 et 24 , et / ou

• cyclisée entre les atomes de carbone 26 et 27 par une liaison (cyclopropane) ou par l'intermédiaire de 1 à 4 atomes de carbone , l' anneau résultant étant saturé , insaturé ou aromatique et étant le cas échéant substitué à l'une quelconque des position(s) possible(s) par un ou plusieurs substituants mentionnés ci-dessus , et / ou

• cyclisée à un atome de carbone ou entre deux atomes de carbone par 1 à 4 atomes pour former un anneau hétérocyclique , y compris aromatique , qui peut être substitué à une quelconque position possible par un ou plusieurs substituants mentionnés ci-dessus , et / ou

• insaturée avec une ou plusieurs liaison(s) C - C double(s) ou triple(s) , ces chaînes insaturées étant le cas échéant substituées à une quelconque position possible par un ou plusieurs substituants mentionnés ci-dessus et /ou

• époxidisée une fois ou davantage entre les atomes de carbone (de préférence entre 22 et 23 , ou entre

23 et 24 , ou entre 24 et 25 , ou entre 25 et 26), les chaîne(s) époxidisées résultantes étant saturées ou insaturées et , lorsque saturées , le cas échéant substituées à une quelconque position possible avec un ou plusieurs substituants mentionnés ci-dessus , et / ou

- deux ou plus des atomes de carbone de la dite chaîne latérale alcoyle X étant liés par une liaison simple ou bien par l'intermédiaire de 1 à 5 atomes de carbone ou d' oxygène ou d'azote ou de soufre pour former un anneau carbocyclique (y compris aromatique) ou hétérocyclique saturé ou insaturé de 3 à 7 membres qui peut être substitué à l'une quelconque des positions possibles avec un ou plusieurs substituants mentionnés ci-dessus , et / ou

• substituée à une position ou davantage par un ou plusieurs anneaux carbocycliques (y compris aromatiques) ou hétérocycliques saturés ou insaturés qui peuvent être substitués à l'une quelconque des position (s) possibles par un ou plusieurs substituants mentionnés ci-dessus ,

y compris toutes les formes isomériques possibles de la dite chaîne latérale alcoyle X .

**2.** Un analogue de vitamine D 2-substituée 14-epi 19-nor selon la revendication 1 , dans lequel R est un groupe alcoyle avec un atome de carbone.

**3.** Un analogue de vitamine D 2-substituée 14-epi 19-nor selon la revendication 1 ou la revendication 2 , dans laquel le dit groupe R est dans une configuration a au carbone 2 .

**4.** Un analogue de vitamine D 2-substituée 14-epi 19-nor selon la revendication 1 , dans lequel R est un groupe alcoyle ayant 2 atomes de carbone.

**5.** Un analogue de vitamine D 2-substituée 14-epi 19-nor selon la revendication 2 , dans lequel le groupe OP est dans une configuration $\alpha$ au carbone 1 .

**6.** Un analogue de vitamine D 2-substituée 14-epi 19-noir selon la revendication 2, dans lequel le groupe OP est dans une configuration $\alpha$ au carbone 3.

**7.** Un analogue de vitamine D 2-substituée 14-epi 19-nor selon la revendication 2 , dans laquel le groupe OP est dans une configuration $\beta$ au carbone 3 .

**8.** Un analogue de vitamine D 2-substituée 14-epi 19-nor ayant l'une des structures suivantes :

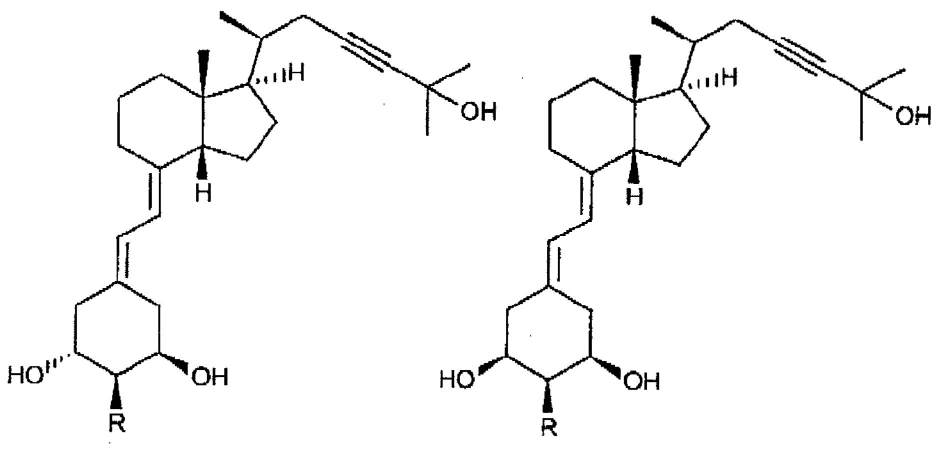

**141** R = Me
**142** R = Et

**143** R = Me
**144** R = Et

**9.** Un analogue de vitamine D 2-substituée 14-pi 19-nor selon la revendication 1 , ayant l'une des structures suivantes :

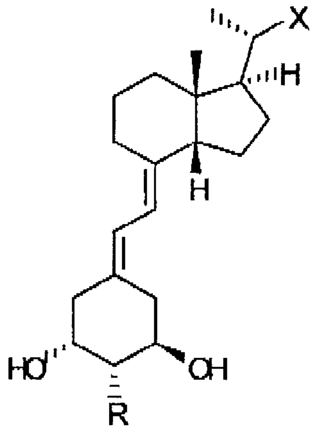

**105, 107, 113, 115**

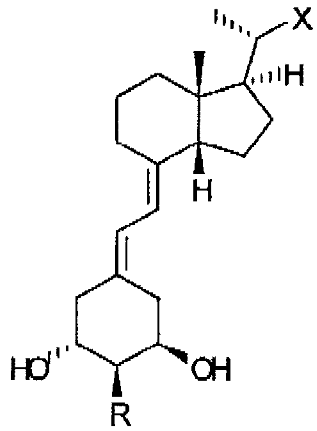

**106, 108, 114, 116**

**105** R = Me X = 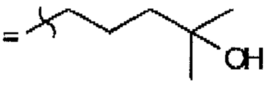

**106** R = Me X = 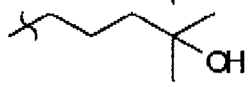

**107** R = Me X = 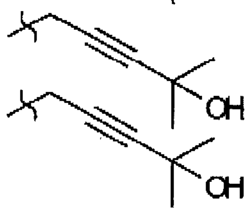

**108** R = Me X =

**113** R = Et X = 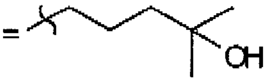

**114** R = Et X = 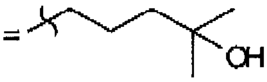

**115** R = Et X = 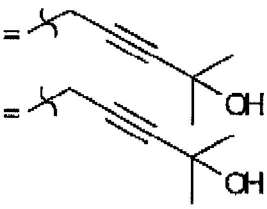

**116** R = Et X =

**10.** Un analogue de vitamine D 2-substituée 14-epi 19-nor selon la revendication 1 , ayant l'une des structures suivantes :

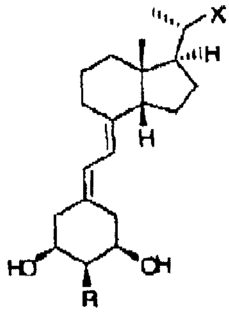

117,119,120,121

117 R = Me X = 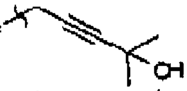

119 R = Me X = 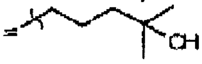

120 R = Et X = 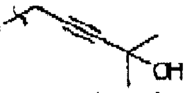

121 R = Et X = 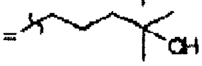

**11.** Un analogue de vitamine D 2-substituée 14-epi 19-nor selon la revendication 1, ayant l'une des structures suivantes :

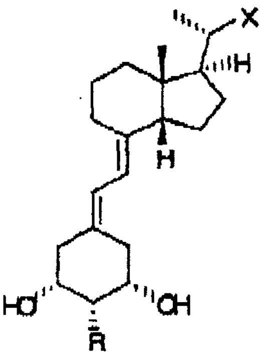

118, 126, 127, 128

118 R = Me X = 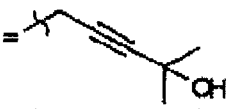

126 R = Me X = 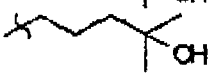

127 R = Et X = 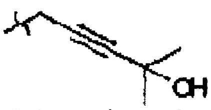

128 R = Et X = 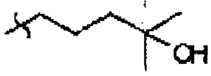

**12.** Un sel pharmaceutiquement acceptable d' un analogue de vitamine D 2-substituée 14-epi 19-nor selon l'une quelconque des revendications 1 à 11 .

**13.** Un solvate d' un analogue de vitamine D 2-substituée 14-epi 19-nor selon l'une quelconque des revendications 1 à 11 ou bien un sel de celui-ci .

**14.** Une préparation pharmaceutique comprenant une quantité thérapeutiquement efficace d'un analogue de vitamine D 2-substituée 14-epi 19-nor selon la revendication 8 , un sel pharmaceutiquement acceptable ou bien un solvate de celui-ci, et un ou plusieurs supports ou diluants pharmaceutiquement et / ou vétérinairement acceptables.

**15.** Une préparation pharmaceutique comprenant une quantité thérapeutiquement efficace d' un analogue de vitamine D 2-substituée 14-epi 19-nor selon l'une quelconque des revendications 1 à 7 et 9 à 11 , un sel pharmaceutiquement acceptable ou bien un solvate de celui-ci , et un ou plusieurs supports ou diluants pharmaceutiquement et / ou vétérinalrement acceptables .

**16.** Une préparation pharmaceutique selon la revendication 15 , comprenant en outre une quantité efficace d' un agent modulateur de l'immunité .

**17.** Une préparation pharmaceutique selon la revendication 15 , comprenant en outre une quantité efficace d' un agent anti-cancéreux .

**18.** Utilisation d' un analogue de vitamine D 2-substitutée 14-epi 19-noir selon l'une quelconque des revendications 1 à 11 , d' un sel pharmaceutiquement acceptable ou bien d' un solvate de celui-ci , pour la préparation d' un médicament pour inhiber la prolifération des cellules et / ou l'induction de la différenciation des cellules .

**19.** Utilisation d' un analogue de vitamine D 2-substituée 14-epi 19-nor selon l' une quelconque des revendications 1 à 11 , d' un sel pharmaceutiquement acceptable ou d' un solvate de celui-ci , pour la préparation d' un médicament pour la thérapie et / ou la prévention de désordres immunitaires .

**20.** Utilisation d'un analogue de vitamine D 2-substituée 14-epi 19-nor selon l' une quelconque des revendications 1 à 11 , d' un sel pharmaceutiquement acceptable ou d'un solvate de celui-ci , pour la préparation d'un médicament pour la thérapie et / ou la prévention de maladies inflammatoires , de désordres de la peau , de désordres hyperprolifératifs ou du cancer.

**21.** Utilisation d' un analogue de vitamine D 2-substituée 14-epi 19-nor selon l' une quelconque des revendications 1 à 11 , d' un sel pharmaceutiquement acceptable ou d' un solvate de celui-ci , pour la préparation d' un médicament pour traiter une maladie métabolique des os .

**22.** Utilisation selon la revendication 21 , dans laquelle la dite maladie métabolique des os est l'ostéoporose.

Figure 1

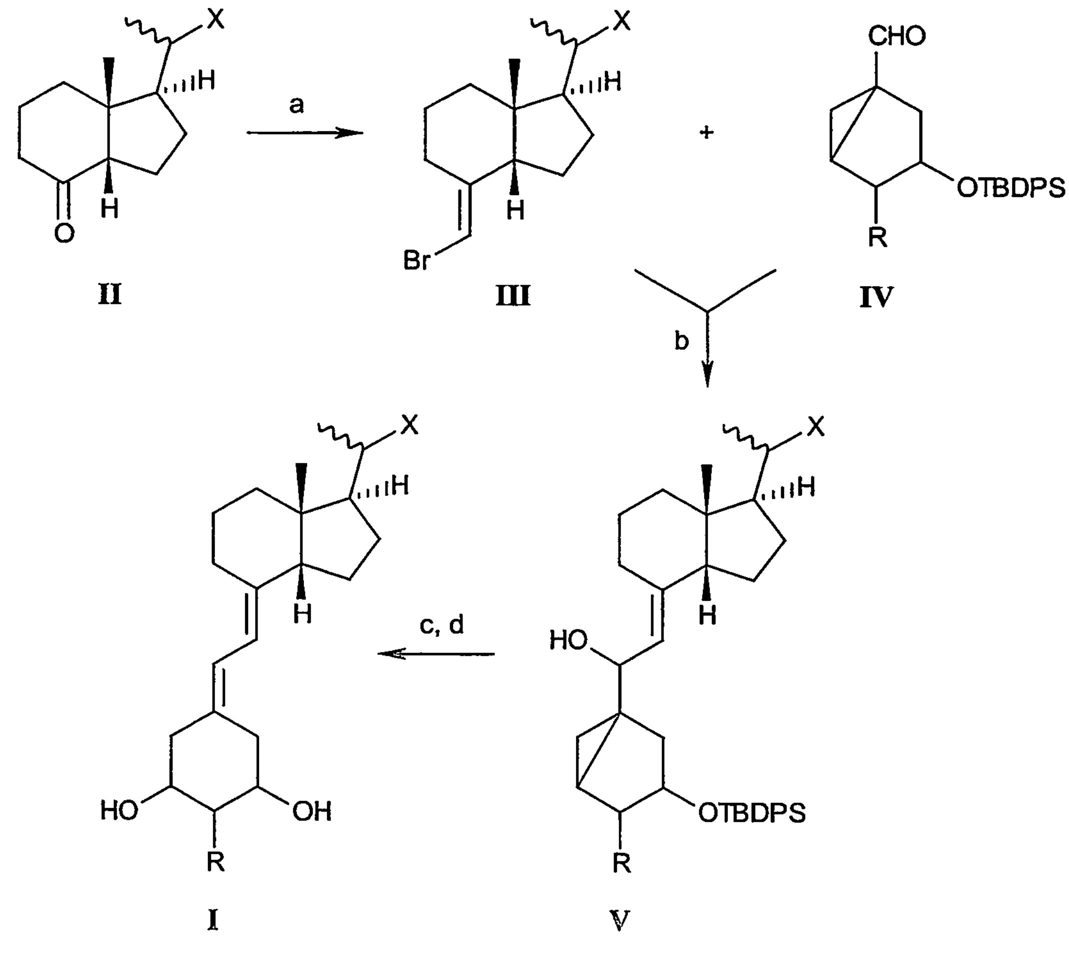


II

III

IV

I

V

Figure 2

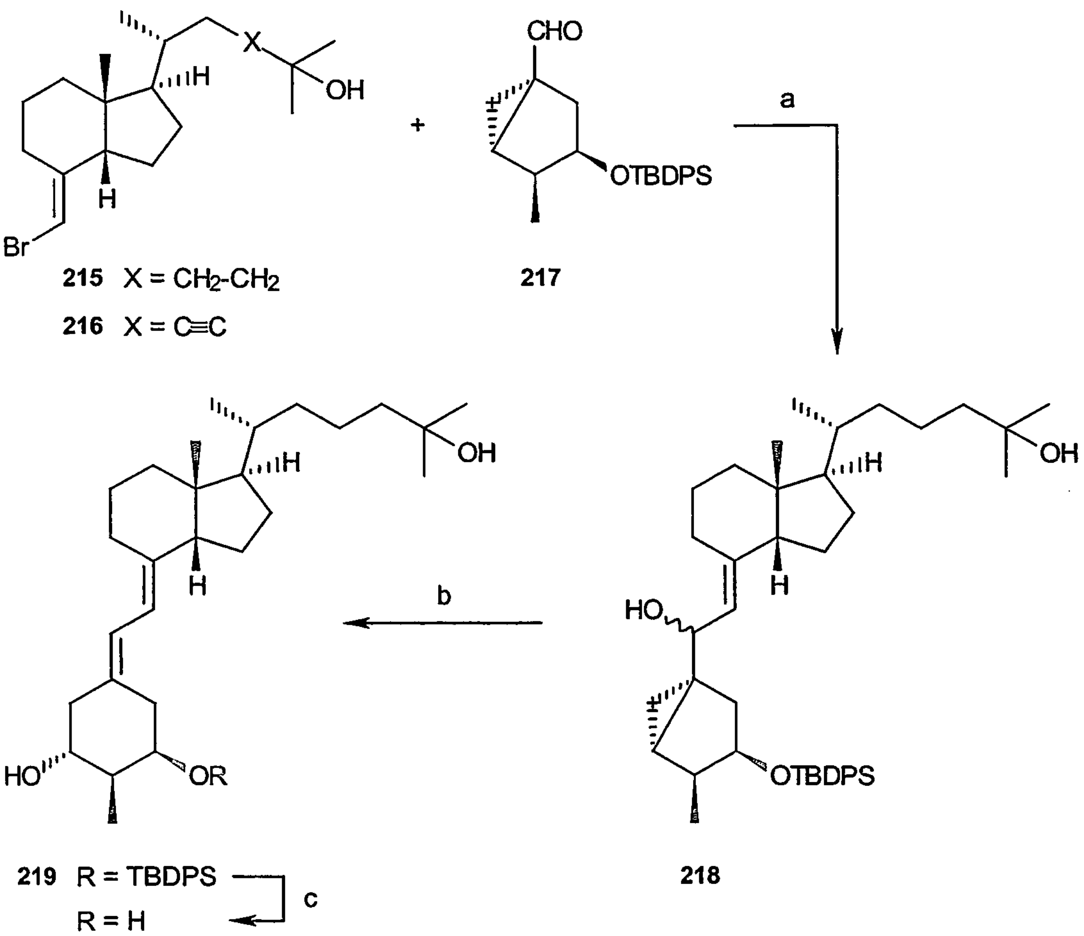


215 X = $CH_2$-$CH_2$

216 X = C≡C

217

218

219 R = TBDPS

R = H

## Figure 3

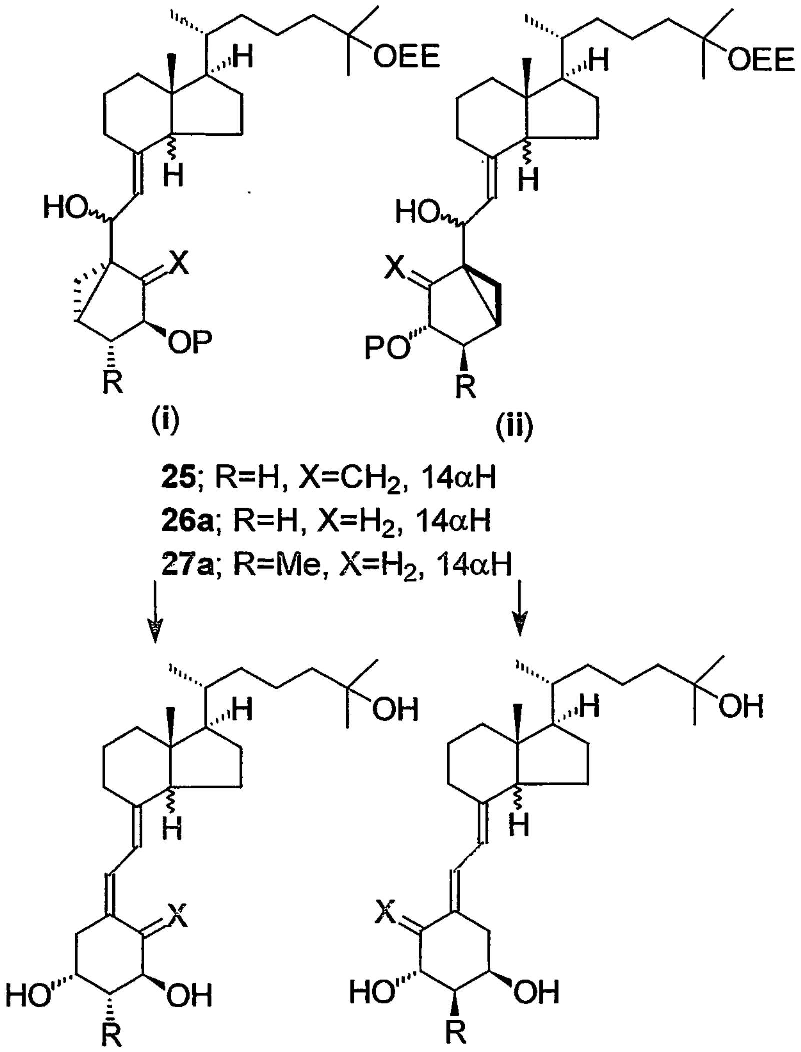

(i) (ii)

**25**; R=H, X=$CH_2$, 14αH
**26a**; R=H, X=$H_2$, 14αH
**27a**; R=Me, X=$H_2$, 14αH

**1**; R=H, X=$CH_2$, 14αH **28**; R=H, X=$CH_2$, 14αH
**2**; R=H, X=$CH_2$, 14αH **102**; R=Me, X=$H_2$, 14αH
**101**; R=Me, X=$H_2$, 14αH

P=TBDPS

**18, 19, 20, 21**; S = 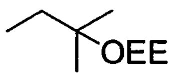

**18', 19', 20', 21'**; S = 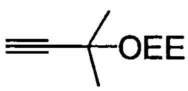

## Figure 4

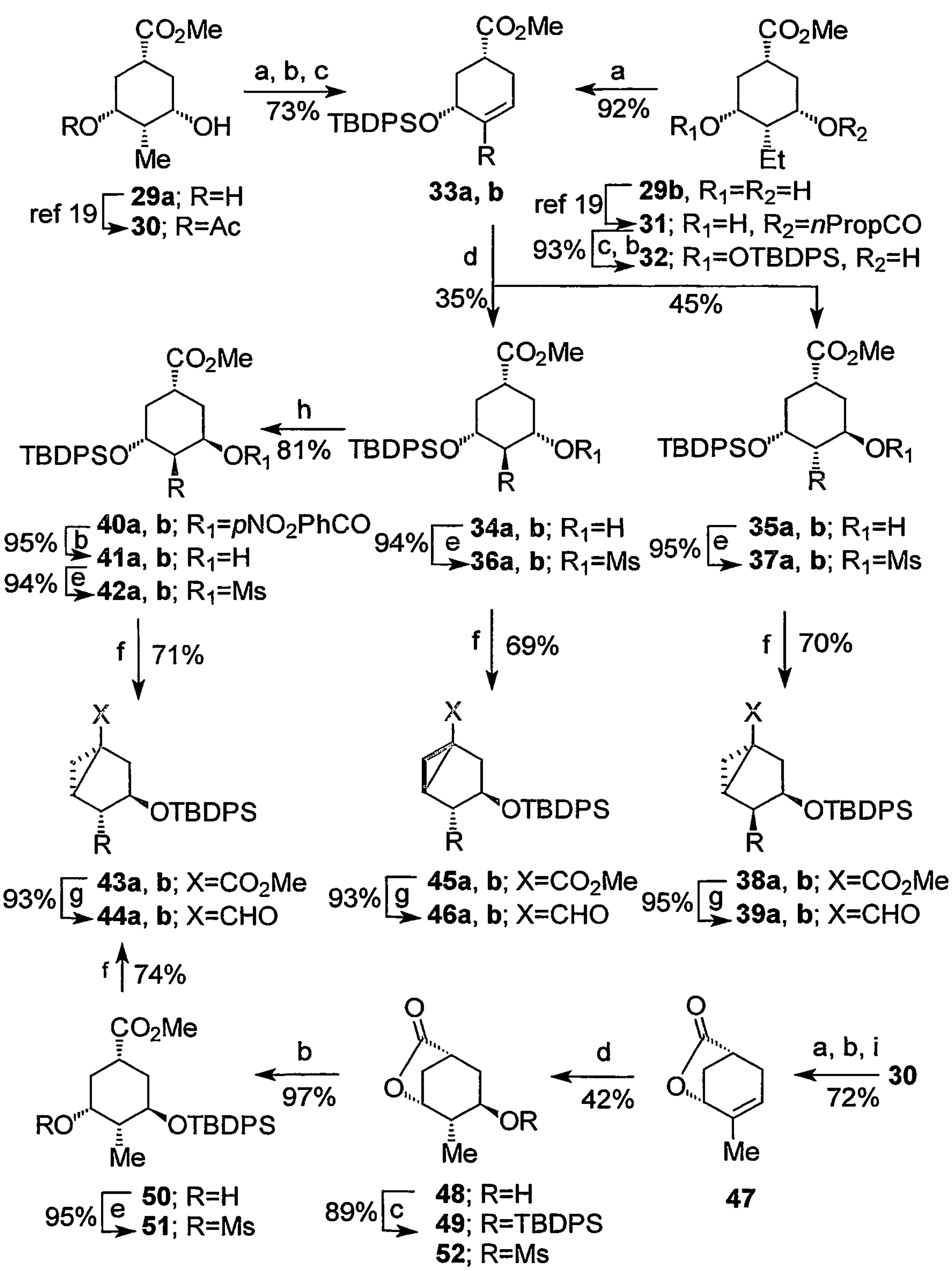
a, b, c
73%
a
92%
29a; R=H
ref 19
30; R=Ac
33a, b
ref 19
29b, $R_1$=$R_2$=H
31; $R_1$=H, $R_2$=nPropCO
93% c, b
32; $R_1$=OTBDPS, $R_2$=H
d
35%
45%
h
81%
95% b
40a, b; $R_1$=pNO$_2$PhCO
41a, b; $R_1$=H
94% e
42a, b; $R_1$=Ms
94% e
34a, b; $R_1$=H
36a, b; $R_1$=Ms
95% e
35a, b; $R_1$=H
37a, b; $R_1$=Ms
f 71%
f 69%
f 70%
93% g
43a, b; X=$CO_2$Me
44a, b; X=CHO
93% g
45a, b; X=$CO_2$Me
46a, b; X=CHO
95% g
38a, b; X=$CO_2$Me
39a, b; X=CHO
f 74%
b
97%
d
42%
a, b, i
72%
30
95% e
50; R=H
51; R=Ms
89% c
48; R=H
49; R=TBDPS
52; R=Ms
47

## Figure 5

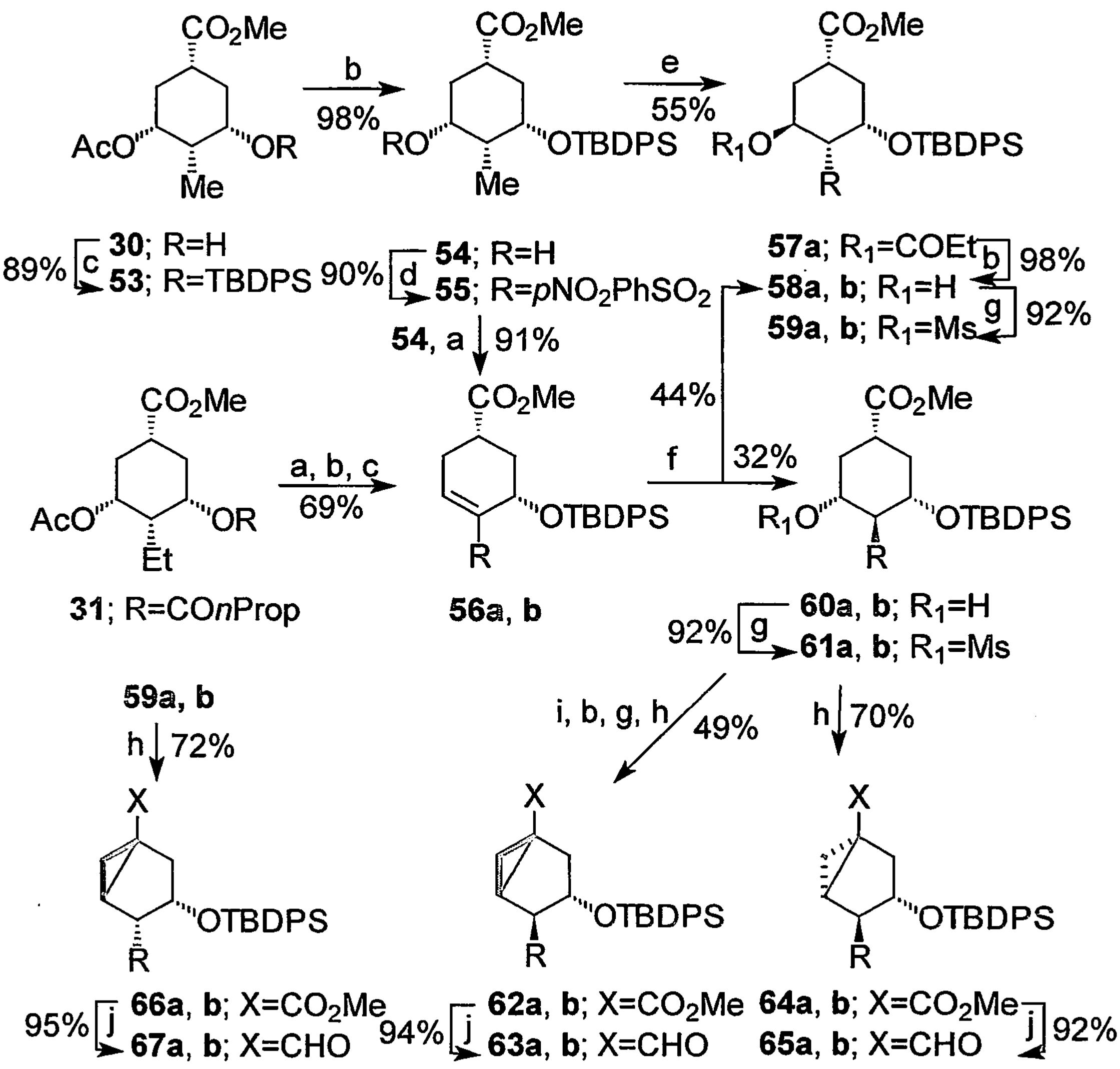
b
98%
e
55%
89% c
30; R=H
53; R=TBDPS
90% d
54; R=H
55; R=pNO2PhSO2
57a; R1=COEt
b 98%
58a, b; R1=H
g 92%
59a, b; R1=Ms
54, a 91%
44%
a, b, c
69%
f
32%
31; R=COnProp
56a, b
92% g
60a, b; R1=H
61a, b; R1=Ms
59a, b
h 72%
i, b, g, h
49%
h 70%
95% j
66a, b; X=CO2Me
67a, b; X=CHO
94% j
62a, b; X=CO2Me
63a, b; X=CHO
64a, b; X=CO2Me
65a, b; X=CHO
j 92%

## Figure 6

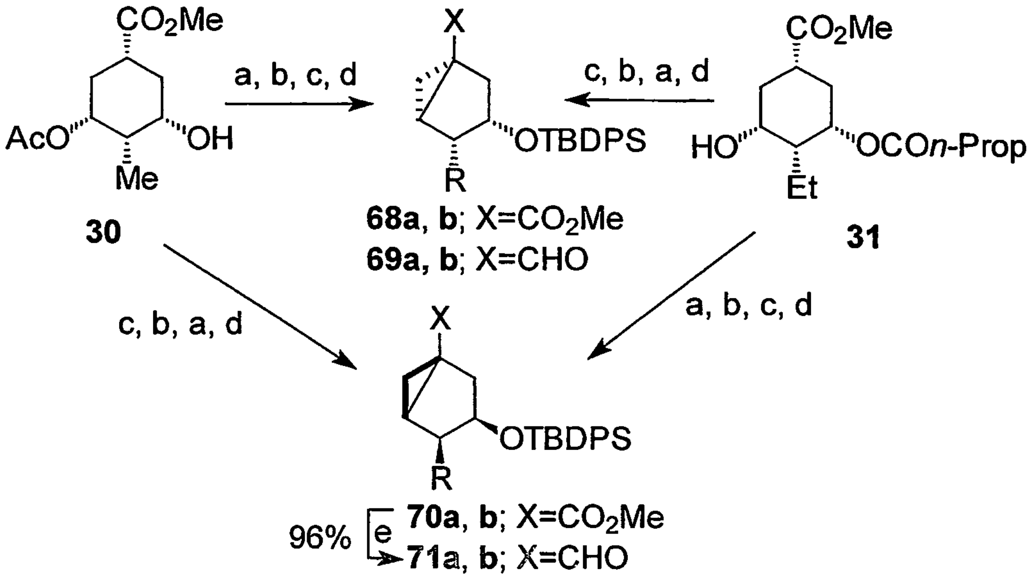


30

31

**68a, b**; X=$CO_2Me$
**69a, b**; X=CHO

**70a, b**; X=$CO_2Me$
**71a, b**; X=CHO

## Figure 7

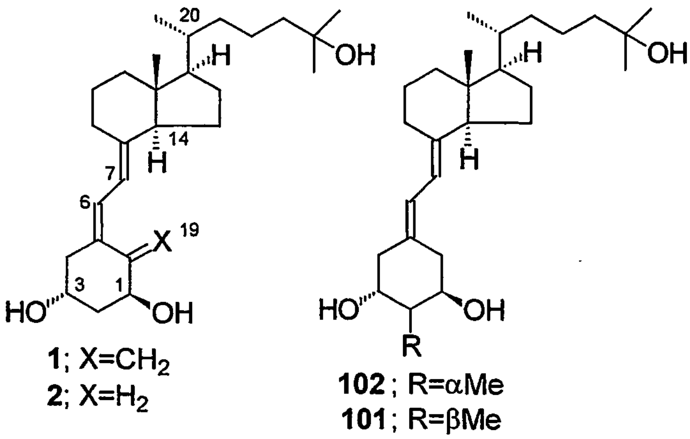

**1**; X=$CH_2$
**2**; X=$H_2$

**102** ; R=αMe
**101** ; R=βMe
**110** ; R=αEt
**109** ; R=βEt

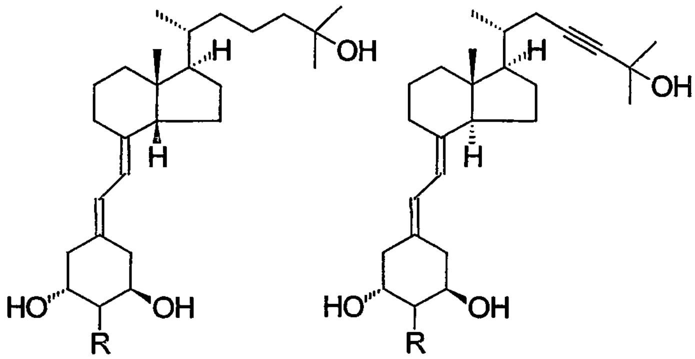

**3**; R=H
**106**; R=αMe
**105**; R=βMe
**114**; R=αEt
**113**; R=βEt

**104** ; R=αMe
**103** ; R=βMe
**112** ; R=αEt
**111** ; R=βEt

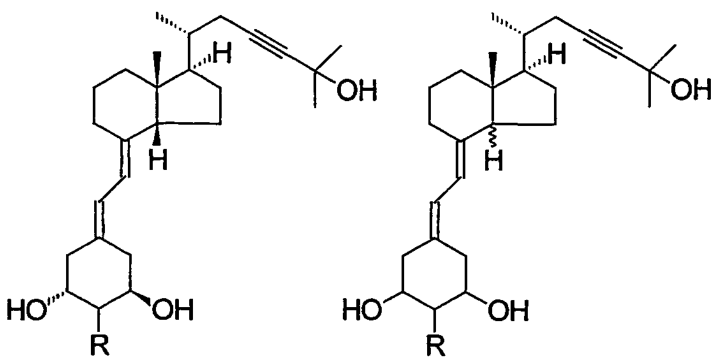

**4**; R=H
**108**; R=αMe
**107**; R=βMe
**116**; R=αEt
**115**; R=βEt

**213**

**Figure 8**

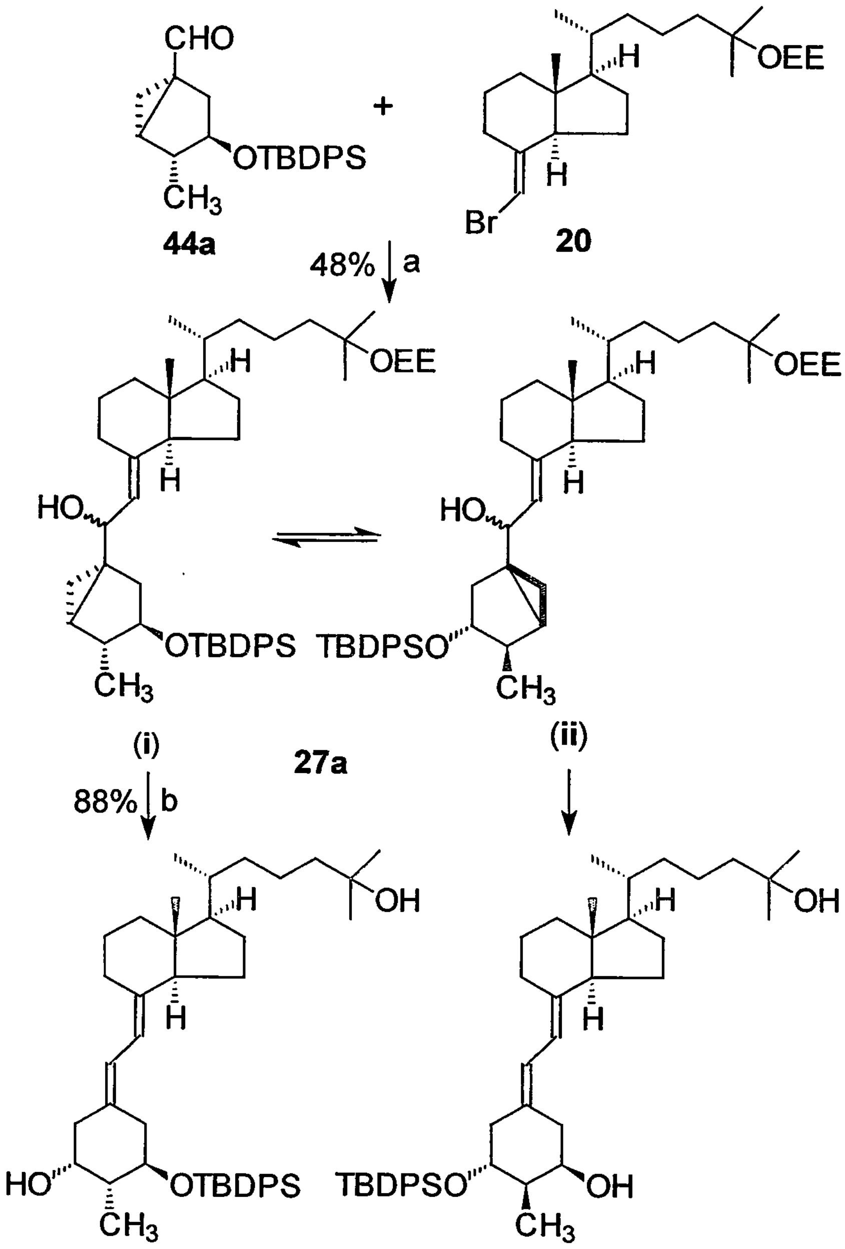


44a

20

27a

**72a**; R=TBDPS
**101**; R=H

**73a**; R=TBDPS
**102**; R=H

## Figure 9

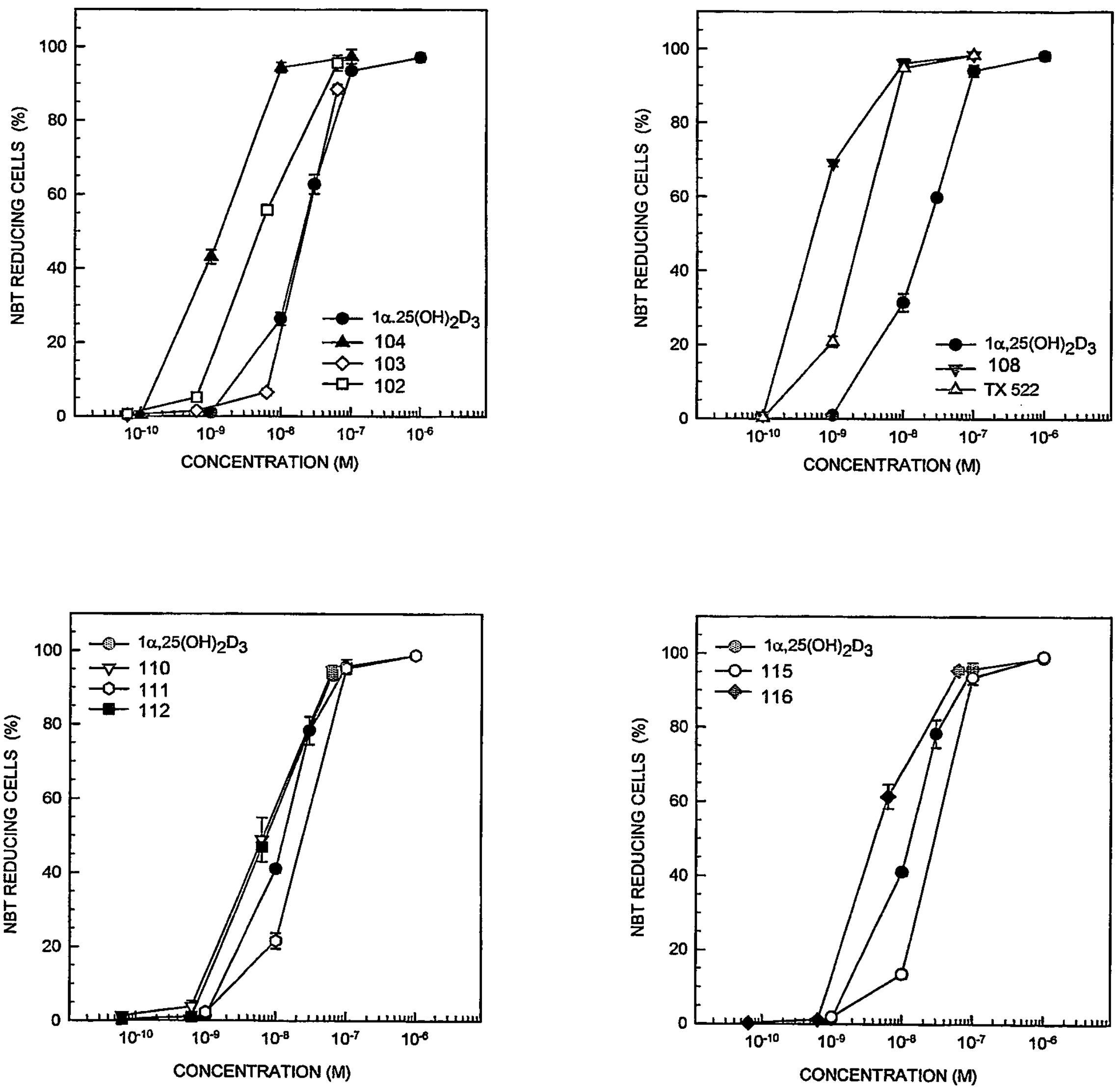
NBT REDUCING CELLS (%)
CONCENTRATION (M)
1α,25(OH)2D3
104
103
102
108
TX 522
110
111
112
115
116

## Figure 10

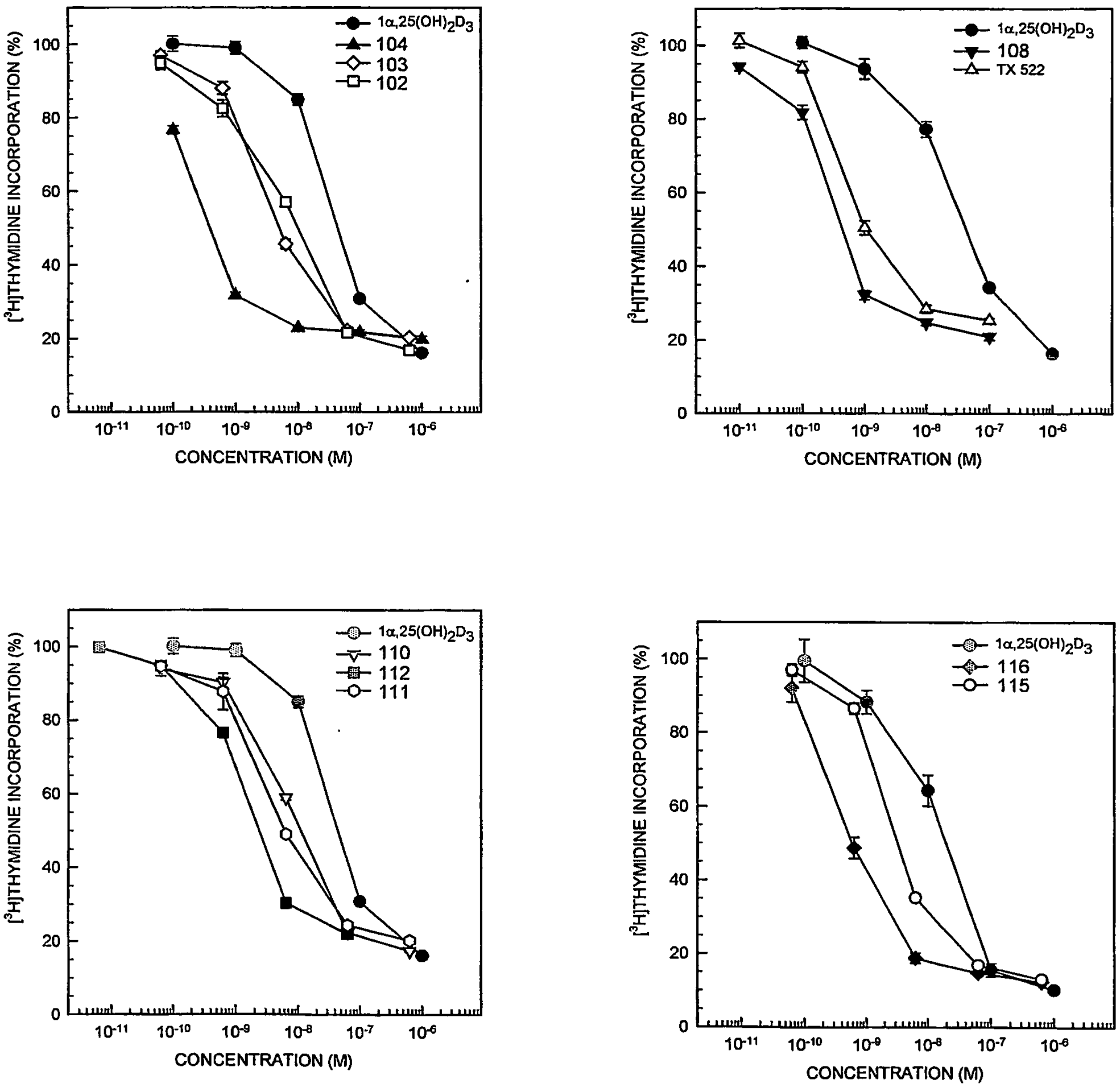

[3H]THYMIDINE INCORPORATION (%)
CONCENTRATION (M)
1α,25(OH)2D3
104
103
102
108
TX 522
110
112
111
116
115
0
20
40
60
80
100
10-11
10-10
10-9
10-8
10-7
10-6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6017907 A
- EP 0972762 A2
- US 4666634 A
- US 6127559 A
- US 5936133 A
- US 5936105 A
- WO 0142251 A


### Non-patent literature cited in the description


- **Nikagawa et al.** *Biochem Pharmacol,* 2000, vol. 60, 1937-1947
- **Nakagawa et al.** *Biochem Pharmacol,* 2000, vol. 59, 691-702
- **Takayama et al.** *Steroids,* 2001, vol. 66, 277-285
- **Fujishima et al.** *Bioorg Med Chem Lett,* 1988, vol. 8, 2145-2148
- **Suhara et al.** *J Org Chem,* 2001, vol. 66, 8760-8771
- **Konno et al.** *J Med Chem,* 2000, vol. 43, 4247-4265
- *J Med Chem,* 1994, vol. 37, 3730-3738
- **Sicinski et al.** *J Med Chem,* 2002, vol. 45, 3366-338
- **Sicinski et al.** *J Med Chem,* 1998, vol. 41, 4662-4674
- **Chou et al.** *Adv. Enzyme Reg.,* 1984, vol. 22, 27
- Handbook of Pharmaceutical Excipients. 1986
- McCutcheon's Detergents and Emulsifiers Annual. MC Publishing Crop, 1981
- Tensid-Taschenbucw. Hanser Verlag, 1981
- Encyclopaedia of Surfactants. Chemical Publishing Co, 1981
- **Trost et al.** *J. Am. Chem. Soc.,* 1992, vol. 114, 9836-9844
- **Wu et al.** *Eur. J. Org. Chem.,* 2001, 3779-3788
- **Van Gool et al.** *Eur. J. Org. Chem.,* 1998, 2241-2248
- **Verstuyf A. et al.** *J Bone Mineral Res,* 1998, vol. 13, 549-558
- **Ostrem V.K et al.** *Proc Natl Acad Sci USA,* 1987, vol. 84, 2610-2614
- **Bouillon R. et al.** *Clin Chem,* 1992, vol. 38, 2055-60